# EUROPEAN PATENT SPECIFICATION

(11) **EP 1 690 915 B1**
(45) Date of publication and mention of the grant of the patent: **26.03.2008**
(21) Application number: 06000901.6
(22) Date of filing: 17.01.2006
(51) Int. Cl.: C09K 19/30, C09K 19/20, C09K 19/46, C09K 19/04

(54) **Mesogenic compounds, liquid crystal medium and liquid crystal display**
Mesogene Verbindungen, flüssigkristallines Medium und Flüssigkristallanzeigevorrichtung
Composés mésogènes, milieu liquide cristallin et dispositif d'affichage à cristaux liquides

(30) Priority: 14.02.2005 EP 05003035
(43) Date of publication of application: 16.08.2006
(73) Proprietor: Merck Patent GmbH, 64293 Darmstadt (DE)
(72) Inventor: Farrand, Louise Diane, Dr., Blandford Forum Dorset DT1 9ED (GB); Saxton, Patricia Eileen, Romsey Hampshire SO51-8PB (GB); Patrick, John, Poole Dorset BH20 4DH (GB)

(56) References cited:
- WO-A-20/04046805
- DE-A1- 10 217 273
- US-A- 5 998 499
- US-B1- 6 493 055
- PRASAD V ET AL: "DIMERIC COMPOUNDS CONTAINING MALONIC ACID AS THE CENTRAL LINKING UNIT: SYNTHESIS AND MESOMORPHIC PROPERTIES" LIQUID CRYSTALS, TAYLOR AND FRANCIS, ABINGDON, GB, vol. 29, no. 9, September 2002 (2002-09), pages 1113-1119, XP001116891 ISSN: 0267-8292

## Description

### Field of the invention

The present invention relates to mesogenic compounds, liquid crystal media comprising these compounds and to electro-optical displays comprising these mesogenic media as light modulation media, in particular to displays which are operated at a temperature at which the mesogenic modulation media are in an optically isotropic phase, preferably in a blue phase.

### Problem to be solved and state of the art

Electro-optical displays and mesogenic light modulation media, which are in the isotropic phase when being operated in the display are described in DE 102 17 273 A. Electro-optical displays, and mesogenic light modulation media, which are in the optically isotropic blue phase, when being operated in the display are described in WO 2004/046 805.

The mesogenic media and displays described in these references provide several significant advantages compared to well-known and widely used displays using liquid crystals in the nematic phase, like for example liquid crystal displays (LCDs) operating in the twisted nematic (TN)-, the super twisted nematic (STN)-, the electrically controlled birefringence (ECB)-mode with its various modifications and the in-plane switching (IPS)-mode. Amongst these advantages are most pronounced their much faster switching times, and significantly wider optical viewing angle.

Whereas, compared to displays using mesogenic media in another liquid crystalline phase, as e.g. in the smectic phase in surface stabilized ferroelectric liquid crystal displays (SSF LCDs), the displays of DE 102 17 273.0 and WO 2004/046 805 are much easier to be produced. For example, they do not require a very thin cell gap in the first place and the electro-optical effect is not very sensitive to small variations of the cell gap as well.

However, the liquid crystal media described in these mentioned patent applications still require operating voltages, which are not low enough for some applications. Further the operating voltages of these media vary with temperature, and it is generally observed, that at a certain temperature the voltage dramatically increases with increasing temperature. This limits the applicability of liquid crystal media in the blue phase for display applications. A further disadvantage of the liquid crystal media described in these patent applications is their moderate reliability which is insufficient for very demanding applications. This moderate reliability may be for example expressed in terms of the voltage holding ratio parameter (VHR), which in liquid crystal media as described above may be below 90%.

Some compounds and compositions have been reported which possess a blue phase between the cholesteric phase and the isotropic phase and can usually be observed by optical microscopy. These compounds or compositions for which the blue phases are observed are typically single mesogenic compounds or mixtures showing a high chirality. However, generally the blue phases observed only extend over a very small temperature range, which is typically less than 1 degree centigrade wide, and/or the blue phase is located at rather inconvenient temperatures.

US 6,493,055 B1 discloses i.a. a liquid crystal compound, where three identical mesogenic groups are attached via three identical linking groups to a propane-1,2,3-triyl central unit.

In order to operate the novel fast switching display mode of WO 2004/046 805 the light modulation medium to be used has to be in the blue phase over a broad range of temperatures encompassing ambient temperature, however. Thus, a light modulation medium possessing a blue phase which is as wide as possible and conveniently located is required.

Therefore there is a strong need for a modulation medium with a blue phase with a wide phase range, which may be achieved either by an appropriate mixture of mesogenic compounds themselves or, preferably by mixing a host mixture with appropriate mesogenic properties with a single dopant or a mixture of dopants that stabilises the blue phase over a wide temperature range.

Summarizing, there is a need for liquid crystal media, which can be operated in liquid crystal displays which are operated at temperatures where the media is in the blue phase, which provide the following technical improvements:
- a reduced operating voltage,
- a reduced temperature dependency of the operating voltage and
- an improved reliability, e.g. VHR.

### Present invention

Surprisingly, it has now been found that compounds of formula I enhance the width of the blue phase in respective media or lead to a decreased temperature dependence of the electro-optical response or an increase of the range of temperatures over which the temperature dependence is negligible or a to a combination of two or of all three of these effects.

In a preferred embodiment of the present invention the compounds used according to the present invention are chiral compounds, preferably they comprise at least one chirally substituted atom and most preferably a chirally substituted C-atom.

According to the present invention are compounds of formula I 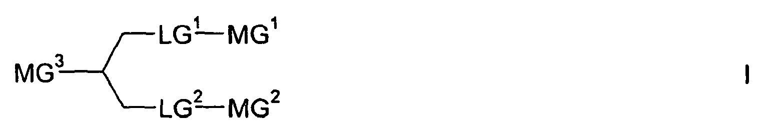 wherein
- LG¹ and LG²: are, independently of one another, LG, and
- LG: is -O-, -O-(C=O)- or OCF₂,
- MG¹, MG² and MG³: are, independently of each other, MG, and
- MG: in each occurrence, independently of one another, is 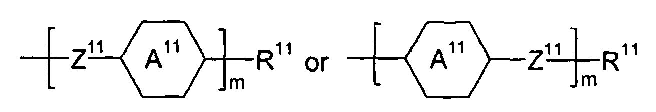
wherein
- R¹¹: is H, F, Cl, CN, NCS, SF₅, SO₂CF₃ or alkyl, which is straight chain or branched, preferably has 1 to 20 C-atoms, is unsubstituted, mono- or poly-substituted by F, Cl, or CN, and in which one or more non-adjacent CH₂ groups are optionally replaced, in each case independently from one another, by -O-, -S-, -NH-,-NR⁰¹-, -SiR⁰¹R⁰²-, -CO-, -COO-, -OCO-, -OCO-O-, -S-CO-, -CO-S-, -CY⁰¹=CY⁰¹- or -C≡C- in such a manner that O and/or S atoms are not linked directly to one another, preferably H, Halogen, n-alkyl, n-alkoxy with 1 to 9 C-atoms preferably 2 to 5 C-atoms, alkenyl, alkenyloxy or alkoxyalkyl with 2 to 9 C-atoms, preferably with 2 to 5 C-atoms or CN, NCS, halogen, preferably F, Cl, halogenated alkyl, alkenyl or alkoxy, preferably mono-, di fluorinated or oligofluorinated alkyl, alkenyl or alkoxy, especially preferred CF₃, OCF₂H or OCF₃, 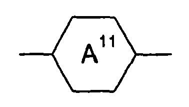 is and, in case it is occurring more than once, also these are in each occurrence, independently of each other, an aromatic and/or alicyclic ring, or a group comprising two or more fused aromatic or alicyclic rings, wherein these rings optionally contain one or more hetero atoms selected from N, O and/or S, and are optionally monosubstituted or polysubstituted by R, preferably at least one of, more preferably at least two of and most preferably all three of 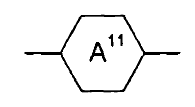 next to the central C-atom shown in formula I are an optionally substituted aromatic ring, preferably a phenyl ring,
- R: has the meaning given for R¹¹ and preferably is alkyl with 1 to 12 C-atoms,
- Z¹¹: is, and in case it is occurring more than once, these are in each occurrence, independently of each other, -CO-O-, -O-CO-, -S-CO-, -CO-S-, -CO-NR⁰¹-, -NR⁰¹-CO-, -OCH₂-, -CH₂O-, -SCH₂-, -CH₂S-, -CF₂O-, -OCF₂-, -CF₂S-, -SCF₂-, -CH₂CH₂-, -CF₂CH₂-, -CH₂CF₂-, -CF₂CF₂-, -CH=N-, -N=CH-, -N=N-, -CH=CR⁰¹-, -CR⁰¹=CH-, -CY⁰¹=CY⁰²-, -C≡C-, -(CH₂)₄-, -CH=CH-CO-O-, -O-CO-CH=CH- or a single bond, preferably at least one of Z¹¹ next to the central C-atom is, preferably all of them next to the central C-atom are a single bond,
- Y⁰¹ and Y⁰²: are, independently of each other, F, Cl or CN, and alternatively one of them may be H,
- R⁰¹ and R⁰²: are, independently of each other, H or alkyl with 1 to 12 C-atoms,
- m: is , in each occurrence independently, 1, 2 or 3, preferably 1 or 2, whereby preferably the sum of m of all three mesogenic groups MG¹ to MG³ is 3, 4, 5, 6, 7 or 8, preferably 3, 4, or 5,
and chiral compounds of formula I are encompassed too.

Compounds of formula I are also an object of the present application.

Preferred are compounds of formula I wherein the parameters have the following meaning
- R¹¹: is F, Cl, CN, NCS, CF₃, OCF₃, SF₅, alkyl, alkoxy, alkenyl or alkynyl, preferably F, Cl, CF₃, SF₅, alkyl or alkoxy, 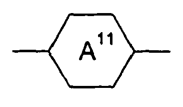 preferably is, independently of each other in each occurrence, 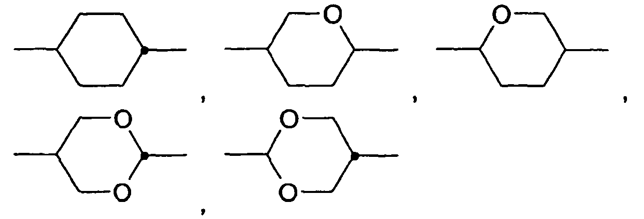 or 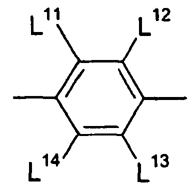 and
- L¹¹ to L¹⁴: are, independently of each other, H or F, preferably two or more, most preferably three or more of them are F, and
- LG¹ and LG²: preferably are, identically to each other.

In a preferred embodiment MG¹ and MG² are identical to each other.

The compounds of formula I are preferably selected from its sub-formulae I' and I" 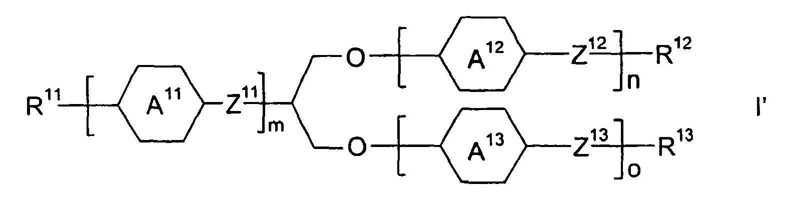 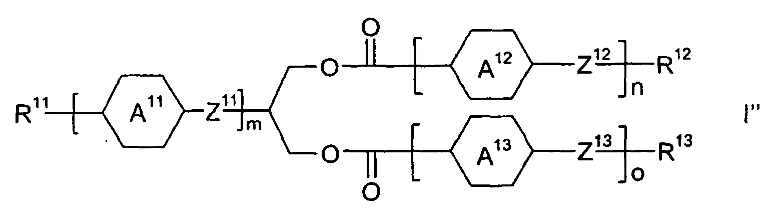 wherein the parameters have the respective meanings given under formula I above and
- R¹² and R¹³,: independently from one another, have the meaning given for R¹¹ under formula I above, 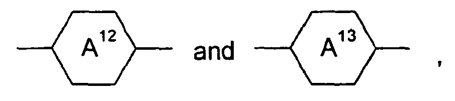 independently of each other in each occurrence, have the meaning given for 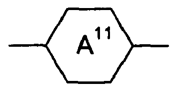 under formula I above,
- Z¹² and Z¹³_{,}: independently from one another, have the meaning given for Z¹¹ under formula I above,
- n and o,: dependently from one another, have the meaning given for m under formula I above, and are preferably, independently of one another, 1 or 2, most preferably 1,
and chiral compounds of formulae I' and I" are encompassed too.

Particularly preferred are compounds of formula I', wherein
- at least one of Z¹¹ in at least one of MG¹, MG² and MG³, preferably at least one of Z¹¹ in each of MG¹ and MG², and most preferably in each of MG¹, MG² and MG³ is selected from -CO-O-, -O-CO-, -CH₂-O-, -O-CH₂-, -CF₂-O-, -O-CF₂-, -CH=CH- or -CEC-, most preferably from -CF₂-O- or -O-CF₂-,
- one or more of the rings A¹¹, which are present, is, respectively are, phenylene, that is optionally substituted by one or more groups R and/or F-atoms and/or
- R¹¹ is alkyl with 1 to 12, preferably 1 to 8 C-atoms, or alkenyl or alkynyl with 2 to 12, preferably 2 to 7 C-atoms.

MG¹ and MG² are preferably selected independently of each other from 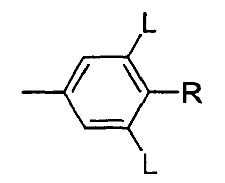 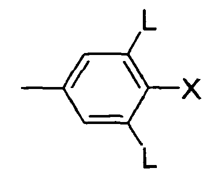 and
- R: is alkyl, alkenyl, alkoxy, oxaalkyl, and most preferably alkoxy, or alkenyl,
- L ,: in each occurrence independently of one another, is H or F, and
- X: CN, NCS, halogen, halogenated alkyl, alkoxy, alkenyl or alkenyloxy or SF₅, preferably F, -CF₃ or -OCF₃. and most preferably CF₃,
and MG³ preferably is selected from 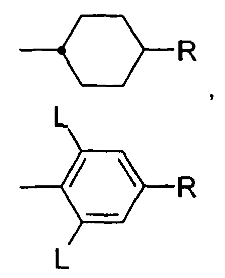 and 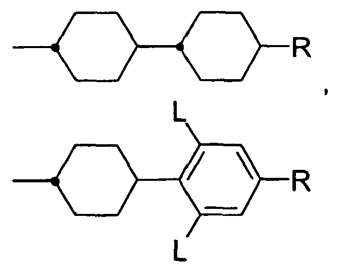 and 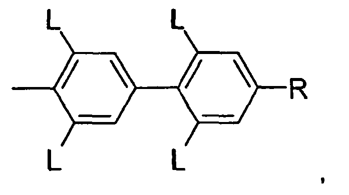 wherein preferably
- R: is alkyl, alkenyl, alkoxy, oxaalkyl, and most preferably alkyl, and
- L ,: in each occurrence independently of one another, is H or F.

In a preferred embodiment of the present invention rings A¹¹ to A¹³ are, independently of each other, an aromatic or alicyclic ring, preferably a 5-, 6- or 7-membered ring, or a group comprising two or more, preferably two or three, fused aromatic or alicyclic rings, wherein these rings optionally contain one or more hetero atoms selected from N, O and/or S, and are optionally mono- or polysubstituted with L, wherein L is F, Cl, Br, CN, OH, NO₂, and/or an alkyl, alkoxy, alkylcarbonyl or alkoxycarbonyl group with 1 to 12 C atoms, wherein one or more H atoms are optionally replaced by F or Cl.

L is preferably F, Cl, CN, OH, NO₂, CH₃, C₂H₅, OCH₃, OC₂H₅, COCH₃, COC₂H₅, COOCH₃, COOC₂H₅, CF₃, OCF₃, OCHF₂ or OC₂F₅, in particular F, Cl, CN, CH₃, C₂H₅, OCH₃, COCH₃ or OCF₃, most preferably F, Cl, CH₃, OCH₃ or COCH₃.

Preferred rings A¹¹ to A¹³ are for example furan, pyrrol, thiophene, oxazole, thiazole, thiadiazole, imidazole, phenylene, cyclohexylene, cyclohexenylene, pyridine, pyrimidine, pyrazine, azulene, indane, naphthalene, tetrahydronaphthalene, decahydronaphthalene, tetrahydropyrane, anthracene, phenanthrene and fluorene.

Particularly preferably one or more of these rings A¹¹ to A¹³ is, respectively are, selected from furane-2,5-diyl, thiophene-2,5-diyl, thienothiophene-2,5-diyl, dithienothiophene-2,6-diyl, pyrrol-2,5-diyl, 1,4-phenylene, azulene-2,6-diyl, pyridine-2,5-diyl, pyrimidine-2,5-diyl, naphthalene-2,6-diyl, 1,2,3,4-tetrahydro-naphthalene-2,6-diyl, indane-2,5-diyl, or 1,4-cyclohexylene wherein one or two non-adjacent CH₂ groups are optionally replaced by O and/or S, wherein these groups are unsubstituted, mono- or polysubstituted by L as defined above.

Preferably 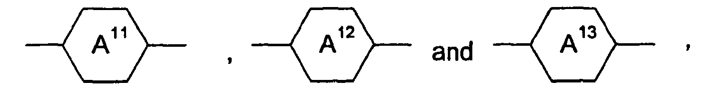 independently of each other in each occurrence, are 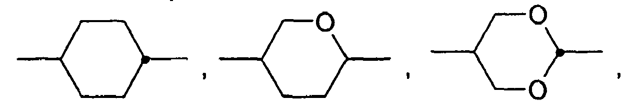 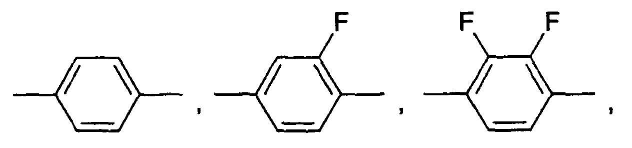 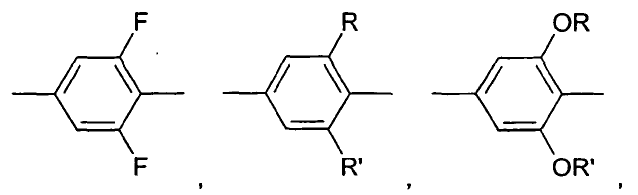 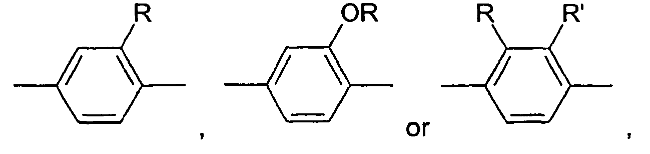 wherein
- R and R': have, independently of each other the meaning given above for R, preferably for R¹¹ and most preferably are, independently of each other, alkyl with 1 to 12 C-atoms, preferably with 1 to 7 C-atoms, or alkenyl or alkynyl with 2 to 12 C-atoms, preferably with 2 to 7 C-atoms, in both of which one or more non-adjacent -CH₂- groups, not adjacent to the phenyl ring, may be replaced by -O- and/or -CH=CH- and/or one or more H-atoms may be replaced by halogen, preferably by F and preferably is alkyl, preferably methyl, ethyl or propyl, preferably methyl, in a preferred embodiment R and R' are identical to each other,
or their mirror images
and more preferably at least one of them, in particular at least one each of them, is 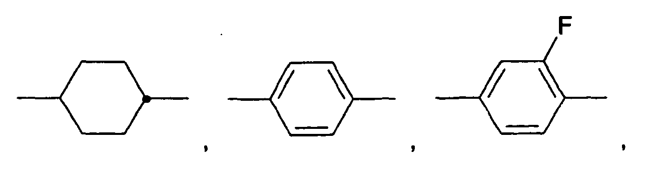 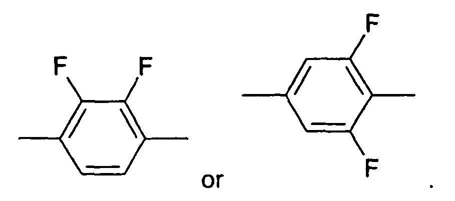 and most preferably at least one of 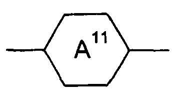 is 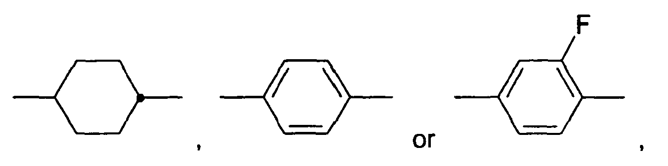 and/or at least of 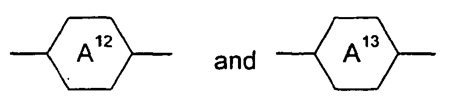 each is

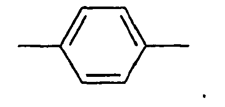

In a preferred embodiment of the present invention each one of the groups 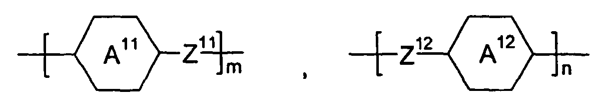 and, 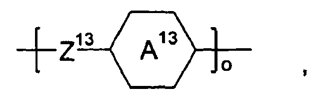 contains, preferably all of them contain only monocyclic rings A¹¹, A¹² and A¹³. Very preferably this is a group/these are groups containing one, two or three 5- and/or 6-membered rings.

Preferred sub-formulae for these groups are listed below. For reasons of simplicity, Phe in these groups is 1,4-phenylene, PheL is a 1,4-phenylene group which is substituted by 1 to 4 groups L as defined above, Cyc is 1,4-cyclohexylene, Pyd is pyridine-2,5-diyl and Pyr is pyrimidine-2,5-diyl. The following list of preferred groups is comprising the sub formulae A-1 to A-20 as well as their mirror images,

| | |
|---|---|
| -Phe- | A-1 |
| -Pyd- | A-2 |
| -Pyr- | A-3 |
| -PheL- | A-4 |
| -Cyc- | A-5 |
| -Phe-Z-Cyc- | A-6 |
| -Cyc-Z-Cyc- | A-7 |
| -PheL-Cyc- | A-8 |
| -Phe-Z-Phe- | A-9 |
| -Phe-Z-Pyd- | A-10 |
| -Pyd-Z-Phe- | A-11 |
| -Phe-Z-Pyr- | A-12 |
| -Pyr-Z-Phe- | A-13 |
| -PheL-Z-Phe- | A-14 |
| -PheL-Z-Pyd- | A-15 |
| -PheL-Z-Pyr- | A-16 |
| -Pyr-Z-Pyd- | A-17 |
| -Pyd-Z-Pyd- | A-18 |
| -Pyr-Z-Pyr- | A-19 |
| -PheL-Z-PheL- | A-20 |

In these preferred groups Z has the meaning of Z¹¹ as given in formula I. Preferably Z is -CF₂-O- or -O-CF₂- or a single bond.

Very preferably, at least one of the groups 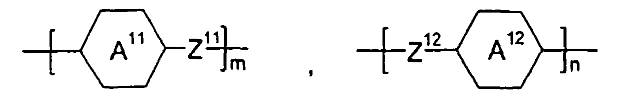 and, 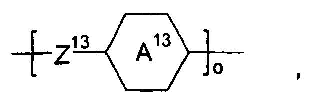 most preferably all of them, are selected from the following formulae la to Ir and their respective mirror images 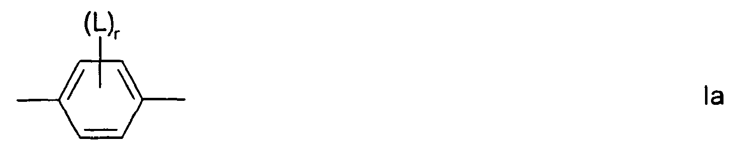 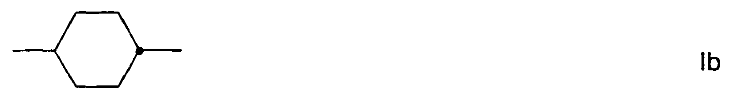 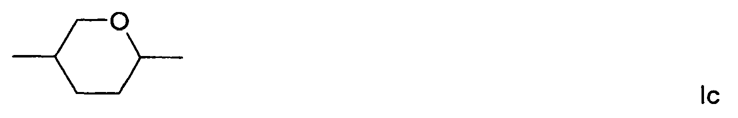 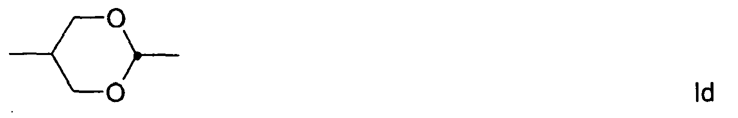 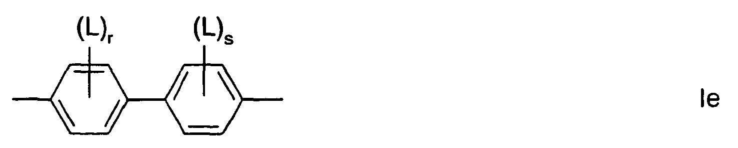 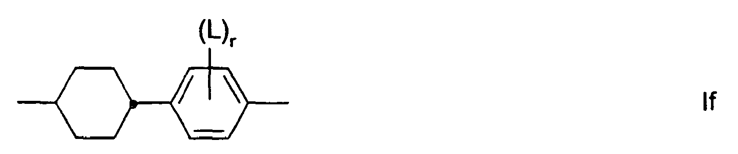 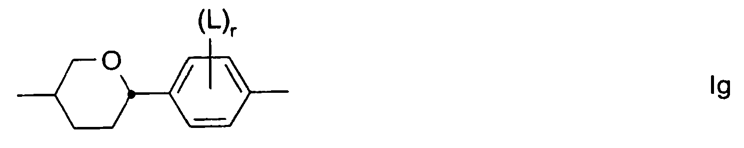 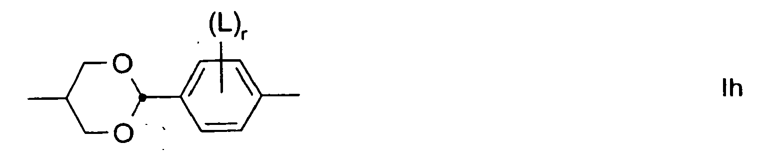 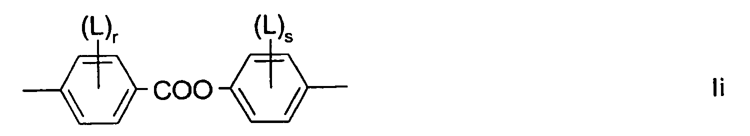 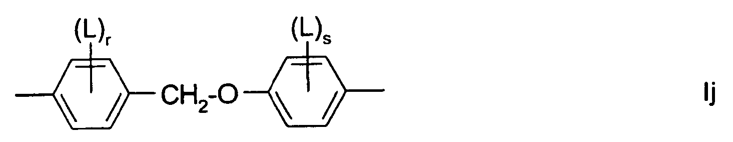 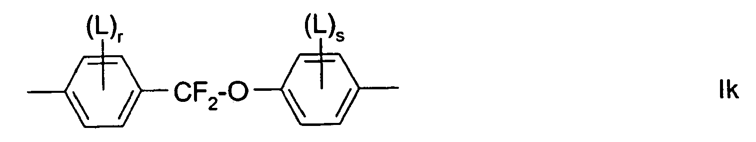 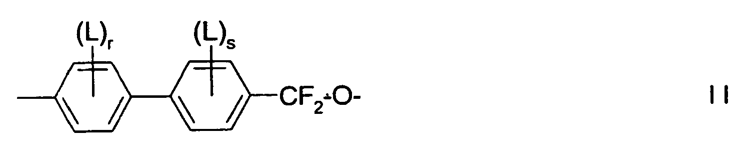 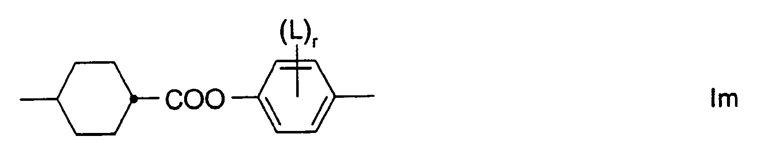 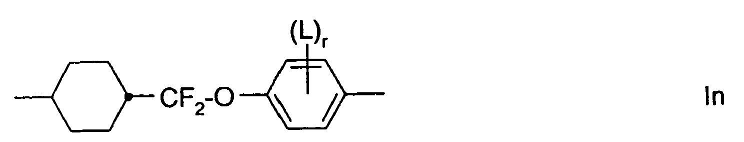 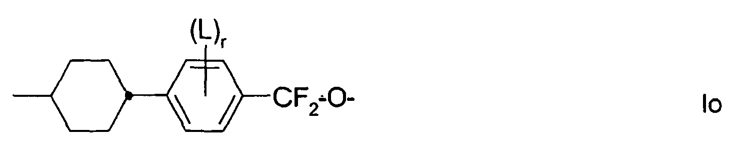 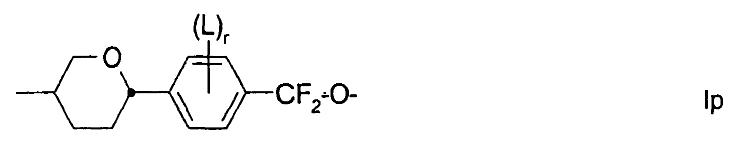 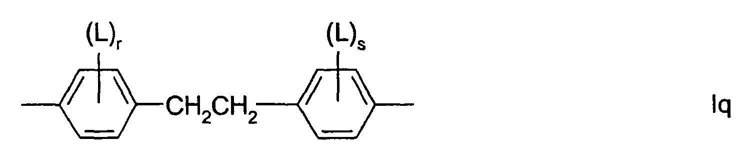 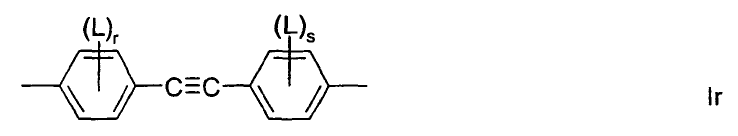 wherein L has the meaning given above and r and s are independently of each other, 0, 1, 2, 3 or 4, preferably 0, 1 or 2. 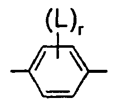 in these preferred formulae is very preferably 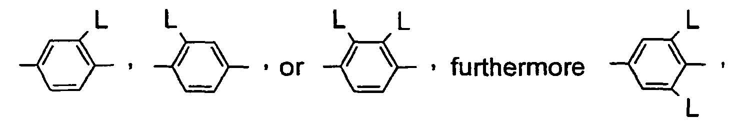 with L having each independently one of the meanings given above.

Especially preferred compounds of formula I comprise at least one group each in rings A¹¹ and A¹² of the formula 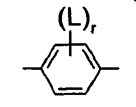 wherein r is 1 or 2.

Further preferred compounds of formula I comprise at least one group each in rings A¹¹, A¹² and A¹³ of the formula 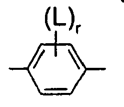 wherein r is 2 and/or at least one group each of the formula 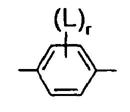 wherein r is 0, 1 or 2.

Very preferably, at least one of the groups 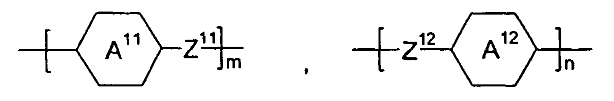 and, 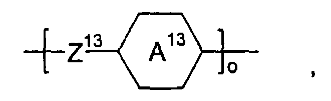 most preferably all of them, are selected from the following formulae and their respective mirror images 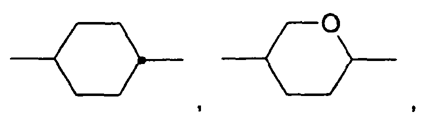 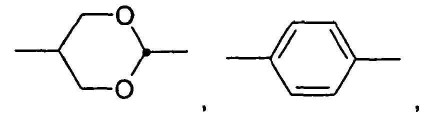 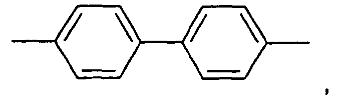 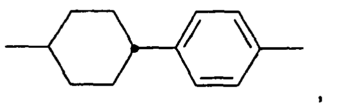 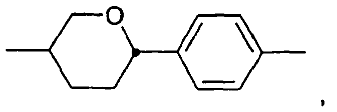 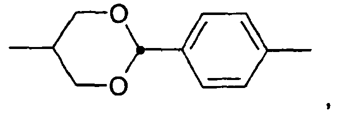 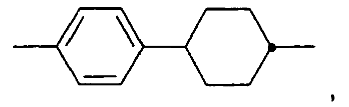 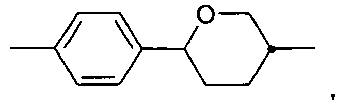 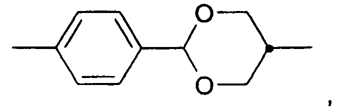 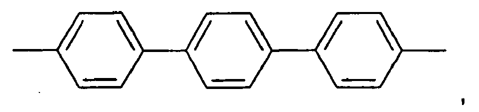 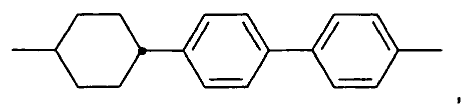 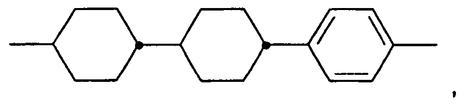 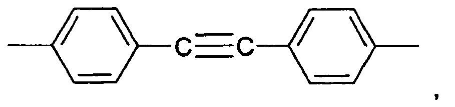 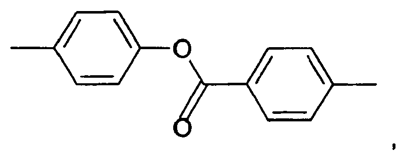 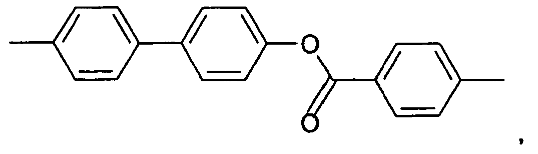 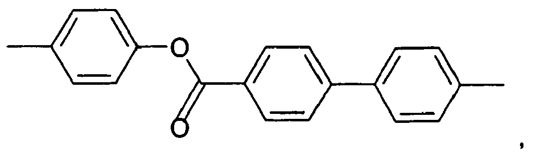 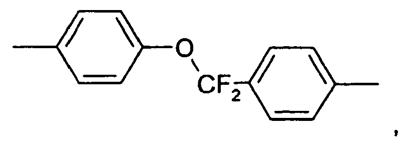 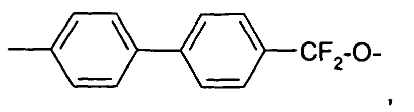 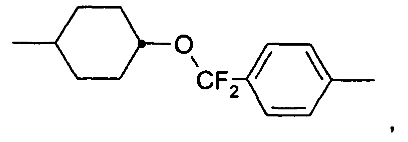 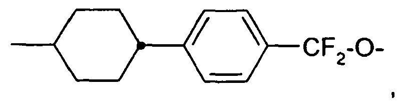 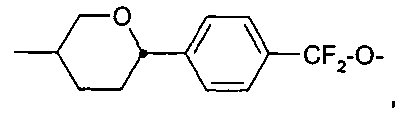 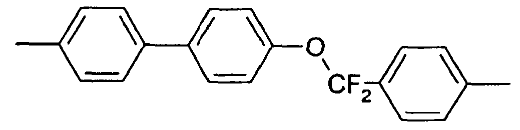 or 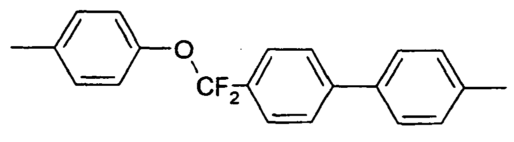 wherein the 1,4-phenylene rings may optionally be substituted by R or L, preferably by alkyl, preferably by methyl, and/or by alkoxy and/or by halogen, preferably F.

More preferably at least one of the groups 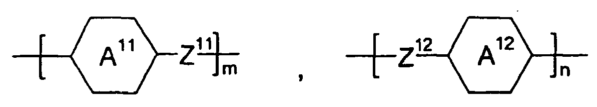 and, 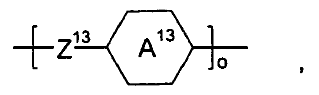 most preferably all of them, are selected from the following formulae and their respective mirror images 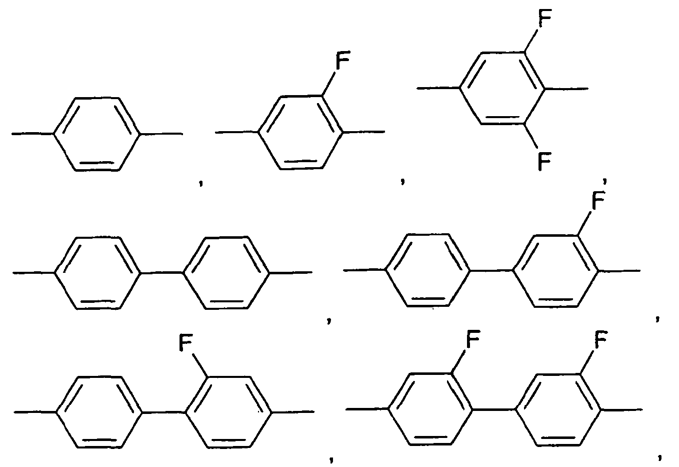 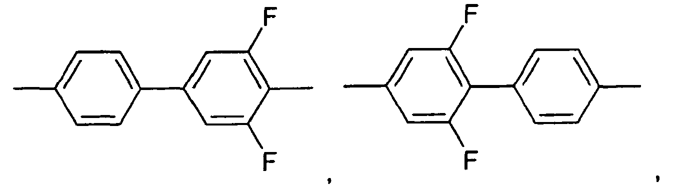 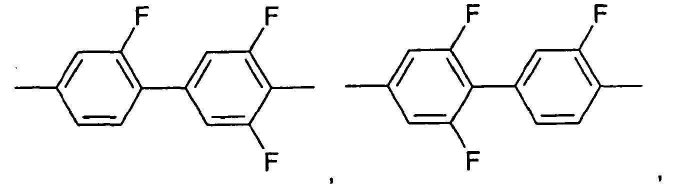 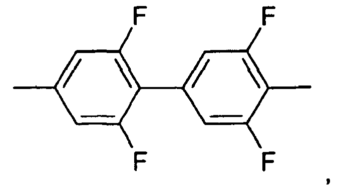 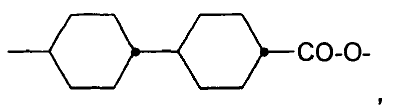 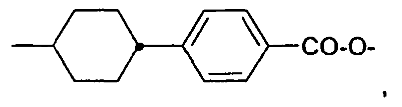 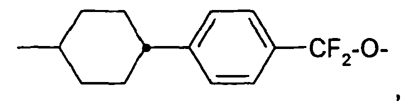 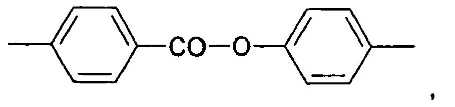 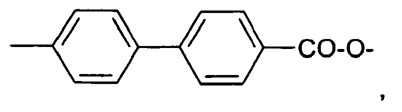 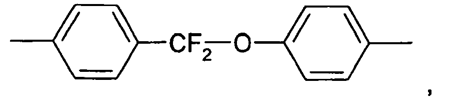 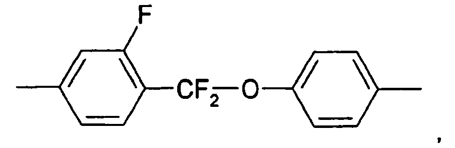 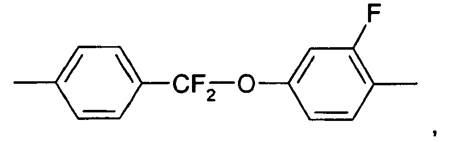 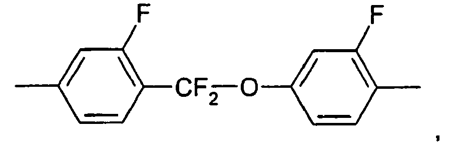 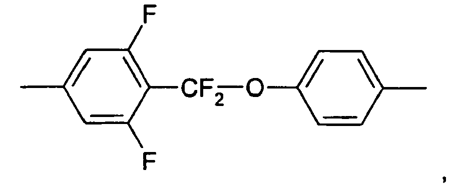 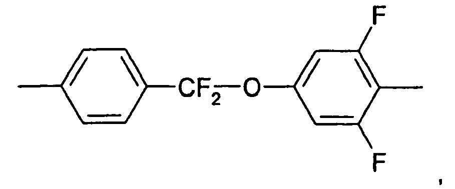 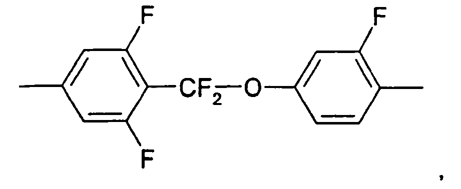 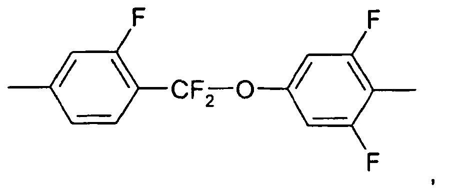 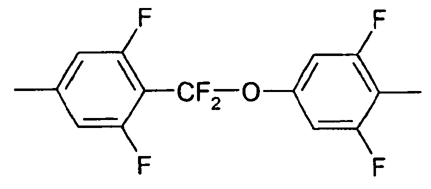 or

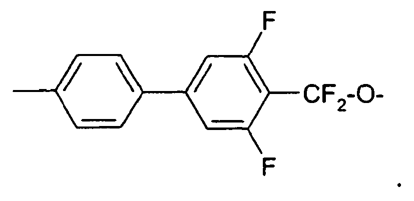

An alkyl or an alkoxy radical, i.e. an alkyl where the terminal CH₂ group is replaced by -O-, in this application may be straight-chain or branched. It is preferably straight-chain, has 1, 2, 3, 4, 5, 6, 7 or 8 carbon atoms and accordingly is preferably methyl, ethyl, propyl, butyl, pentyl, hexyl, heptyl, octyl, ethoxy, propoxy, butoxy, pentoxy, hexoxy, heptoxy, or octoxy, furthermore nonyl, decyl, undecyl, dodecyl, tridecyl, tetradecyl, pentadecyl, nonoxy, decoxy, undecoxy, dodecoxy, tridecoxy or tetradecoxy, for example.

Oxaalkyl, i.e. an alkyl group in which one non-terminal CH₂ group is replaced by -O-, is preferably straight-chain 2-oxapropyl (= methoxymethyl), 2- (= ethoxymethyl) or 3-oxabutyl (= 2-methoxyethyl), 2-, 3-, or 4-oxapentyl, 2-, 3-, 4-, or 5-oxahexyl, 2-, 3-, 4-, 5-, or 6-oxaheptyl, 2-, 3-, 4-, 5-, 6- or 7-oxaoctyl, 2-, 3-, 4-, 5-, 6-, 7- or 8-oxanonyl or 2-, 3-, 4-, 5-, 6-,7-, 8- or 9-oxadecyl, for example.

An alkenyl group, i.e. an alkyl group wherein one or more CH₂ groups are replaced by -CH=CH-, may be straight-chain or branched. It is preferably straight-chain, has 2 to 10 C atoms and accordingly is preferably vinyl, prop-1-, or prop-2-enyl, but-1-, 2- or but-3-enyl, pent-1-, 2-, 3- or pent-4-enyl, hex-1-, 2-, 3-, 4- or hex-5-enyl, hept-1-, 2-, 3-, 4-, 5- or hept-6-enyl, oct-1-, 2-, 3-, 4-, 5-, 6- or oct-7-enyl, non-1-, 2-, 3-, 4-, 5-, 6-, 7- or non-8-enyl, dec-1-, 2-, 3-, 4-, 5-, 6-, 7-, 8- or dec-9-enyl.

Especially preferred alkenyl groups are C₂-C₇-1E-alkenyl, C₄-C₇-3E-alkenyl, C₅-C₇-4-alkenyl, C₆-C₇-5-alkenyl and C₇-6-alkenyl, in particular C₂-C₇-1E-alkenyl, C₄-C₇-3E-alkenyl and C₅-C₇-4-alkenyl. Examples for particularly preferred alkenyl groups are vinyl, 1E-propenyl, 1E-butenyl, 1E-pentenyl, 1E-hexenyl, 1E-heptenyl, 3-butenyl, 3E-pentenyl, 3E-hexenyl, 3E-heptenyl, 4-pentenyl, 4Z-hexenyl, 4E-hexenyl, 4Z-heptenyl, 5-hexenyl, 6-heptenyl and the like. Groups having up to 5 C atoms are generally preferred.

In an alkyl group, wherein one CH₂ group is replaced by -O- and one by -CO-, these radicals are preferably neighboured. Accordingly these radicals together form a carbonyloxy group -CO-O- or an oxycarbonyl group -O-CO-. Preferably such an alkyl group is straight-chain and has 2 to 6 C atoms.

It is accordingly preferably acetyloxy, propionyloxy, butyryloxy, pentanoyloxy, hexanoyloxy, acetyloxymethyl, propionyloxymethyl, butyryloxymethyl, pentanoyloxymethyl, 2-acetyloxyethyl, 2-propionyloxyethyl, 2-butyryloxyethyl, 3-acetyloxypropyl, 3-propionyloxypropyl, 4-acetyloxybutyl, methoxycarbonyl, ethoxycarbonyl, propoxycarbonyl, butoxycarbonyl, pentoxycarbonyl, methoxycarbonylmethyl, ethoxycarbonylmethyl, propoxycarbonylmethyl, butoxycarbonylmethyl, 2-(methoxycarbonyl)ethyl, 2-(ethoxycarbonyl)ethyl, 2-(propoxycarbonyl)ethyl, 3-(methoxycarbonyl)propyl, 3-(ethoxycarbonyl)propyl, 4-(methoxycarbonyl)-butyl.

An alkyl group wherein two or more CH₂ groups are replaced by -O- and/or -COO-, it can be straight-chain or branched. It is preferably straight-chain and has 3 to 12 C atoms. Accordingly it is preferably bis-carboxy-methyl, 2,2-bis-carboxy-ethyl, 3,3-bis-carboxy-propyl, 4,4-bis-carboxy-butyl, 5,5-bis-carboxy-pentyl, 6,6-bis-carboxy-hexyl, 7,7-bis-carboxy-heptyl, 8,8-bis-carboxy-octyl, 9,9-bis-carboxy-nonyl, 10,10-bis-carboxy-decyl, bis-(methoxycarbonyl)-methyl, 2,2-bis-(methoxycarbonyl)-ethyl, 3,3-bis-(methoxycarbonyl)-propyl, 4,4-bis-(methoxycarbonyl)-butyl, 5,5-bis-(methoxycarbonyl)-pentyl, 6,6-bis-(methoxycarbonyl)-hexyl, 7,7-bis-(methoxycarbonyl)-heptyl, 8,8-bis-(methoxycarbonyl)-octyl, bis-(ethoxycarbonyl)-methyl, 2,2-bis-(ethoxycarbonyl)-ethyl, 3,3-bis-(ethoxycarbonyl)-propyl, 4,4-bis-(ethoxycarbonyl)-butyl, 5,5-bis-(ethoxycarbonyl)-hexyl.

A alkyl or alkenyl group that is monosubstituted by CN or CF₃ is preferably straight-chain. The substitution by CN or CF₃ can be in any desired position.

An alkyl or alkenyl group that is at least monosubstituted by halogen, it is preferably straight-chain. Halogen is preferably F or Cl, in case of multiple substitution preferably F. The resulting groups include also perfluorinated groups. In case of monosubstitution the F or Cl substituent can be in any desired position, but is preferably in ω-position. Examples for especially preferred straight-chain groups with a terminal F substituent are fluoromethyl, 2-fluoroethyl, 3-fluoropropyl, 4-fluorobutyl, 5-fluoropentyl, 6-fluorohexyl and 7-fluoroheptyl. Other positions of F are, however, not excluded.

Halogen means F, Cl, Br and I and is preferably F or Cl, most preferably F. Each of R¹¹, R¹², R¹³, R, R' and R" may be a polar or a non-polar group. In case of a polar group, it is preferably selected from CN, SF₅, halogen, OCH₃, SCN, COR⁵, COOR⁵ or a mono- oligo- or polyfluorinated alkyl or alkoxy group with 1 to 4 C atoms. R⁵ is optionally fluorinated alkyl with 1 to 4, preferably 1 to 3 C atoms. Especially preferred polar groups are selected of F, Cl, CN, OCH₃, COCH₃, COC₂H₅, COOCH₃, COOC₂H₅, CF₃, CHF₂, CH₂F, OCF₃, OCHF₂, OCH₂F, C₂F₅ and OC₂F₅, in particular F, Cl, CN, CF₃, OCHF₂ and OCF₃. In case of a non-polar group, it is preferably alkyl with up to 15 C atoms or alkoxy with 2 to 15 C atoms.

Each of R¹¹, R¹², R¹³ R, and R' may be an achiral or a chiral group. In case of a chiral group it is preferably of formula I*: 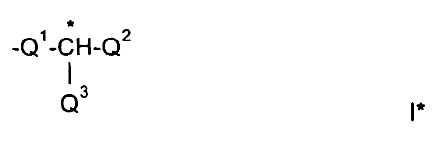 wherein
- Q¹: is an alkylene or alkylene-oxy group with 1 to 9 C atoms or a single bond,
- Q²: is an alkyl or alkoxy group with 1 to 10 C atoms which may be unsubstituted, mono- or polysubstituted by F, Cl, Br or CN, it being also possible for one or more non-adjacent CH₂ groups to be replaced, in each case independently from one another, by -C≡C-, -O-, -S-, -NH-, -N(CH₃)-, -CO-, -COO-, -OCO-, -OCO-O-, -S-CO- or -CO-S- in such a manner that oxygen atoms are not linked directly to one another,
- Q³: is F, Cl, Br, CN or an alkyl or alkoxy group as defined for Q² but being different from Q².

In case Q¹ in formula I* is an alkylene-oxy group, the O atom is preferably adjacent to the chiral C atom.

Preferred chiral groups of formula I* are 2-alkyl, 2-alkoxy, 2-methylalkyl, 2-methylalkoxy, 2-fluoroalkyl, 2-fluoroalkoxy, 2-(2-ethin)-alkyl, 2-(2-ethin)-alkoxy, 1,1,1-trifluoro-2-alkyl and 1,1,1-trifluoro-2-alkoxy.

Particularly preferred chiral groups I* are 2-butyl (=1-methylpropyl), 2-methylbutyl, 2-methylpentyl, 3-methylpentyl, 2-ethylhexyl, 2-propylpentyl, in particular 2-methylbutyl, 2-methylbutoxy, 2-methylpentoxy, 3-methylpentoxy, 2-ethylhexoxy, 1-methylhexoxy, 2-octyloxy, 2-oxa-3-methylbutyl, 3-oxa-4-methylpentyl, 4-methylhexyl, 2-hexyl, 2-octyl, 2-nonyl, 2-decyl, 2-dodecyl, 6-methoxyoctoxy, 6-methyloctoxy, 6-methyloctanoyloxy, 5-methylheptyloxycarbonyl, 2-methylbutyryloxy, 3-methylvaleroyloxy, 4-methylhexanoyloxy, 2-chlorpropionyloxy, 2-chloro-3-methylbutyryloxy, 2-chloro-4-methylvaleryloxy, 2-chloro-3-methylvaleryloxy, 2-methyl-3-oxapentyl, 2-methyl-3-oxahexyl, 1-methoxypropyl-2-oxy, 1-ethoxypropyl-2-oxy, 1-propoxypropyl-2-oxy, 1-butoxypropyl-2-oxy, 2-fluorooctyloxy, 2-fluorodecyloxy, 1,1,1-trifluoro-2-octyloxy, 1,1,1-trifluoro-2-octyl, 2-fluoromethyloctyloxy for example. Very preferred are 2-hexyl, 2-octyl, 2-octyloxy, 1,1,1-trifluoro-2-hexyl, 1,1,1-trifluoro-2-octyl and 1,1,1-trifluoro-2-octyloxy.

In addition, compounds containing an achiral branched alkyl group may occasionally be of importance, for example, due to a reduction in the tendency towards crystallization. Branched groups of this type generally do not contain more than one chain branch. Preferred achiral branched groups are isopropyl, isobutyl (= methylpropyl), isopentyl (= 3-methylbutyl), isopropoxy, 2-methyl-propoxy and 3-methylbutoxy.

In a preferred embodiment of the present invention one or more of R¹¹, R¹², R¹³, R, and R' are -SG-PG.

Particularly preferred are compounds of formula I and its sub-formulae wherein R¹¹ is -SG-PG and additionally preferred m is 0 at the same time.

The polymerisable or reactive group PG is preferably selected from CH₂=CW¹-COO-, 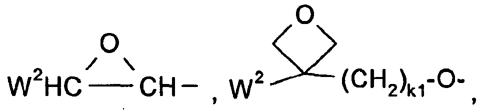 CH₂=CW²-(O)ₖ₁-, CH₃-CH=CH-O-, (CH₂=CH)₂CH-OCO-, (CH₂=CH-CH₂)₂CH-OCO-, (CH₂=CH)₂CH-O-, (CH₂=CH-CH₂)₂N-, HO-CW²W³-, HS-CW²W³-, HW²N-, HO-CW²W³-NH-, CH₂=CW¹-CO-NH-, CH₂=CH-(COO)ₖ₁-Phe-(O)ₖ₂-, Phe-CH=CH-, HOOC-, OCN-, and W⁴W⁵W⁶Si-, with W¹ being H, F, Cl, CN, phenyl or alkyl with 1 to 5 C-atoms, in particular H, F, Cl or CH₃, W² and W³ being independently of each other H or alkyl with 1 to 5 C-atoms, in particular methyl, ethyl or n-propyl, W⁴, W⁵ and W⁶ being independently of each other Cl, oxaalkyl or oxacarbonylalkyl with 1 to 5 C-atoms, Phe being 1,4-phenylene and k₁ and k₂ being independently of each other 0 or 1.

Especially preferably PG is a vinyl group, an acrylate group, a methacrylate group, an oxetane group or an epoxy group, especially preferably an acrylate or methacrylate group.

As for the spacer group SG all groups can be used that are known for this purpose to those skilled in the art. The spacer group SG is preferably of formula SG'-X, such that PG-SG- is PG-SG'-X-, wherein
- SG': is alkylene with up to 20 C atoms which may be unsubstituted, mono- or poly-substituted by F, Cl, Br, I or CN, it being also possible for one or more non-adjacent CH₂ groups to be replaced, in each case independently from one another, by -O-, -S-, -NH-, -NMR⁰¹-, -SiR⁰¹R⁰²-, -CO-, -COO-, -OCO-, -OCO-O-, -S-, -CO-, -CO-S-, -CH=CH- or -C≡C- in such a manner that O and/or S atoms are not linked directly to one another,
- X: is -O-, -S-, -CO-, -COO-, -OCO-, -O-COO-, -CO-NR⁰¹-, -NR⁰¹-CO-,-OCH₂-, -CH₂O-, -SCH₂-, -CH₂S-, -CF₂O-, -OCF₂-, -CF₂S-, -SCF₂-, -CF₂CH₂-, -CH₂CF₂-, -CF₂CF₂-, -CH=N-, -N=CH-, -N=N-, -CH=CR⁰¹-, -CY⁰¹=CY⁰²-, -C≡C-, -CH=CH-COO-, -OCO-, -CH=CH- or a single bond,and
- R⁰¹, R⁰², Y⁰¹ and Y⁰²: have one of the respective meanings given above.
- X: is preferably -O-, -S-, -OCH₂-, -CH₂O-, -SCH₂-, -CH₂S-, -CF₂O-, -OCF₂-, -CF₂S-, -SCF₂-, -CH₂CH₂-, -CF₂CH₂-, -CH₂CF₂-, -CF₂CF₂-, -CH=N-, -N=CH-, -N=N-, -CH=CR⁰-, -CY⁰²=CY⁰²-, -C≡C- or a single bond, in particular -O-, -S-, -C≡C-, -CY⁰¹=CY⁰²- or a single bond, very preferably a group that is able to from a conjugated system, such as -C≡C- or -CY⁰¹=CY⁰²-, or a single bond.

Typical groups SG' are, for example, -(CH₂)ₚ-, -(CH₂CH₂O)_{q}-CH₂CH₂-, -CH₂CH₂-S-CH₂CH₂- or -CH₂CH₂-NH-CH₂CH₂- or -(SiR⁰R⁰⁰-O)ₚ-, with p being an integer from 2 to 12, q being an integer from 1 to 3 and R⁰, R⁰⁰ and the other parameters having the meanings given above.

Preferred groups SG' are ethylene, propylene, butylene, pentylene, hexylene, heptylene, octylene, nonylene, decylene, undecylene, dodecylene, octadecylene, ethyleneoxyethylene, methyleneoxybutylene, ethylene-thioethylene, ethylene-N-methyl-iminoethylene, 1-methylalkylene, ethenylene, propenylene and butenylene for example.

In another preferred embodiment SG' is a chiral group of formula I*': 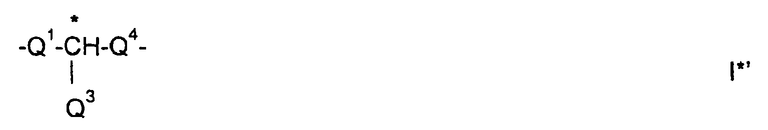 wherein
- Q¹ and Q³: have the meanings given in formula I*, and
- Q⁴: is an alkylene or alkylene-oxy group with 1 to 10 C atoms or a single bond, being different from Q¹,
with Q¹ being linked to the polymerisable group PG.

Further preferred are compounds with one or two groups PG-SG- wherein SG is a single bond.

In case of compounds with two groups PG-SG, each of the two polymerisable groups PG and the two spacer groups SG can be identical or different.

Preferred compounds of formula I' according to the present invention are selected from the following group of compounds of formulae I'-1 to I'-6 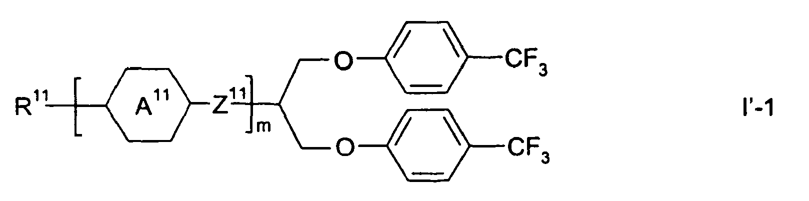 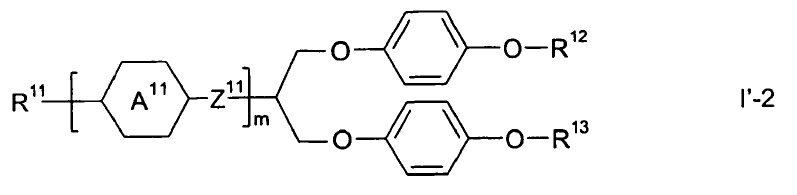 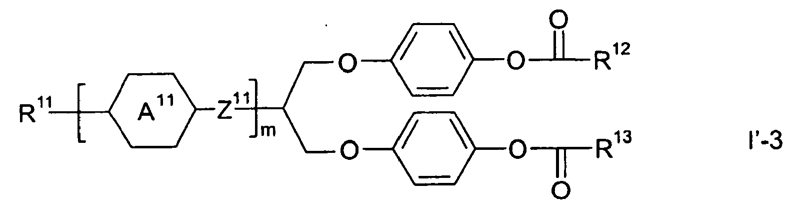 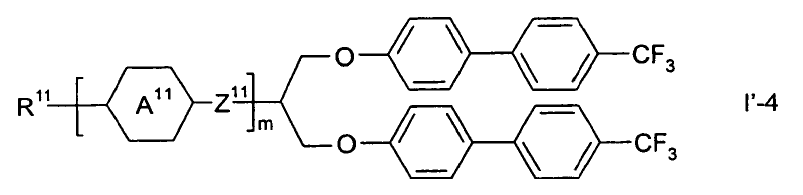 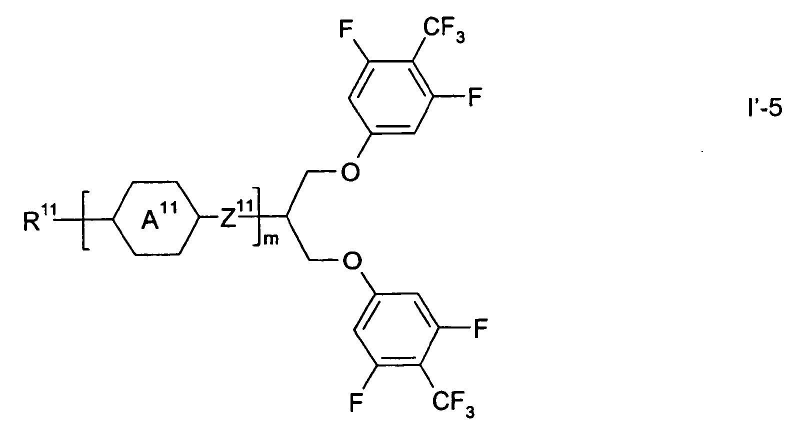 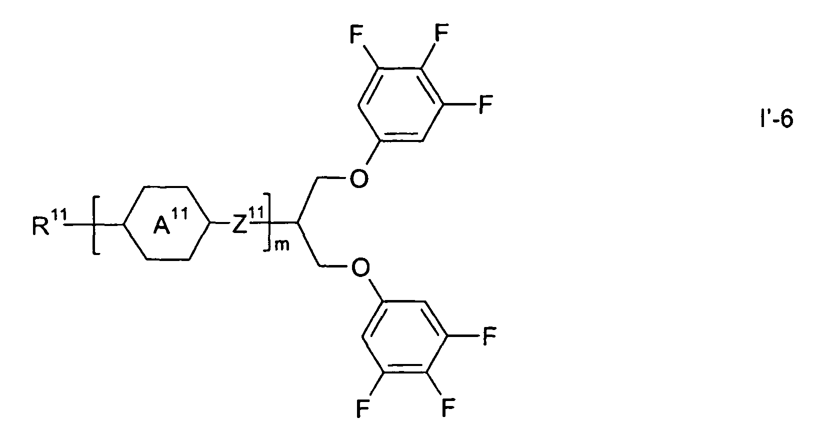 wherein the parameters have the respective meanings given above and preferably
- R¹² and R¹³: in formula I'-3 are alkenyl, most preferably vinyl.

Preferred compounds of formula I" according to the present invention are selected from the following group of compounds of formulae I"-1 to I"-6 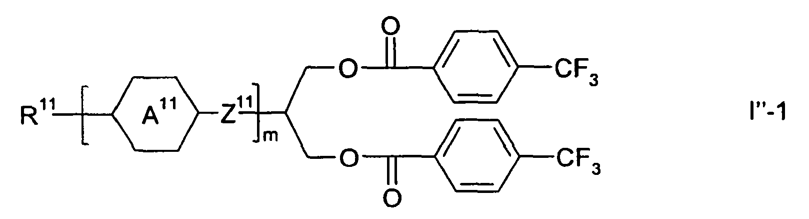 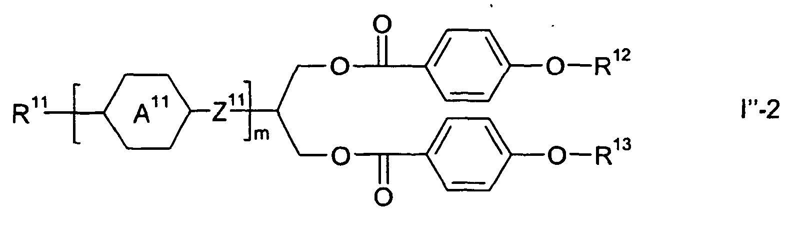 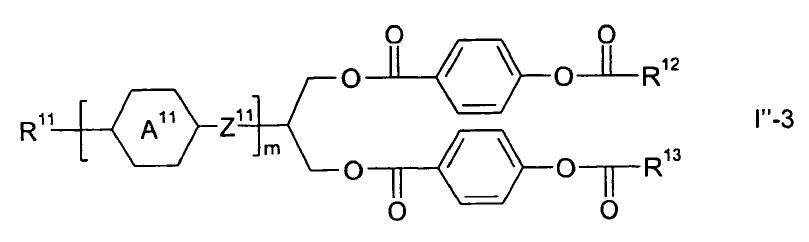 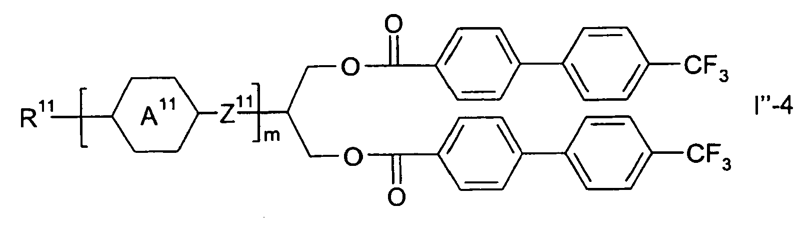 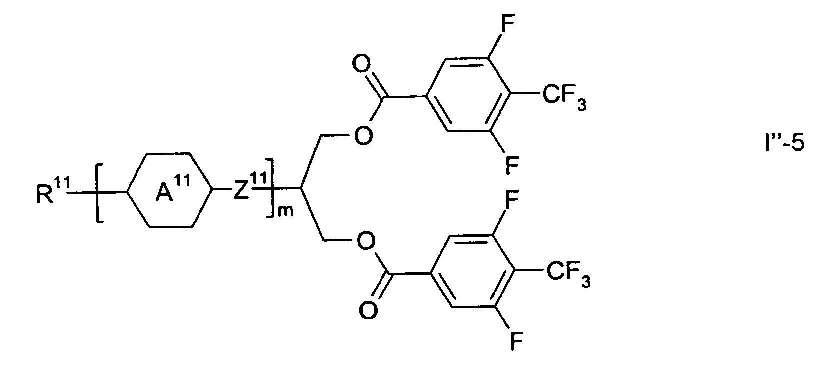 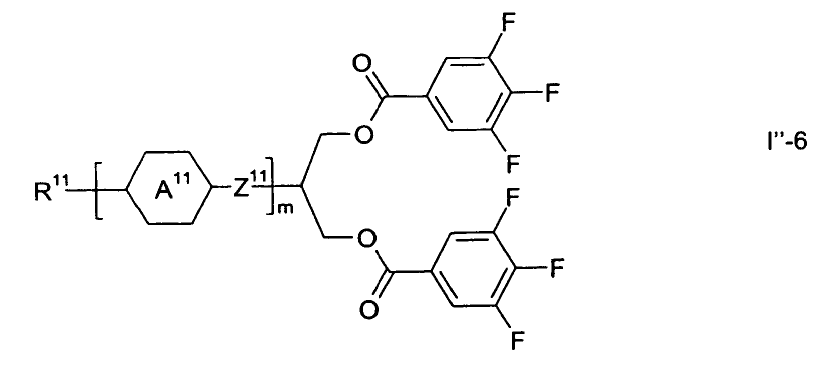 wherein the parameters have the respective meanings given above and preferably
- R¹² and R¹³: in formula I"-3 are alkenyl, most preferably vinyl.

Preferable compounds according to the instant invention are the following exemplary compounds

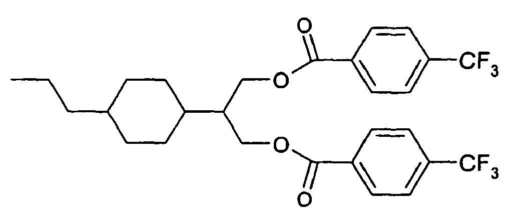

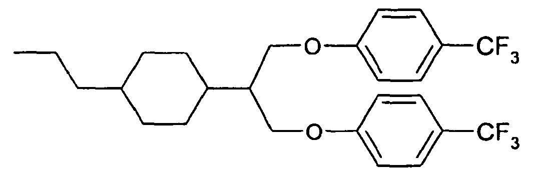

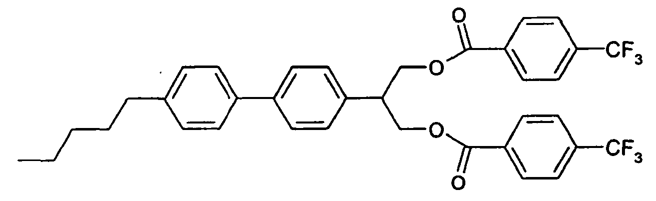

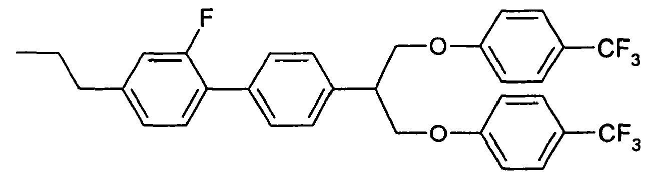

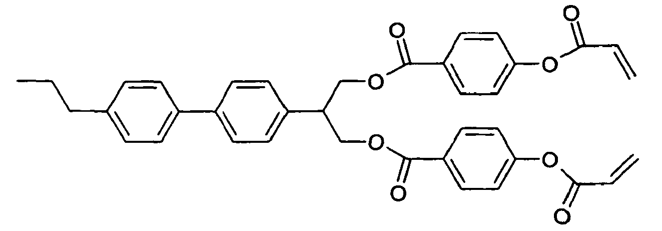

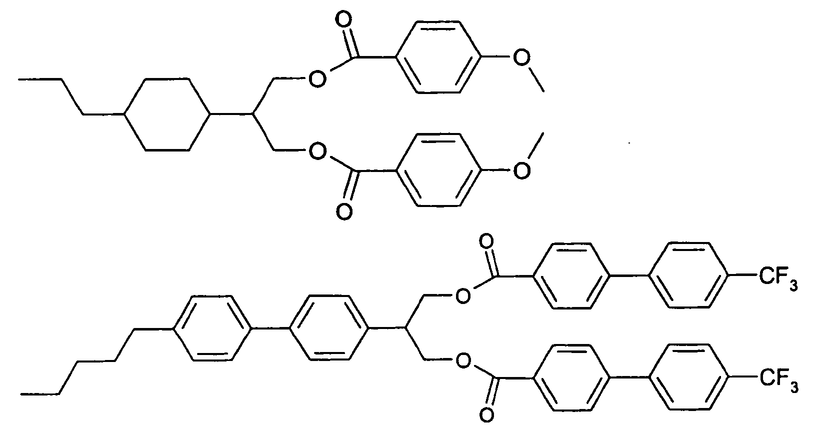

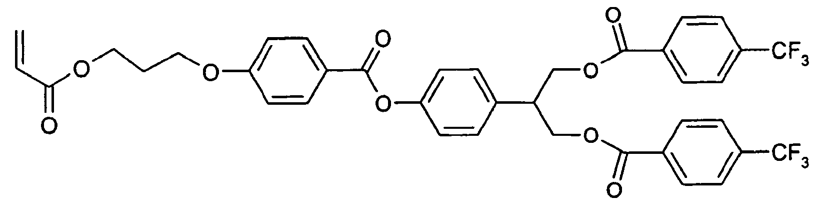

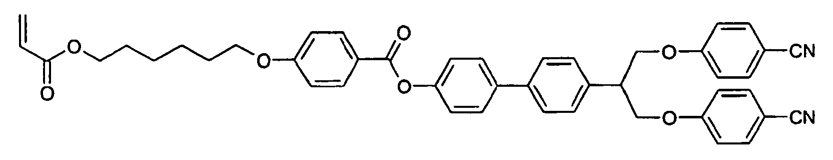

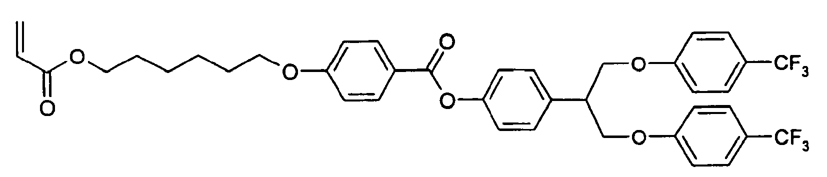

Preferably the liquid crystalline media according to the instant invention contain a component A comprising, preferably predominantly consisting of and most preferably entirely consisting of compounds of formula I.

The compounds of formula I are accessible by the usual methods known to the expert.

Compounds of formula I are beneficially prepared e.g. according to one of the following two exemplary reaction schemes (schemes I and II) and analogous synthetic routes. 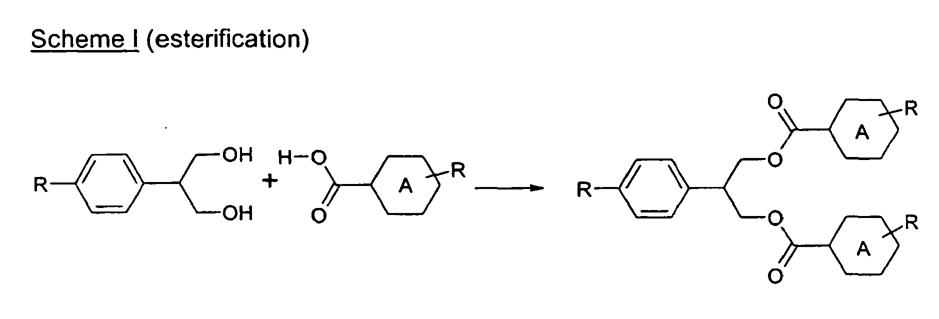 wherein the parameters have the respective meanings given above and preferably
- R,: in each occurrence independently of each other, is alkyl, F, CF₃, alkoxy or alkynyl, and 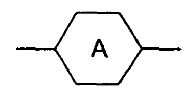 one of the meanings given above for 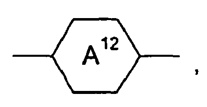
and the phenyl ring may optionally be substituted by one or more F-atoms 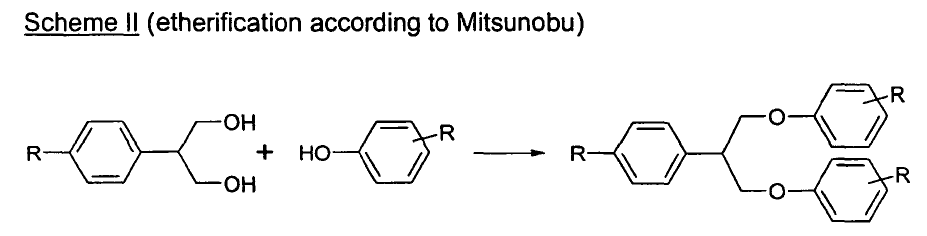 wherein the parameters have the respective meanings given above and preferably
- R,: in each occurrence independently of each other, is alkyl, F, CF₃, alkoxy or alkynyl
and the phenyl rings each, independently from each other, may optionally be substituted by one or more F-atoms.

Comprising in this application means in the context of compositions that the entity referred to, e.g. the medium or the component, contains the compound or compounds in question, preferably in a total concentration of 10 % or more and most preferably of 20 % or more.

Predominantly consisting, in this context, means that the entity referred to contains 80 % or more, preferably 90 % or more and most preferably 95 % or more of the compound or compounds in question.

Entirely consisting, in this context, means that the entity referred to contains 98 % or more, preferably 99 % or more and most preferably 100.0 % of the compound or compounds in question.

The concentration of the compounds according to the present application are contained in the media according to the present application preferably is in the range from 0.5% or more to 30% or less, more preferably in the range from 1% or more to 20% or less and most preferably in the range from 5% or more to 12% or less.

In a preferred embodiment the mesogenic modulation media according to the instant invention comprise
- a component A, preferably in a concentration of 1 % to 25 % by weight, comprising, preferably predominantly consisting of and most preferably entirely consisting of, one compound or more compounds of the formula I given above and
- optionally a dielectrically positive component B comprising, preferably predominantly consisting of and most preferably entirely consisting of one compound or of more compounds of formula II 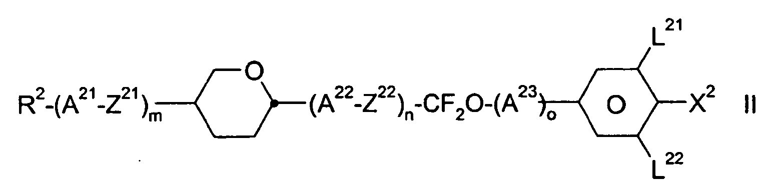 wherein
   - R²: has the meaning given under formula I for R¹¹,
   - A²¹, A²² and A²³: are, each independently of each other, 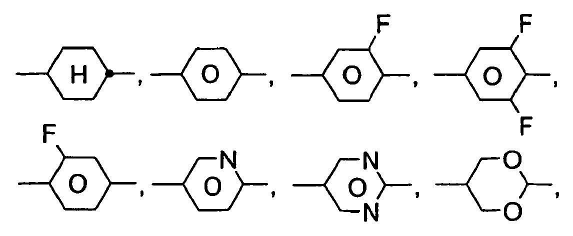 whereby each of A²¹ and A²² may have the same or a different meaning if present twice,
   - Z²¹ and Z²²: are, each independently of each other, a single bond, -(CH₂)₄)-, -CH₂CH₂-, -CF₂-CF₂-, -CF₂-CH₂-, -CH₂-CF₂-, -CH=CH-, -CF=CF-, -CF=CH-, -(CH₂)₃O-, -O(CH₂)₃-, -CH=CF-, -C≡C-, -CH₂O-, -OCH₂-, -CF₂O-, -OCF₂-, -CO-O- or -O-CO-, whereby each of Z²¹ and Z²² may have the same or a different meaning if present twice,
   - X²: is halogen, -CN, -NCS, -SF₅, -SO₂CF₃, alkyl, alkenyl, alkenyloxy or alkylalkoxy or alkoxy radical each mono- or polysubstituted by CN and/or halogen,
   - L²¹ and L²²: are, each independently of each other, H or F, and
   - m: is 0, 1 or 2,
   - n: is 0, 1, 2 or 3,
   - o: is 0, 1 or 2, preferably 0 or 1 and
   - m + n + o: is 3 or less, preferably 2 or less,
- optionally a component C, preferably in a concentration of 1 % to 25 % by weight, comprising, preferably predominantly consisting of and most preferably entirely consisting of one compound or of more compounds of formula III 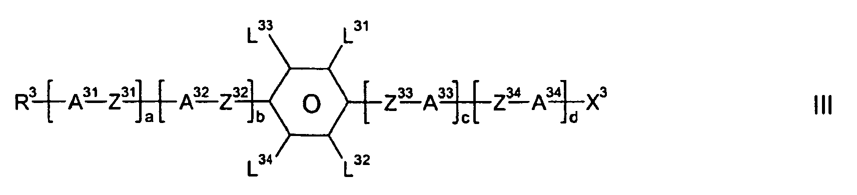 wherein
   - a, b, c and d: are each independently of each other 0, 1 or 2, whereby
   - a+b+c+d: is 4 or less,
   - A³¹, A³², A³³ and A³⁴: are, each independently of each other, 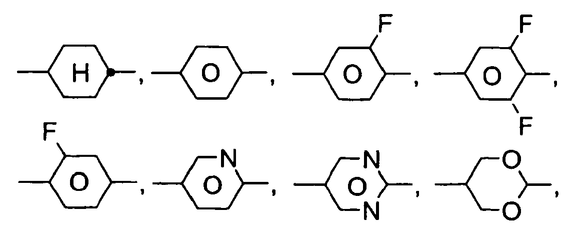 whereby each of A³¹, A³², A³³ and A³⁴ may have the same or a different meaning if present twice,
   - Z³¹, Z³², Z³³ and Z³⁴: are, each independently of each other, a single bond, -(CH₂)₄)-, -CH₂CH₂-, -CF₂-CF₂-, -CF₂-CH₂-, -CH₂-CF₂-, -CH=CH-, -CF=CF-, -CF=CH-, -(CH₂)₃O-, -O(CH₂)₃-, -CH=CF-, -C≡D-, -CH₂O-, -OCH₂-, -CF₂O-, -OCF₂-, -CO-O- or -O-CO-, whereby each of Z³¹, Z³², Z³³ and Z³⁴ may have the same or a different meaning if present twice,
   - R³: is an alkyl or alkoxy radical having from 1 to 15 carbon atoms, wherein one or more methylene groups of said alkyl or alkoxy radical may be replaced independently of each other by -O-, -S-, -SiR^{x}R^{y}-, -CH=CH-, -C≡D-, -CO-O- and/or -O-CO- such that oxygen and/or sulfur atoms are not linked directly to each other, said alkyl or alkoxy radical being unsubstituted or mono-substituted with a -CN group or mono- or poly-substituted with halogen, preferably R¹¹ is a straight-chain alkyl, alkoxy, alkenyl, alkenyloxy or -O-alkylene-O-alkyl radical with up to 10 carbon atoms, said radicals being unsubstituted or mono- or poly-substituted with halogen,
   - L³¹, L³², L³³ and L³⁴: are each independently of each other hydrogen, halogen, a CN group, an alkyl or alkoxy radical having from 1 to 15 carbon atoms wherein one or more methylene groups of said alkyl or alkoxy radical may be replaced independently of each other by -O-, -S-, -SiR^{x}R^{y}-, -CH=CH-, -C≡D-, -CO-O- and/or -O-CO- such that oxygen and/or sulfur atoms are not linked directly to each other, said alkyl or alkoxy radical being unsubstituted or mono-substituted with a -CN group or mono- or poly-substituted with halogen, with the proviso that at least one of L³¹, L³², L³³ and L³⁴ is not hydrogen,
   - X³: is F, Cl, CF₃, OCF₃, CN, NCS, -SF₅ or -SO₂-R²,
   - R^{x} and R^{y}: are independently of each other hydrogen or an alkyl radical having from 1 to 7 carbon atoms; preferably R^{x} and R^{y} are both methyl, ethyl, propyl or butyl, and
   - R^{z}: is an alkyl radical having from 1 to 7 carbon atoms, said alkyl radical being unsubstituted or mono- or polysubstituted with halogen; preferably R^{z} is CF₃, C₂F₅ or n-C₄F₉ and
- 1-20 % by weight of component D comprising one chiral compound or more chiral compounds with a HTP of ≥20 µm.

The inventive mixtures contain 1-25 wt.%, preferably 2-20 wt.% and most preferably 3-15 wt.% of component A.

Suitable chiral compounds of component D are those, which have an absolute value of the helical twisting power of 20 µm or more, preferably of 40 µm or more and most preferably of 60 µm or more. The HTP is measured in MLC-6260 at a temperature of 20°C.

The chiral component D comprises preferably one or more chiral compounds which have a mesogenic structure und exhibit preferably one or more mesophases themselves, particularly at least one cholesteric phase. Preferred chiral compounds being comprised in the chiral component D are, amongst others, well known chiral dopants like cholesteryl- nonanoate (CN), R/S-811, R/S-1011, R/S-2011, R/S-3011, R/S-4011, R/S-5011, CB-15 (Merck KGaA, Darmstadt, Germany). Preferred are chiral dopants having one or more chiral moieties and one or more mesogenic groups or having one or more aromatic or alicyclic moieties forming, together with the chiral moiety, a mesogenic group. More preferred are chiral moieties and mesogenic chiral compounds disclosed in DE 34 25 503, DE 35 34 777, DE 35 34 778, DE 35 34 779, DE 35 34 780, DE 43 42 280, EP 01 038 941 and DE 195 41 820 that disclosure is incorporated within this application by way of reference. Particular preference is given to chiral binaphthyl derivatives as disclosed in EP 01 111 954.2, chiral binaphthol derivatives as disclosed in WO 02/34739, chiral TADDOL derivatives as disclosed in WO 02/06265 as well as chiral dopants having at least one fluorinated linker and one end chiral moiety or one central chiral moiety as disclosed in WO 02/06196 and WO 02/06195.

The controlling medium of the present invention has a characteristic temperature, preferably a clearing point, in the range from about -30 °C to about 80 °C, especially up to about 55 °C.

The inventive mixtures contain one ore more (two, three, four or more) chiral compounds in the range of 1-25 wt.%, preferably 2-20 wt.%. Especially preferred are mixtures containing 3-15 wt.% of a chiral compound.

Preferred embodiments are indicated below:
- The medium comprises one, two or more compounds of formula I;
- Component B preferably contains besides one compound ore more compounds of formula II one ester compound or more ester compounds of the formula Z 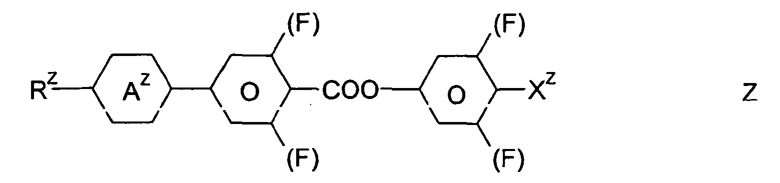 wherein R^{z} has the meaning given under formula I for R¹¹, 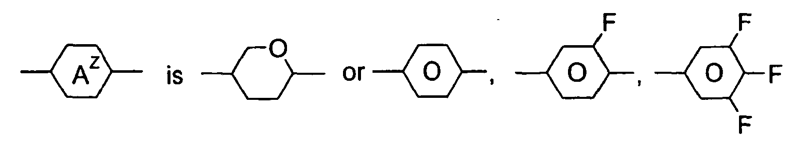
   - X^{z}: is F, Cl, CN, NCS, OCF₃, CF₃ or SF₅.
   wherein R^{z} has the meaning given under formula II for R².
   Especially preferred are mixtures containing 5 % to 35 %, preferably 10 % to 30 % and especially preferred 10 % to 20 % of compounds of formula Z.
- The component B preferably contains additionally one or more compounds of formula N 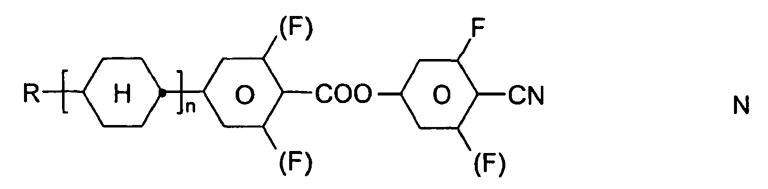 wherein
   - R: has the meaning given under formula I for R¹¹ and preferably is alkyl or Alkyl-C≡C,
   - "Alkyl": is alkyl with 1 to 7 C-atoms, preferably n-alkyl, and
   - n: is 0 or 1.
- The component B preferably additionally comprises one or more compounds selected from the group of ester compounds of formula E 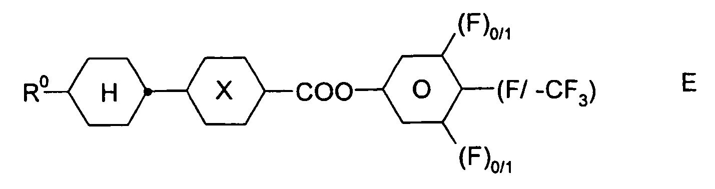 in which R⁰ has the meaning given for R¹¹ under formula I and preferably is alkyl and 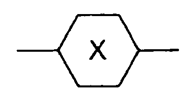 is 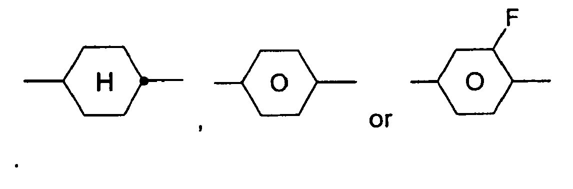
- The proportion of the compounds of formula E is preferably 10-30% by weight, in particular 15 % to 25 %.
- The medium preferably comprises one compound or more compounds selected from the group of formulae Q-1 and Q-2 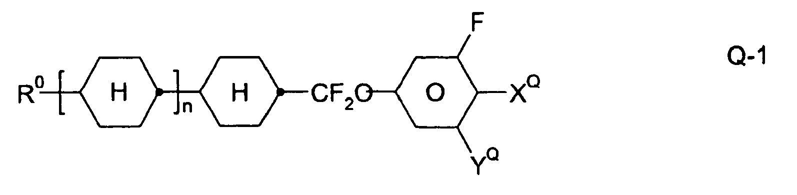 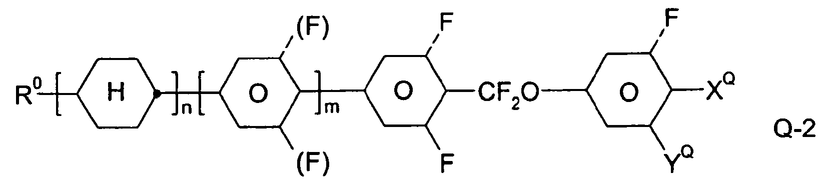 wherein R⁰ has the meaning given for R¹¹ under formula I and n and m are, independently of each other 0 or 1.
- The medium preferably comprises one compound or more compounds selected from the group of compounds of formula II in which R⁰ is methyl.
- The medium preferably comprises one dioxane compound, two or more dioxane compounds, preferably one dioxane compound or two dioxane compounds, selected from the group of formulae Dx-1 and Dx-2 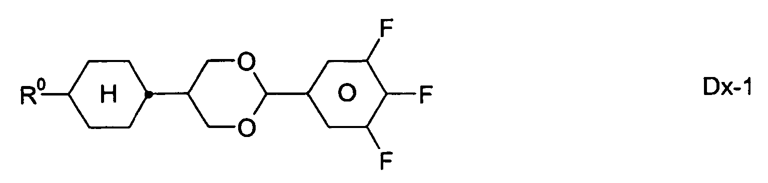 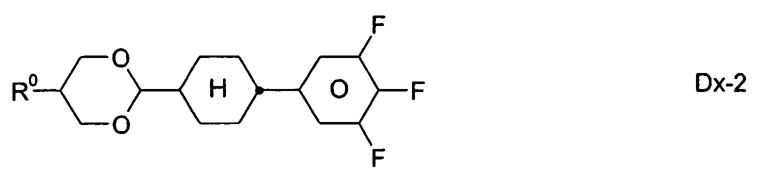 wherein R⁰ has the meaning given for R¹¹ under formula I.

It has been found that even a relatively small proportion of compounds of the formula I mixed with conventional liquid-crystal materials, but in particular with one or more compounds of the formulae II and III, results in a lower operating voltage and a broader operating temperature range. Preference is given, in particular, to mixtures which, besides one or more compounds of the formula I, comprise one or more compounds of the formula II, in particular compounds of the formula II in which X² is F, Cl, CN, NCS, CF₃ or OCF₃. The compounds of the formulae I to III are colourless, stable and readily miscible with one another and with other liquid-crystalline materials.

The optimum mixing ratio of the compounds of the formulae I and II and III depends substantially on the desired properties, on the choice of the components of the formulae I, II and/or III, and on the choice of any other components that may be present. Suitable mixing ratios within the range given above can easily be determined from case to case.

The total amount of compounds of the formulae I to III in the mixtures according to the invention is not crucial. The mixtures can therefore comprise one or more further components for the purposes of optimisation of various properties. However, the observed effect on the operating voltage and the operating temperature range is generally greater, the higher the total concentration of compounds of the formulae I to III.

In a particularly preferred embodiment, the media according to the invention comprise compounds of the formula III which X³ is F, OCF₃, OCHF₂, OCH=CF₂, OCF=CF₂ or OCF₂-CF₂H. A favourable synergistic effect with the compounds of the formula I results in particularly advantageous properties. In particular, mixtures comprising compounds of formula I and of formula II and of formula III are distinguished by their low operating voltages.

The individual compounds of the formulae II to III which can be used in the media according to the invention are either known or can be prepared analogously to the known compounds.

The construction of the MLC display according to the invention from polarisers, electrode base plates and surface-treated electrodes corresponds to the conventional construction for displays of this type. The term conventional construction is broadly drawn here and also covers all derivatives and modifications of the MLC display, in particular including matrix display elements based on poly-Si TFT or MIM, however, particularly preferred are displays, which have electrodes on just one of the substrates, i.e. so called interdigital electrodes, as those used in IPS displays, preferably in one of the established structures.

A significant difference between the displays according to the invention and the conventional displays based on the twisted nematic cell consists, however, in the choice of the liquid-crystal parameters of the liquid-crystal layer.

The media according to the invention are prepared in a manner conventional per se. In general, the components are dissolved in one another, advantageously at elevated temperature. By means of suitable additives, the liquid-crystalline phases in accordance with the invention can be modified in such a way that they can be used in all types of liquid crystal display elements that have been disclosed hitherto. Additives of this type are known to the person skilled in the art and are described in detail in the literature (H. Kelker and R. Hatz, Handbook of Liquid Crystals, Verlag Chemie, Weinheim, 1980). For example, pleochroic dyes can be added for the preparation of coloured guest-host systems or substances can be added in order to modify the dielectric anisotropy, the viscosity and/or the alignment of the nematic phases. Furthermore, stabilisers and antioxidants can be added.

The mixtures according to the invention are suitable for TN, STN, ECB and IPS applications and isotropic switching mode (ISM) applications. Hence, there use in an electro-optical device and an electro-optical device containing liquid crystal media comprising at least one compound according to the invention are subject matters of the present invention.

The inventive mixtures are highly suitable for devices which operate in an optically isotropic state. The mixtures of the invention are surprisingly found to be highly suitable for the respective use.

Electro-optical devices that are operated or operable in an optically isotropic state recently have become of interest with respect to video, TV, and multi-media applications. This is because conventional liquid crystal displays utilizing electro-optical effects based on the physical properties of liquid crystals exhibit a rather high switching time which is undesired for said applications. Furthermore most of the conventional displays show a significant viewing angle dependence of contrast that in turn makes necessary measures to compensate this undesired property.

With regard to devices utilizing electro-optical effects in an isotropic state the German Patent Application DE 102 17 273 A1 for example discloses light controlling (light modulation) elements in which the mesogenic controlling medium for modulation is in the isotropic phase at the operating temperature. These light controlling elements have a very short switching time and a good viewing angle dependence of contrast. However, the driving or operating voltages of said elements are very often unsuitably high for some applications.

German Patent Application DE 102 41 301 yet unpublished describes specific structures of electrodes allowing a significant reduction of the driving voltages. However, these electrodes make the process of manufacturing the light controlling elements more complicated.

Furthermore, the light controlling elements, for example, disclosed in both DE 102 17 273 A1 and DE 102 41 301 show a significant temperature dependence. The electro-optical effect that can be induced by the electrical field in the controlling medium being in an optical isotropic state is most pronounced at temperatures close to the clearing point of the controlling medium. In this range the light controlling elements have the lowest values of their characteristic voltages and, thus, require the lowest operating voltages. As temperature increases the characteristic voltages and hence the operating voltages increase remarkably. Typical values of the temperature dependence are in the range from about a few volts per centigrade up to about ten or more volts per centigrade. While DE 102 41 301 describes various structures of electrodes for devices operable or operated in the isotropic state, DE 102 17 273 A1 discloses isotropic media of varying composition that are useful in light controlling elements operable or operated in the isotropic state. The relative temperature dependence of the threshold voltage in these light controlling elements is at a temperature of 1 centigrade above the clearing point in the range of about 50%/centigrade. That temperature dependence decreases with increasing temperature so that it is at a temperature of 5 centigrade above the clearing point of about 10%/centigrade. However, for many practical applications of displays utilizing said light controlling elements the temperature dependence of the electro-optical effect is too high. To the contrary, for practical uses it is desired that the operating voltages are independent from the operating temperature over a temperature range of at least some centigrades, preferably of about 5 centigrades or more, even more preferably of about 10 centigrades or more and especially of about 20 centigrades or more.

Now it has been found that the use of the inventive mixtures are highly suitable as controlling media in the light controlling elements as described above and in DE 102 17 273 A1, DE 102 41 301 and DE 102 536 06 and broaden the temperature range in which the operating voltages of said electro-optical operates. In this case the optical isotropic state or the blue phase is almost completely or completely independent from the operating temperature.

This effect is even more distinct if the mesogenic controlling media exhibit at least one so-called "blue phase" as described in yet unpublished WO 2004/046 805. Liquid crystals having an extremely high chiral twist may have one or more optically isotropic phases. If they have a respective cholesteric pitch, these phases might appear bluish in a cell having a sufficiently large cell gap. Those phases are therefore also called "blue phases" (Gray and Goodby, "Smectic Liquid Crystals, Textures and Structures", Leonhard Hill, USA, Canada (1984)). Effects of electrical fields on liquid crystals existing in a blue phase are described for instance in H.S. Kitzerow, "The Effect of Electric Fields on Blue Phases", Mol. Cryst. Liq. Cryst. (1991), Vol. 202, p. 51-83, as well as the three types of blue phases identified far, nameiy BP i, BP ii, and BP III, that may be observed in field-free liquid crystals. It is noteworthy, that if the liquid crystal exhibiting a blue phase or blue phases is subjected to an electrical field, further blue phases or other phases different from the blue phases I, II and III might appear.

The inventive mixtures can be used in an electro-optical light controlling element which comprises
- one or more, especially two substrates;
- an assembly of electrodes;
- one or more elements for polarizing the light; and
- said controlling medium;
whereby said light controlling element is operated (or operable) at a temperature at which the controlling medium is in an optically isotropic phase when it is in a non-driven state.

The controlling medium of the present invention has a characteristic temperature, preferably a clearing point, in the range from about -30 °C to about 80 °C, especially up to about 55 °C.

The operating temperature of the light controlling elements is preferably above the characteristic temperature of the controlling medium said temperature being usually the transition temperature of the controlling medium to the blue phase; generally the operating temperature is in the range of about 0.1 ° to about 50 °, preferably in the range of about 0.1 ° to about 10 ° above said characteristic temperature. It is highly preferred that the operating temperature is in the range from the transition temperature of the controlling medium to the blue phase up to the transition temperature of the controlling medium to the isotropic phase which is the clearing point. The light controlling elements, however, may also be operated at temperatures at which the controlling medium is in the isotropic phase.

(For the purposes of the present invention the term "characteristic temperature" is defined as follows:
- If the characteristic voltage as a function of temperature has a minimum, the temperature at this minimum is denoted as characteristic temperature.
- If the characteristic voltage as a function of temperature has no minimum and if the controlling medium has one or more blue phases, the transition temperature to the blue phase is denoted as characteristic temperature; in case there are more than one blue phase, the lowest transition temperature to a blue phase is denoted as characteristic temperature.
- If the characteristic voltage as a function of temperature has no minimum and if the controlling medium has no blue phase, the transition temperature to the isotropic phase is denoted as characteristic temperature.)

In the context of the present invention the term "alkyl" means, as long as it is not defined in a different manner elsewhere in this description or in the claims, straight-chain and branched hydrocarbon (aliphatic) radicals with 1 to 15 carbon atoms. The hydrocarbon radicals may be unsubstituted or substituted with one or more substituents being independently selected from the group consisting of F, Cl, Br, I or CN.

The dielectrics may also comprise further additives known to the person skilled in the art and described in the literature. For example, 0 to 5% of pleochroic dyes, antioxidants or stabilizers can be added.

C denotes a crystalline phase, S a smectic phase, S_{c} a smectic C phase, N a nematic phase, I the isotropic phase and BP the blue phase.

Vₓ denotes the voltage for X% transmission. Thus e.g. V₁₀ denotes the voltage for 10% transmission and V₁₀₀ denotes the voltage for 100% transmission (viewing angle perpendicular to the plate surface). tₒₙ (respectively τₒₙ) denotes the switch-on time and t_{off} (respectively τ_{off}) the switch-off time at an operating voltage corresponding the value of V₁₀₀, respectively of Vₘₐₓ.

Δn denotes the optical anisotropy. Δε denotes the dielectric anisotropy (Δε = ε_{∥ -} ε_{┴}, where ε_{∥} denotes the dielectric constant parallel to the longitudinal molecular axes andε_{∥} denotes the dielectric constant perpendicular thereto). The electro-optical data are measured in a TN cell at the 1^{st} minimum of transmission (i.e. at a (d · Δn) value of 0.5 µm) at 20°C, unless expressly stated otherwise. The optical data are measured at 20°C, unless expressly stated otherwise.

Optionally, the light modulation media according to the present invention can comprise further liquid crystal compounds in order to adjust the physical properties. Such compounds are known to the expert. Their concentration in the media according to the instant invention is preferably 0 % to 30 %, more preferably 0 % to 20 % and most preferably 5 % to 15 %.

Preferably inventive media have a range of the blue phase or, in case of the occurrence of more than one blue phase, a combined range of the blue phases, with a width of 9° or more, preferably of 10° or more, more preferably of 15° or more and most preferably of 20° or more.

In a preferred embodiment this phase range at least from 10°C to 30°C, most preferably at least from 10°C to 40°C and most preferably at least from 0°C to 50°C, wherein at least means, that preferably the phase extends to temperatures below the lower limit and at the same time, that it extends to temperatures above the upper limit.

In another preferred embodiment this phase range at least from 20°C to 40°C, most preferably at least from 30°C to 80°C and most preferably at least from 30°C to 90°C. This embodiment is particularly suited for displays with a strong back light, dissipating energy and thus heating the display.

In the present application the term dielectrically positive compounds describes compounds with Δε > 1,5, dielectrically neutral compounds are compounds with -1,5 ≤ Δε ≤ 1,5 and dielectrically negative compounds are compounds with Δε < -1,5. The same holds for components. Δε is determined at 1 kHz and 20 °C. The dielectrical anisotropies of the compounds is determined from the results of a solution of 10 % of the individual compounds in a nematic host mixture. The capacities of these test mixtures are determined both in a cell with homeotropic and with homogeneous alignment. The cell gap of both types of cells is approximately 20 µm. The voltage applied is a rectangular wave with a frequency of 1 kHz and a root mean square value typically of 0.5 V to 1.0 V, however, it is always selected to be below the capacitive threshold of the respective test mixture.

For dielectrically positive compounds the mixture ZLI-4792 and for dielectrically neutral, as well as for dielectrically negative compounds, the mixture ZLI-3086, both of Merck KGaA, Germany are used as host mixture, respectively. The dielectric permittivities of the compounds are determined from the change of the respective values of the host mixture upon addition of the compounds of interest and are extrapolated to a concentration of the compounds of interest of 100 %.

Components having a nematic phase at the measurement temperature of 20 °C are measured as such, all others are treated like compounds.

The term threshold voltage refers in the instant application to the optical threshold and is given for 10 % relative contrast (V₁₀) and the term saturation voltage refers to the optical saturation and is given for 90 % relative contrast (V₉₀) both, if not explicitly stated otherwise. The capacitive threshold voltage (V₀, also called Freedericksz-threshold V_{Fr}) is only used if explicitly mentioned.

The ranges of parameters given in this application are all including the limiting values, unless explicitly stated otherwise.

Throughout this application, unless explicitly stated otherwise, all concentrations are given in mass percent and relate to the respective complete mixture, all temperatures are given in degrees centigrade (Celsius) and all differences of temperatures in degrees centigrade. All physical properties have been and are determined according to "Merck Liquid Crystals, Physical Properties of Liquid Crystals", Status Nov. 1997, Merck KGaA, Germany and are given for a temperature of 20 °C, unless explicitly stated otherwise. The optical anisotropy (Δn) is determined at a wavelength of 589.3 nm. The dielectric anisotropy (Δε) is determined at a frequency of 1 kHz. The threshold voltages, as well as all other electro-optical properties have been determined with test cells prepared at Merck KGaA, Germany. The test cells for the determination of Δε had a cell gap of 22 µm. The electrode was a circular ITO electrode with an area of 1.13 cm² and a guard ring. The orientation layers were lecithin for homeotropic orientation (ε_{∥}) and polyimide AL-1 054 from Japan Synthetic Rubber for homogenous orientation (ε_{⊥}). The capacities were determined with a frequency response analyser Solatron 1260 using a sine wave with a voltage of 0.3 or 0.1 Vᵣₘₛ. The light used in the electro-optical measurements was white light. The set up used was a commercially available equipment of Otsuka, Japan. The characteristic voltages have been determined under perpendicular observation. The threshold voltage (V₁₀), mid-grey voltage (V₅₀) and saturation voltage (V₉₀) have been determined for 10 %, 50 % and 90 % relative contrast, respectively.

The mesogenic modulation material has been filled into an electro optical test cell prepared at the respective facility of Merck KGaA. The test cells had inter-digital electrodes on one substrate side. The electrode width was 10 µm, the distance between adjacent electrodes was 10 µm and the cell gap was also 10 µm. This test cell has been evaluated electro-optically between crossed polarisers.

At low temperatures, the filled cells showed the typical texture of a chiral nematic mixture, with an optical transmission between crossed polarisers without applied voltage. Upon heating, at a first temperature (T₁) the mixtures turned optically isotropic, being dark between the crossed polarisers. This indicated the transition from the chiral nematic phase to the blue phase at that temperature. Up to a second temperature (T₂) the cell showed an electro-optical effect under applied voltage, typically of some tens of volts, a certain voltage in that range leading to a maximum of the optical transmission. Typically at a higher temperature the voltage needed for a visible electro-optical effect increased strongly, indicating the transition from the blue phase to the isotropic phase at this second temperature (T₂).

The temperature range (ΔT(BP)), where the mixture can be used electro-optically in the blue phase most beneficially has been identified as ranging from T₁ to T₂. This temperature range (ΔT(BP)) is the temperature range given in the examples of this application. The electro-optical displays can also be operated at temperatures beyond this range, i.e. at temperatures above T₂, albeit only at significantly increased operation voltages.

The liquid crystal media according to the present invention can contain further additives and chiral dopants in usual concentrations. The total concentration of these further constituents is in the range of 0 % to 10 %, preferably 0.1 % to 6 %, based in the total mixture. The concentrations of the individual compounds used each are preferably in the range of 0.1 to 3 %. The concentration of these and of similar additives is not taken into consideration for the values and ranges of the concentrations of the liquid crystal components and compounds of the liquid crystal media in this application.

The inventive liquid crystal media according to the present invention consist of several compounds, preferably of 3 to 30, more preferably of 5 to 20 and most preferably of 6 to 14 compounds. These compounds are mixed in conventional way. As a rule, the required amount of the compound used in the smaller amount is dissolved in the compound used in the greater amount. In case the temperature is above the clearing point of the compound used in the higher concentration, it is particularly easy to observe completion of the process of dissolution. It is, however, also possible to prepare the media by other conventional ways, e. g. using so called pre-mixtures, which can be e. g. homologous or eutectic mixtures of compounds or using so called multi-bottle-systems, the constituents of which are ready to use mixtures themselves.

By addition of suitable additives, the liquid crystal media according to the instant invention can be modified in such a way, that they are usable in all known types of liquid crystal displays, either using the liquid crystal media as such, like TN-, TN-AMD, ECB-, VAN-AMD and in particular in composite systems, like PDLD-, NCAP- and PN-LCDs and especially in HPDLCs.

The melting point T(C,N), the transition from the smectic (S) to the nematic (N) phase T(S,N) and the clearing point T (N,I) of the liquid crystals are given in degrees centigrade.

In the present application and especially in the following examples, the structures of the liquid crystal compounds are represented by abbreviations also called acronyms. The transformation of the abbreviations into the corresponding structures is straight forward according to the following two tables A and B. All groups CₙH₂ₙ₊₁ and CₘH₂ₘ₊₁ are straight chain alkyl groups with n respectively m C-atoms. The interpretation of table B is self evident. Table A does only list the abbreviations for the cores of the structures. The individual compounds are denoted by the abbreviation of the core followed by a hyphen and a code specifying the substituents R¹, R², L¹ and L² follows:

| Code for R¹, R², L¹, L² | R¹ | R² | L¹ | L² |
|---|---|---|---|---|
| nm | CₙH₂ₙ₊₁ | CₘH₂ₘ₊₁ | H | H |
| nOm | CₙH₂ₙ₊₁ | OCₘH₂ₘ₊₁ | H | H |
| nO.m | OCₙH₂ₙ₊₁ | CₘH₂ₘ₊₁ | H | H |
| n | CₙH₂ₙ₊₁ | CN | H | H |
| nN.F | CₙH₂ₙ₊₁ | CN | H | F |
| nN.F.F | CₙH₂ₙ₊₁ | CN | F | F |
| nF | CₙH₂ₙ₊₁ | F | H | H |
| nF.F | CₙH₂ₙ₊₁ | F | H | F |
| nF.F.F | CₙH₂ₙ₊₁ | F | F | F |
| nOF | OCₙH₂ₙ₊₁ | F | H | H |
| nCl | CₙH₂ₙ₊₁ | Cl | H | H |
| nCl.F | CₙH₂ₙ₊₁ | Cl | H | F |
| nCl.F.F | CₙH₂ₙ₊₁ | Cl | F | F |
| nCF₃ | CₙH₂ₙ₊₁ | CF₃ | H | H |
| nOCF₃ | CₙH₂ₙ₊₁ | OCF₃ | H | H |
| nOCF₃.F | CₙH₂ₙ₊₁ | OCF₃ | H | F |
| nOCF₃.F.F | CₙH₂ₙ₊₁ | OCF₃ | F | F |
| nOCF₂ | CₙH₂ₙ₊₁ | OCHF₂ | H | H |
| nOCF₂.F | CₙH₂ₙ₊₁ | OCHF₂ | H | F |
| nOCF₂.F.F | CₙH₂ₙ₊₁ | OCHF₂ | F | F |
| nS | CₙH₂ₙ₊₁ | NCS | H | H |
| nS.F | CₙH₂ₙ₊₁ | NCS | H | F |
| nS.F.F | CₙH₂ₙ₊₁ | NCS | F | F |
| rVsN | CᵣH₂ᵣ₊₁-CH=CH-CₛH₂ₛ- | CN | H | H |
| rEsN | CᵣH₂ᵣ₊₁-O-CₛH₂ₛ- | CN | H | H |
| nAm | CₙH₂ₙ₊₁ | COOCₘH₂ₘ₊₁ | H | H |
| nF.Cl | CₙH₂ₙ₊₁ | Cl | H | F |

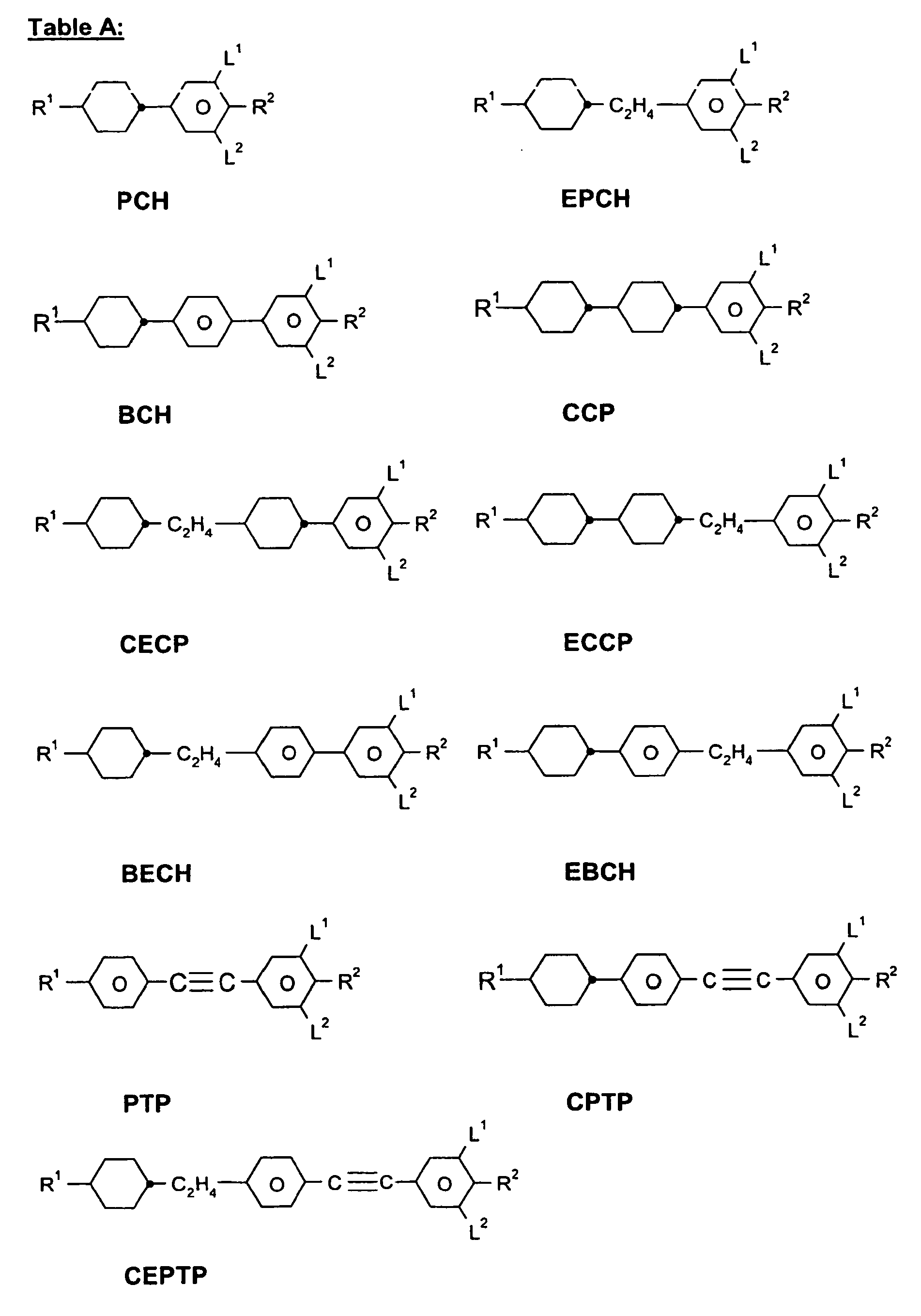

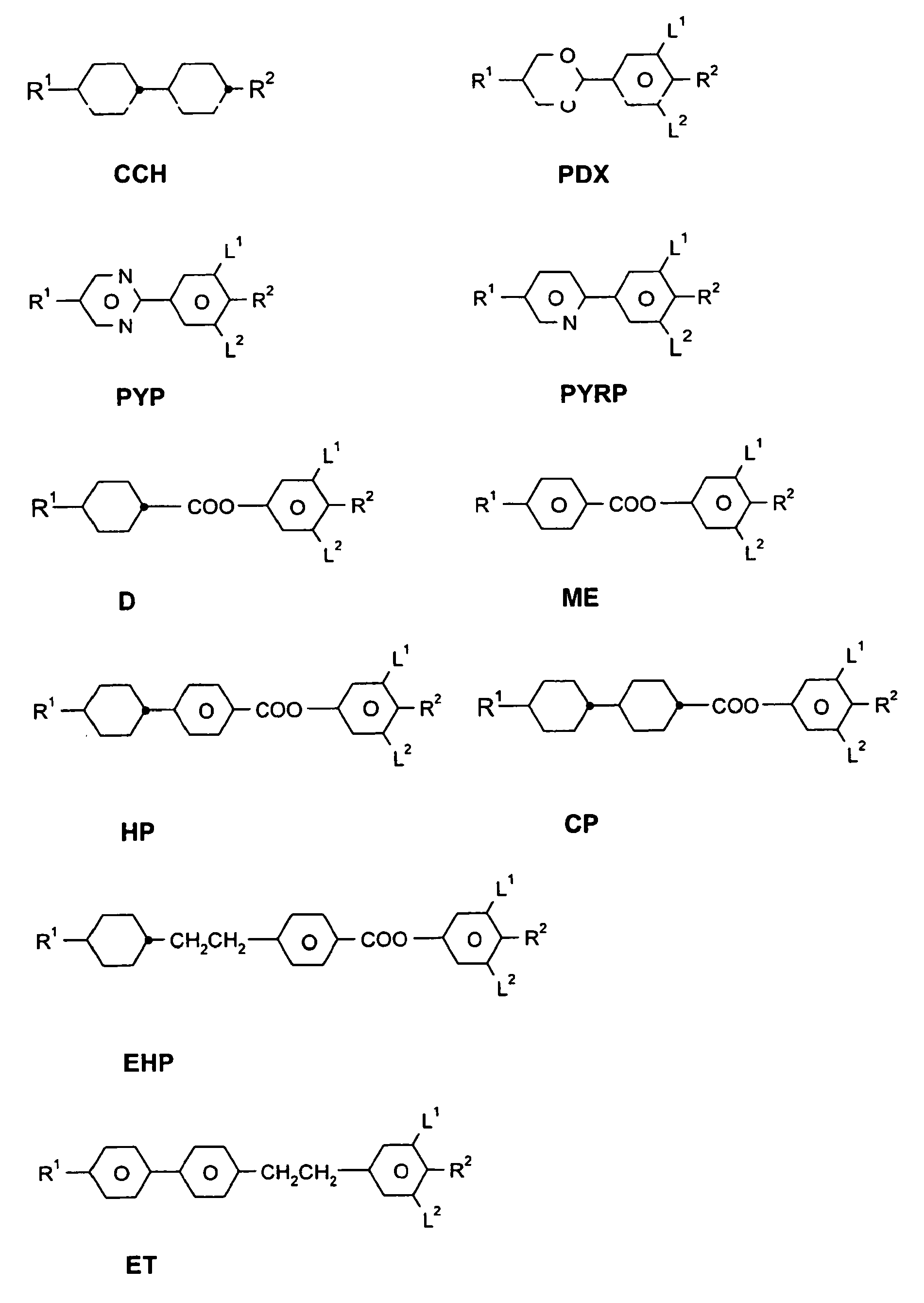

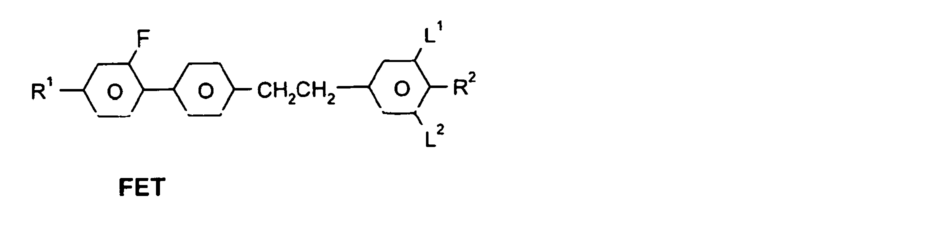

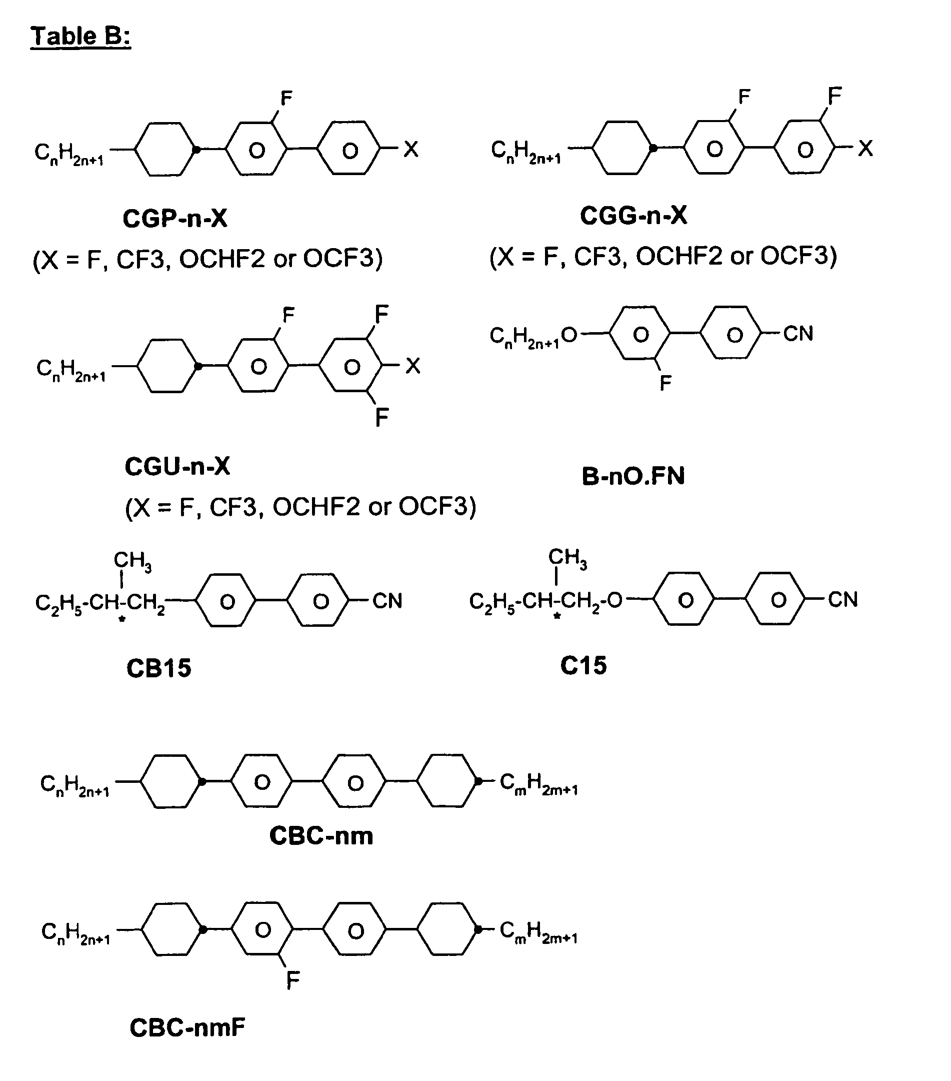

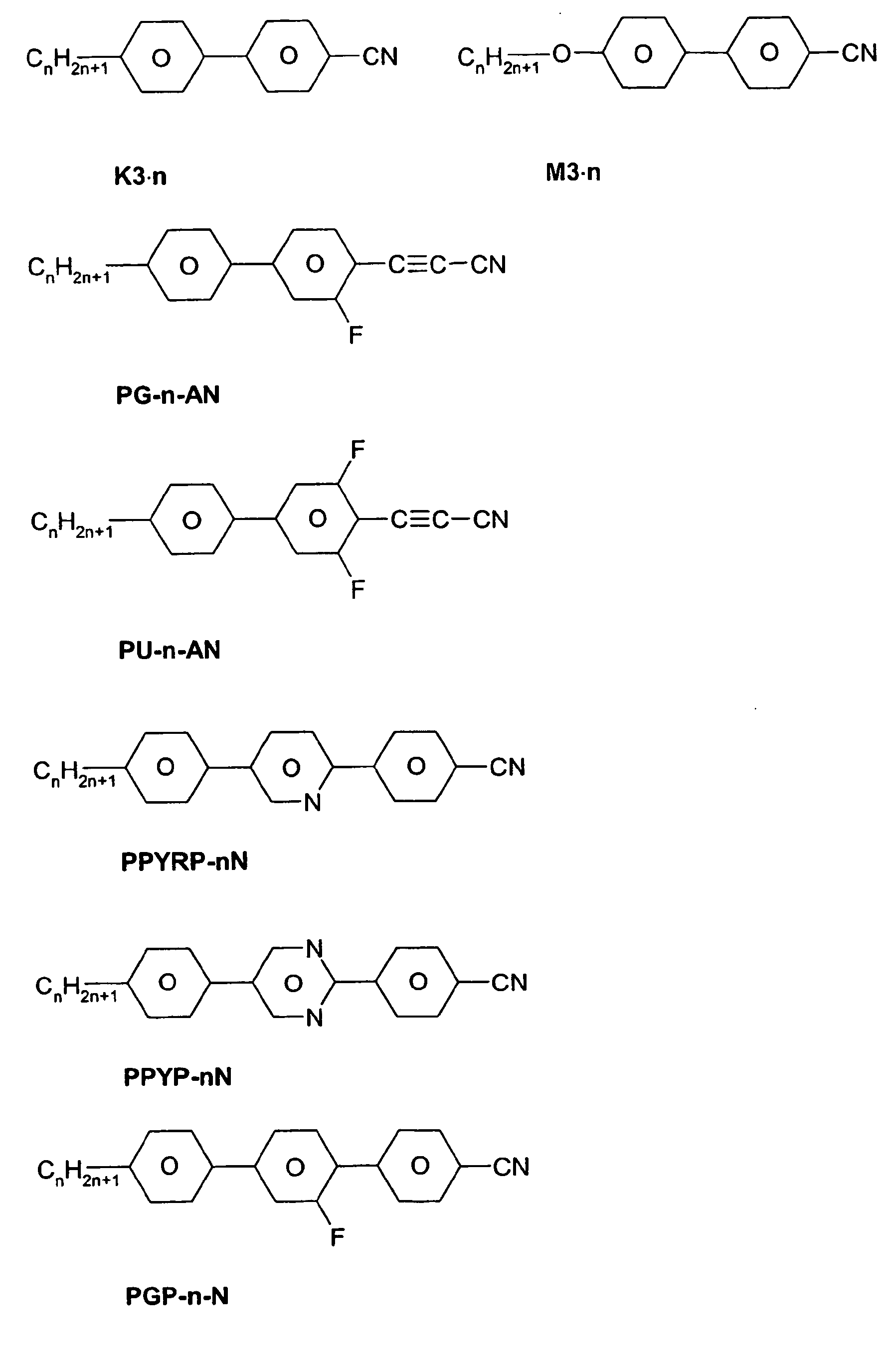

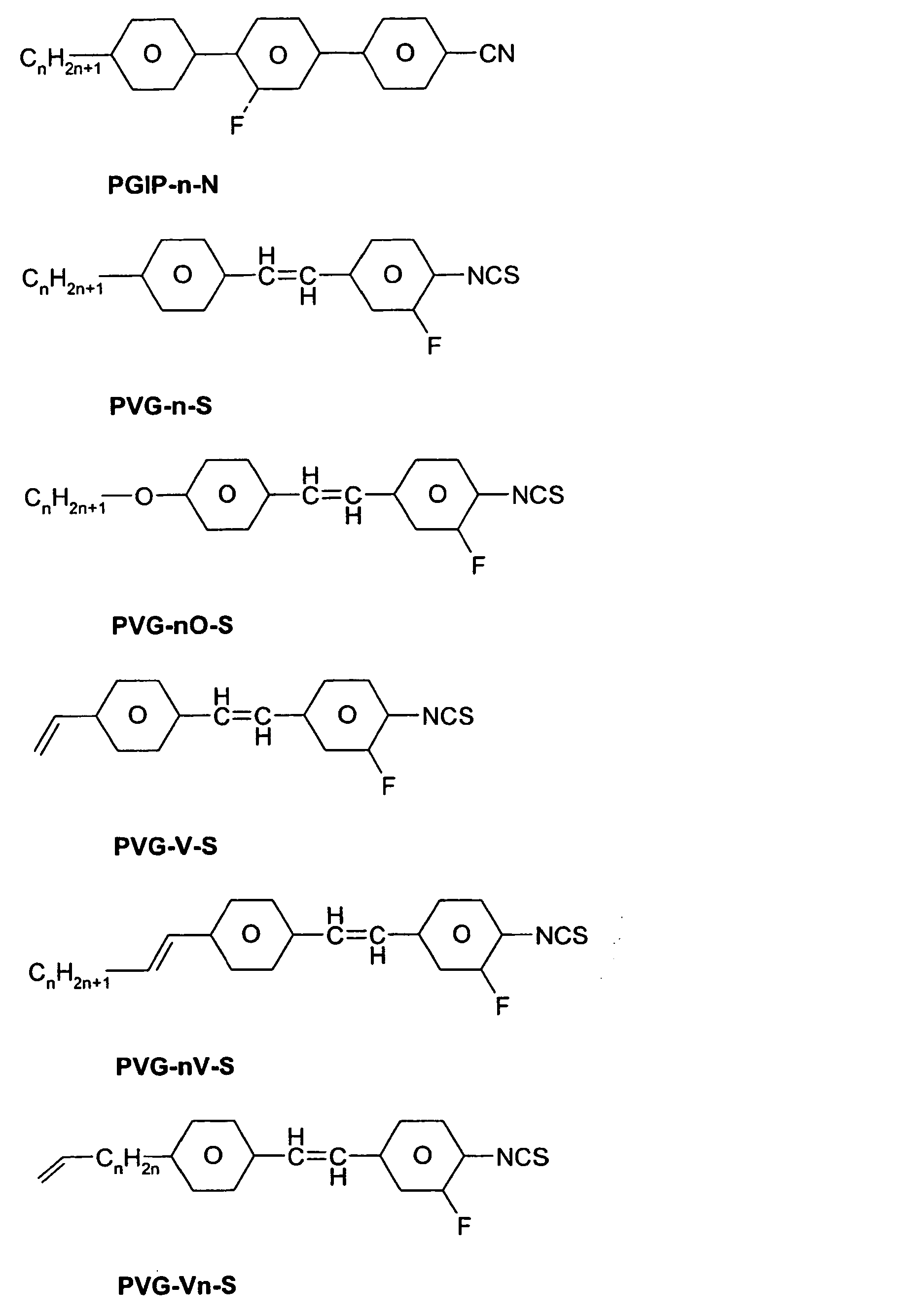

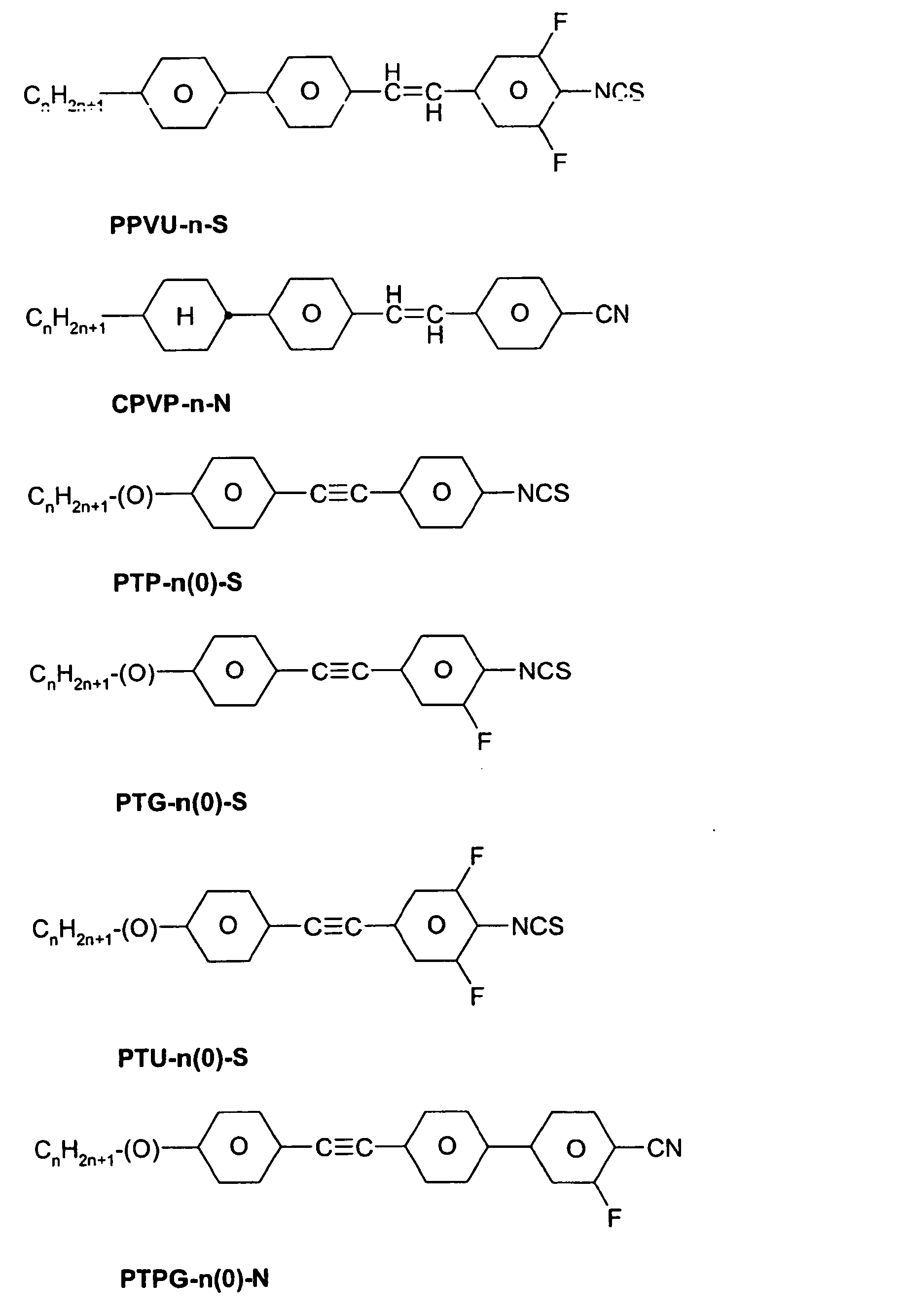

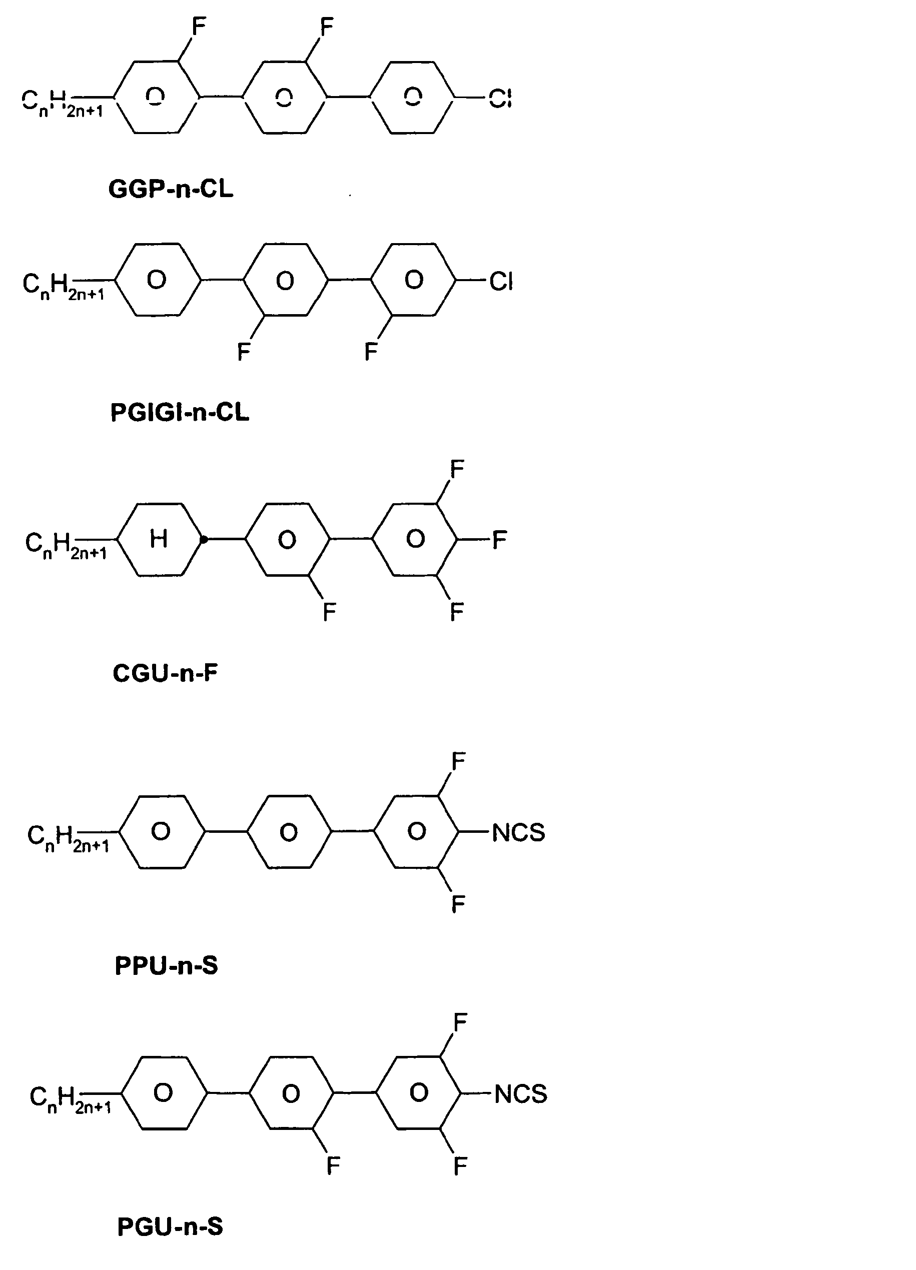

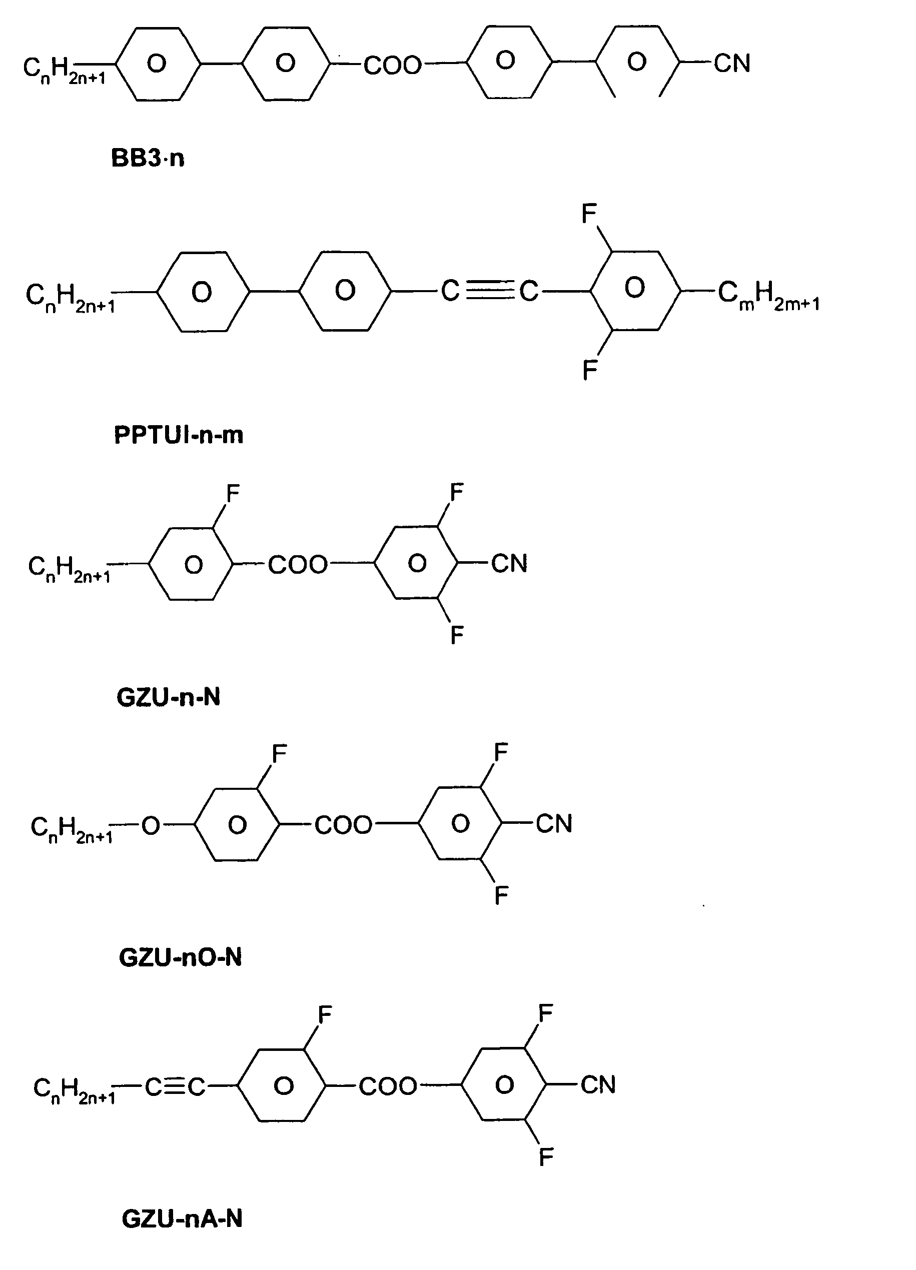

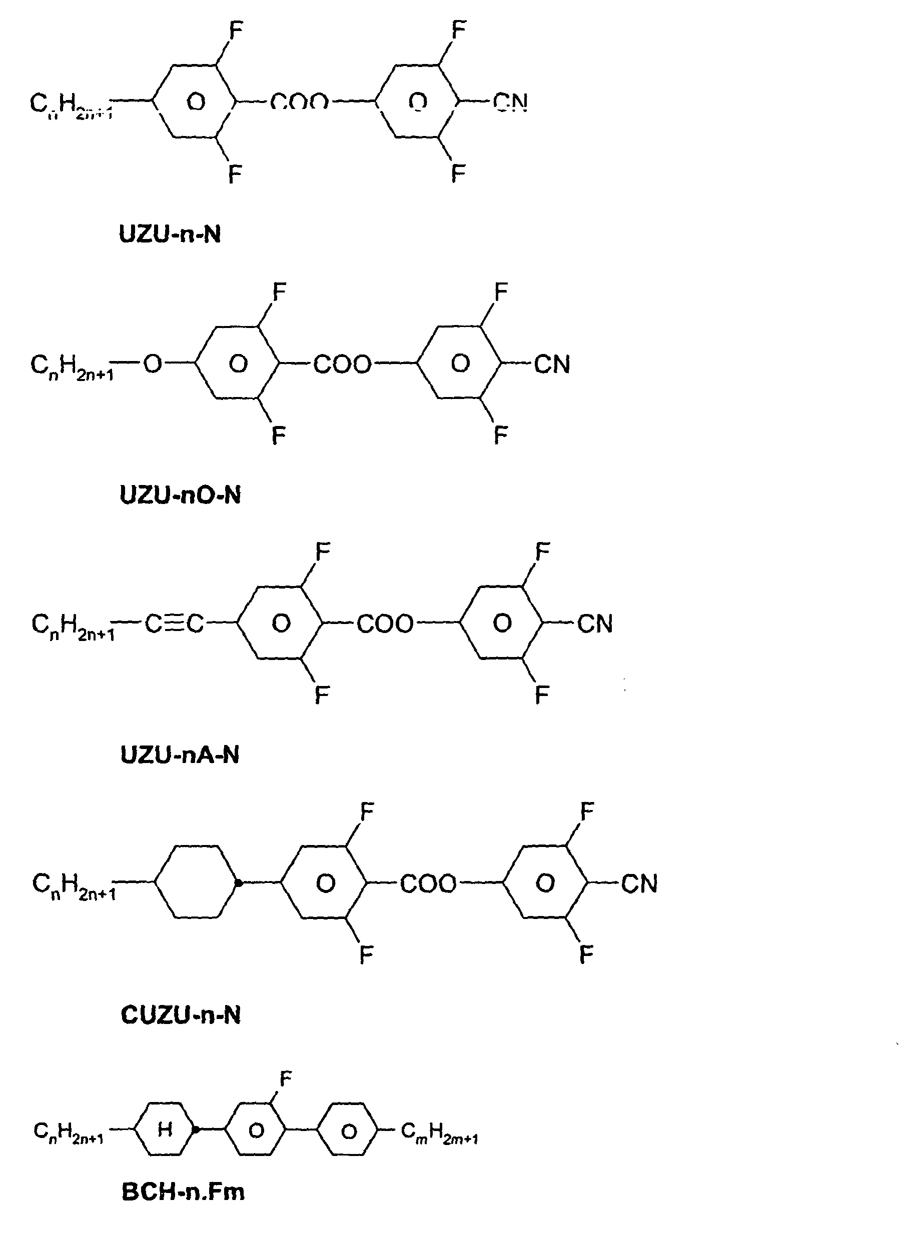

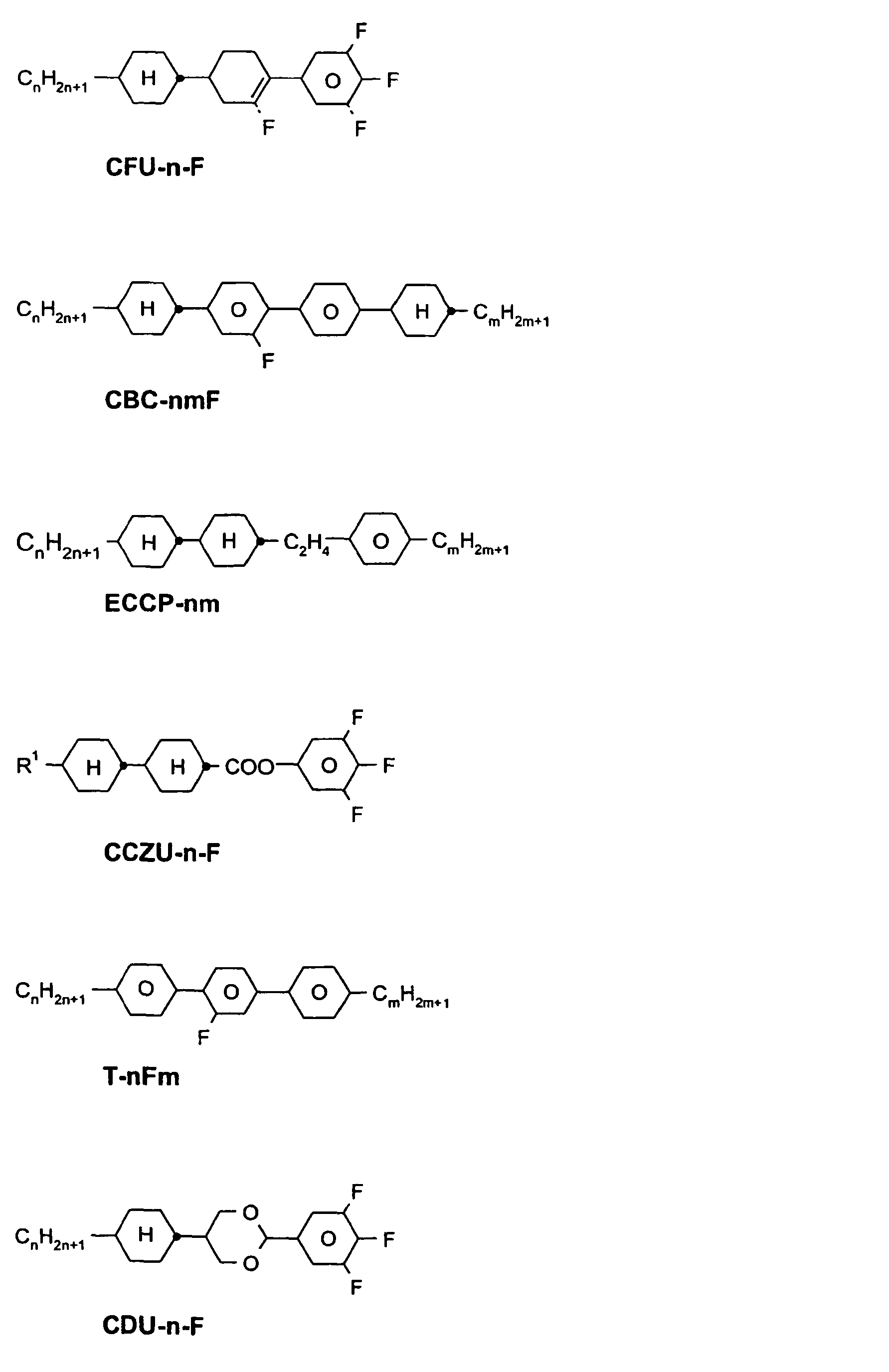

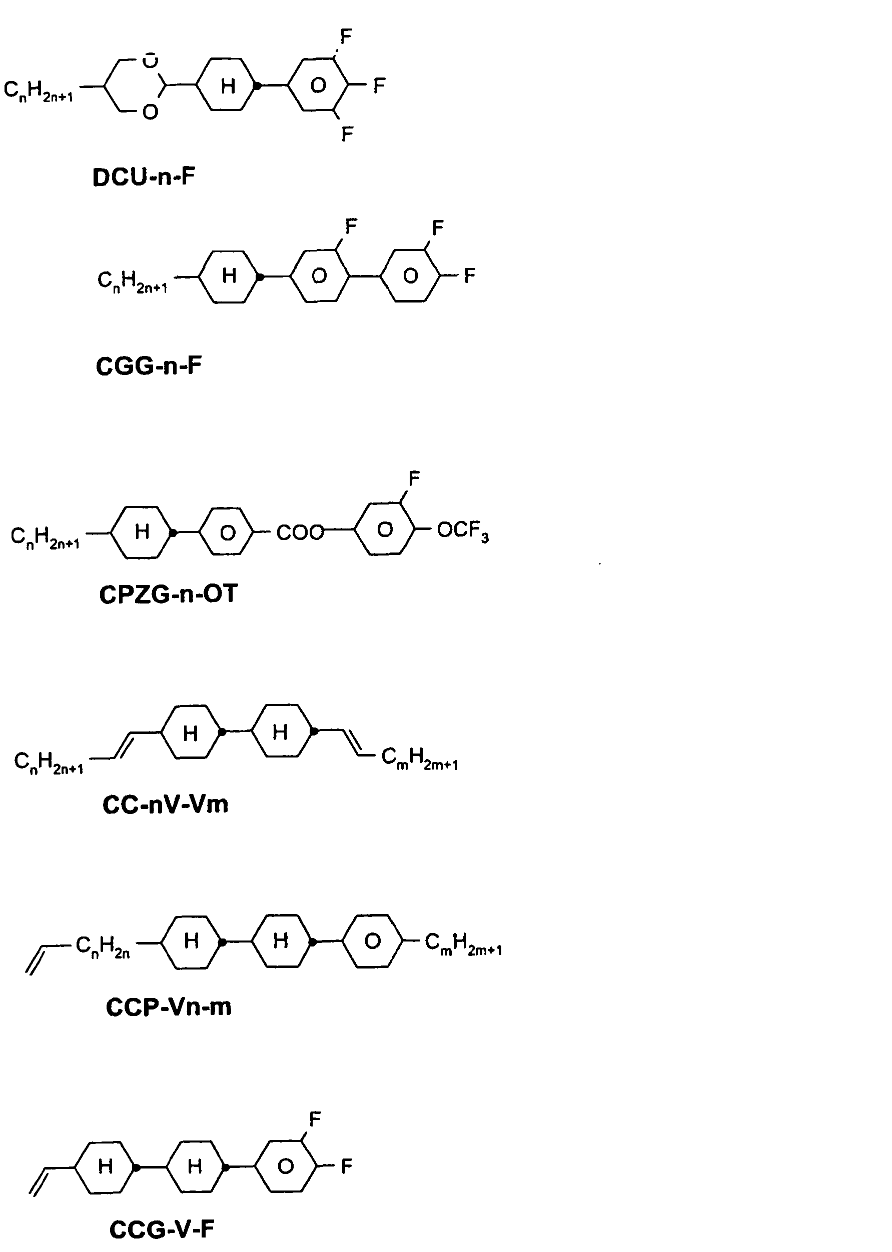

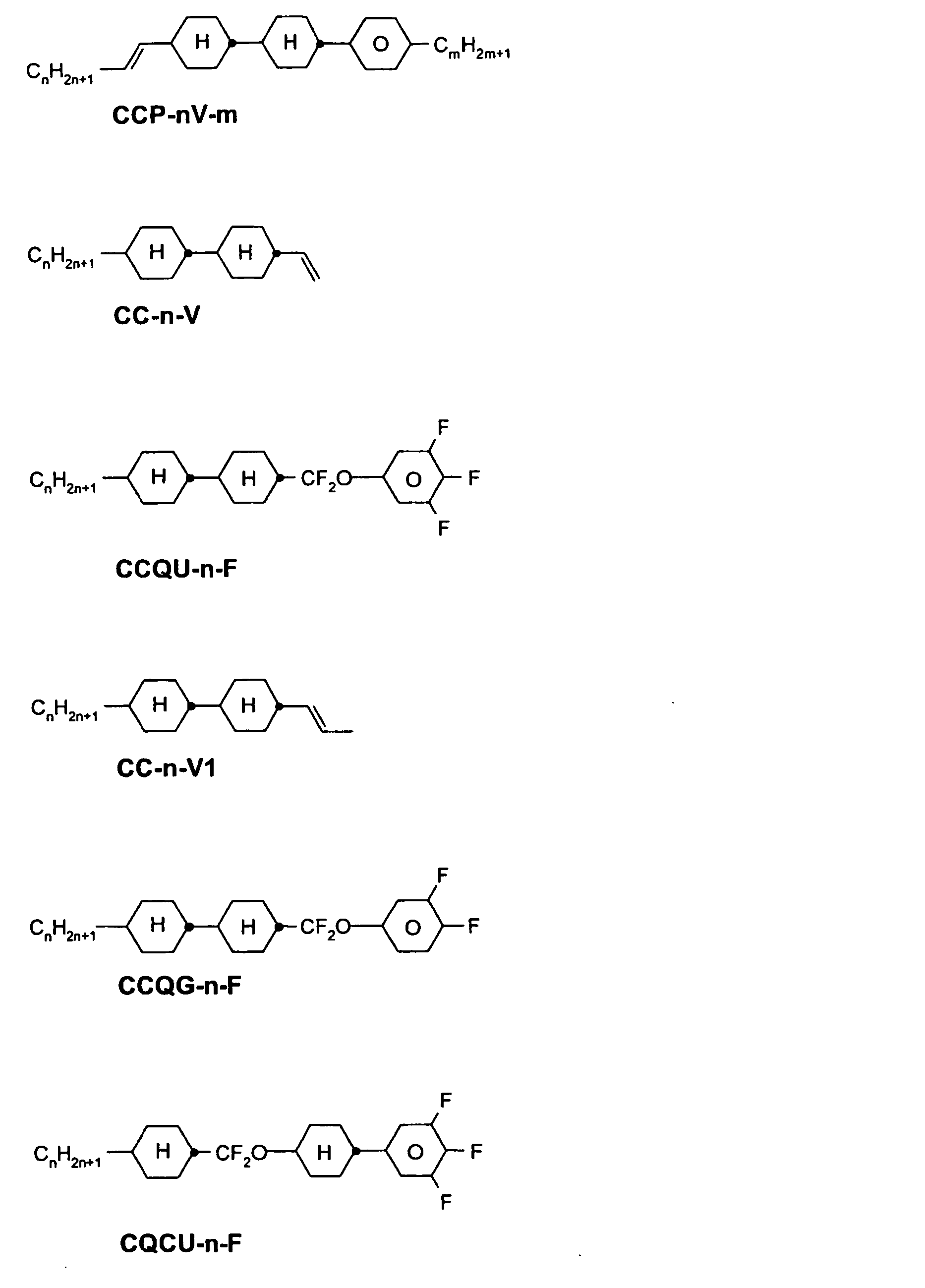

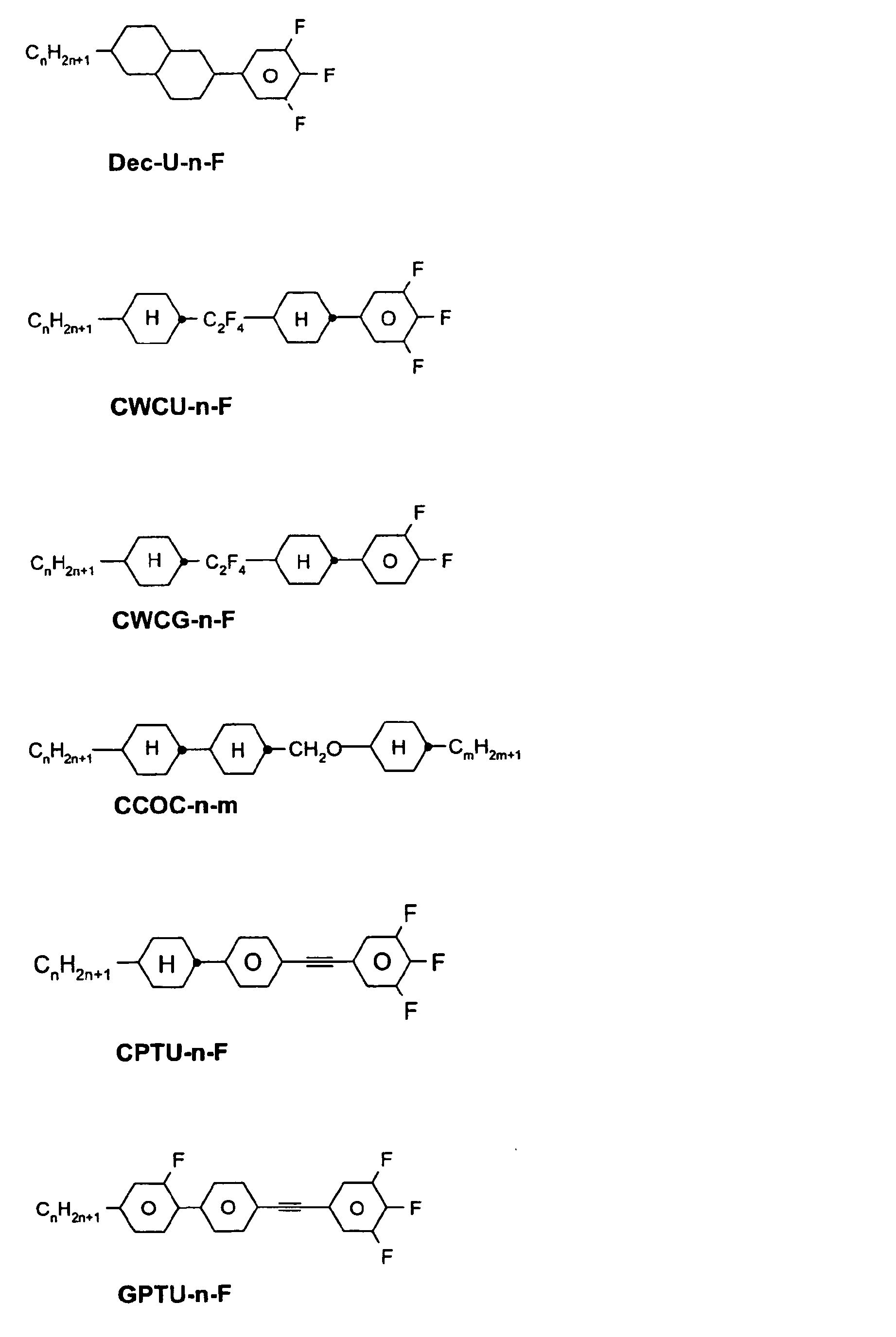

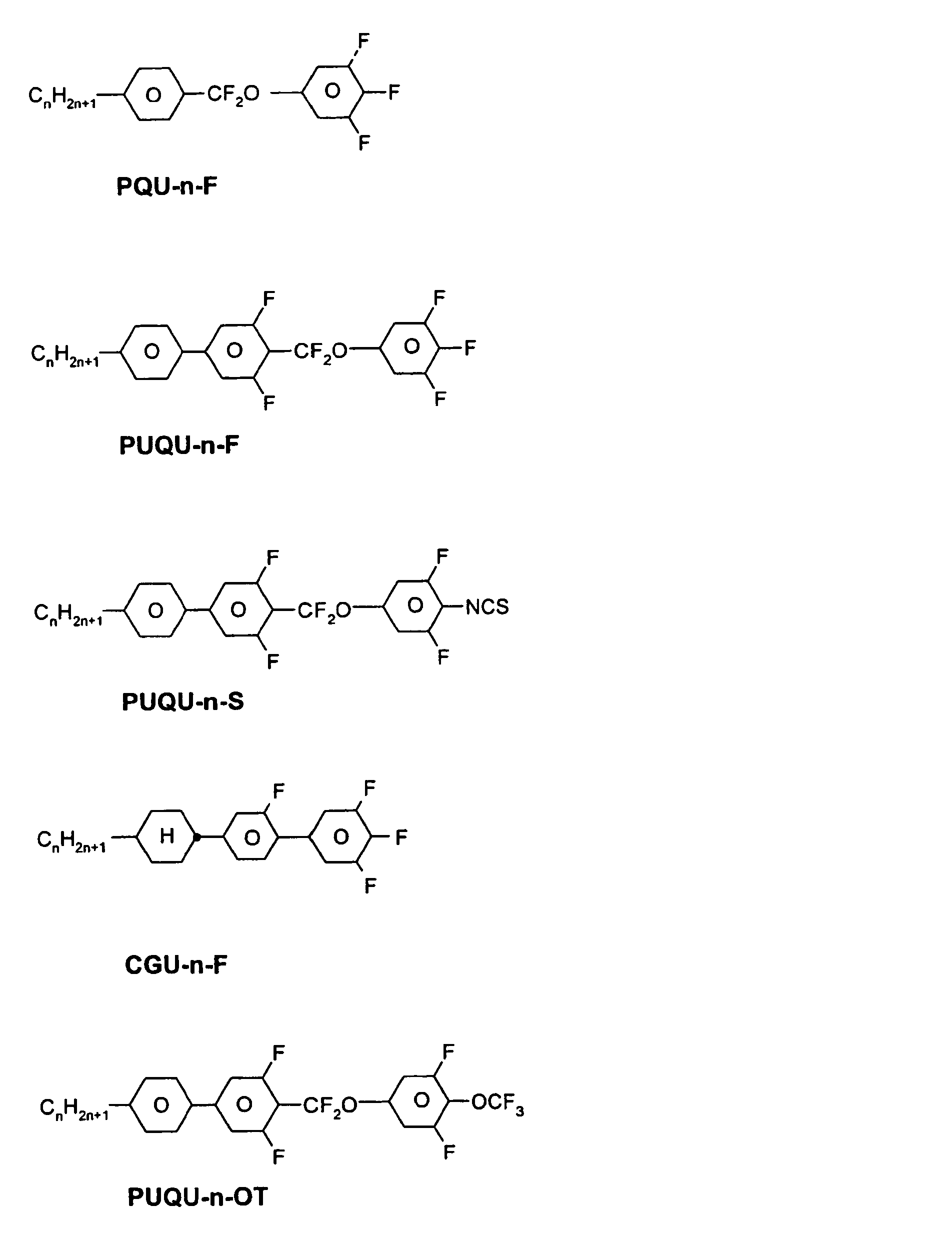

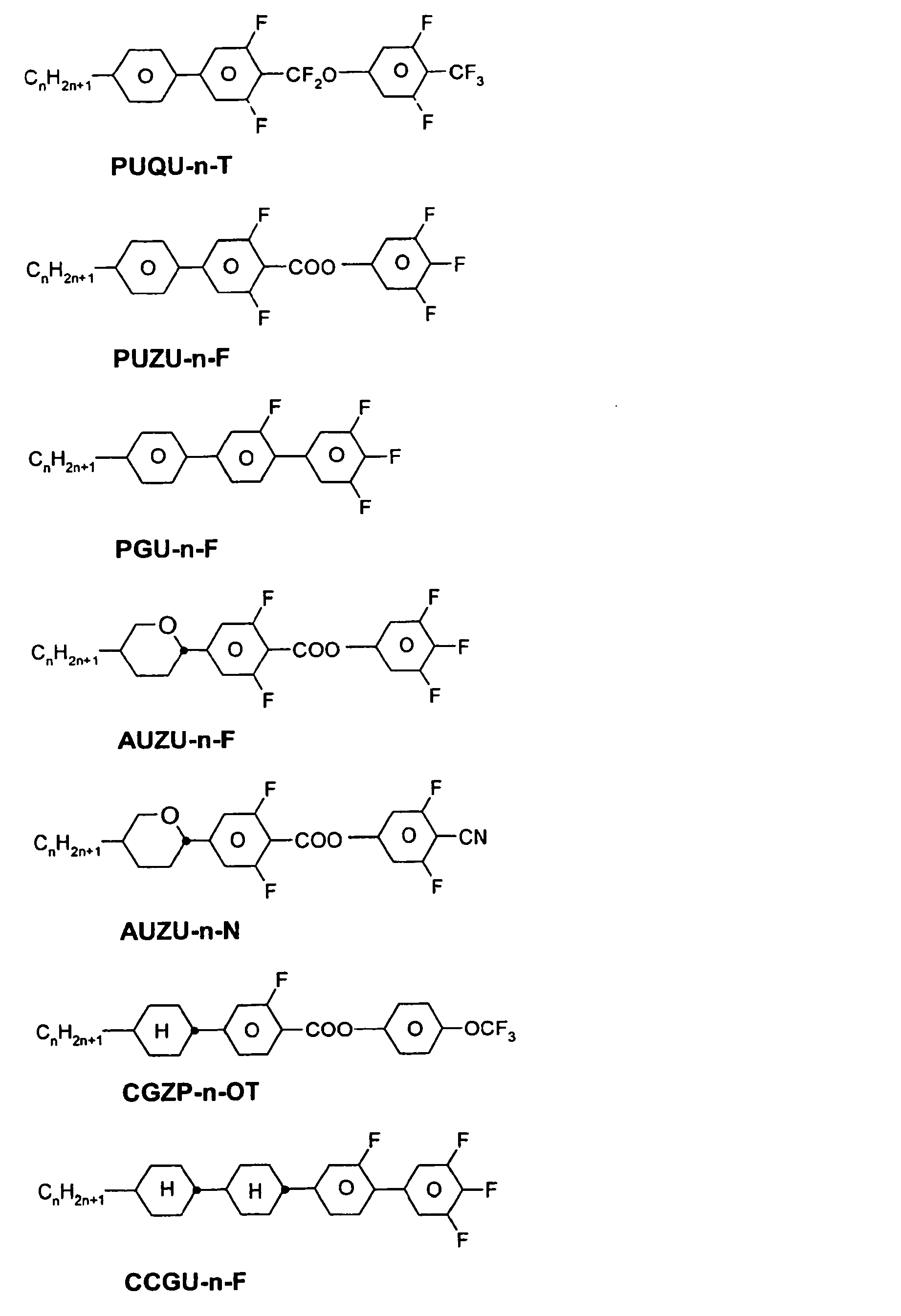

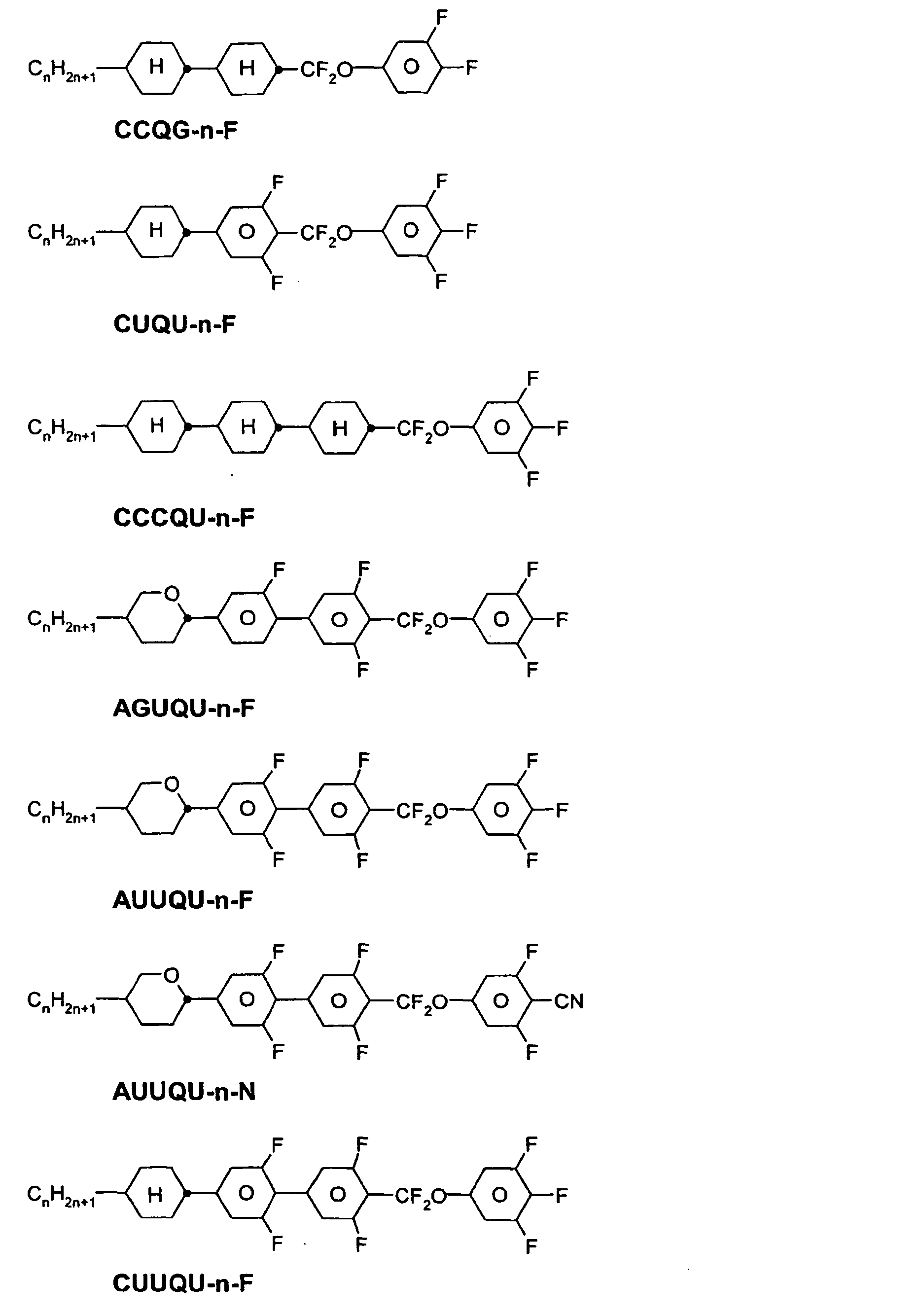

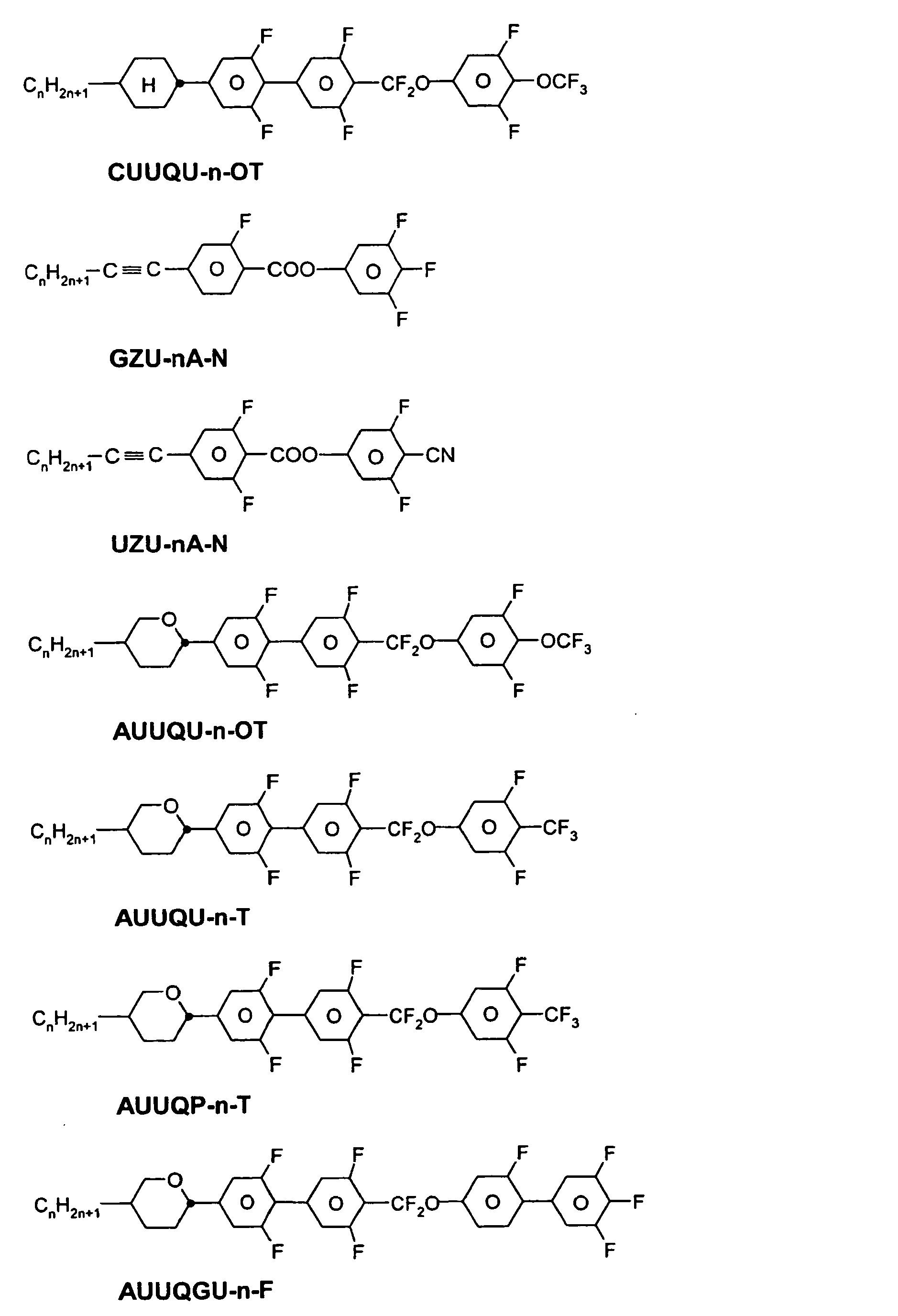

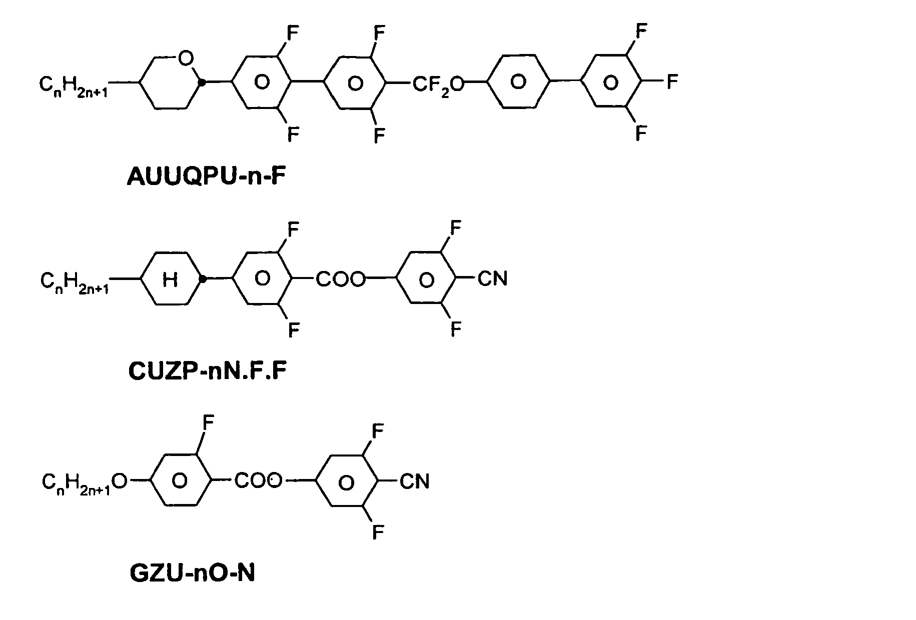

Particular preference is given to liquid-crystalline mixtures which, besides the compounds of the formula I, comprise at least one, two, three or four compounds from Table B.

**Table C:**

| |
|---|
| Table C shows possible dopants according to component D which are generally added to the mixtures alone or in combination two, three or more) according to the invention. |
| 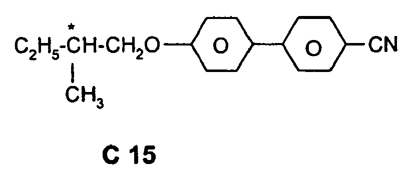 |
| 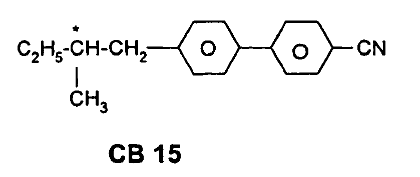 |
| 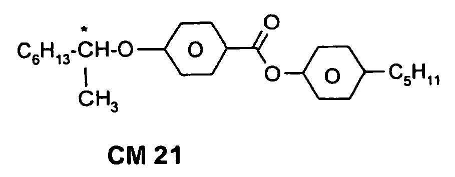 |
| 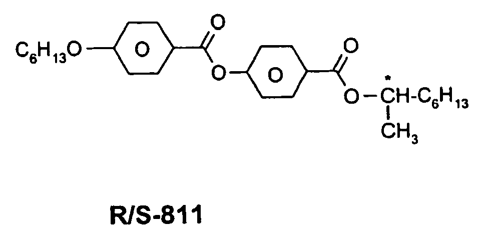 |
| 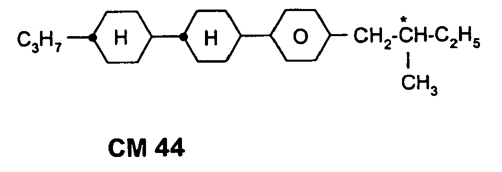 |
| 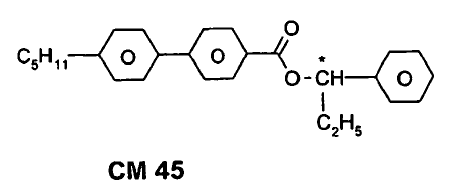 |
| 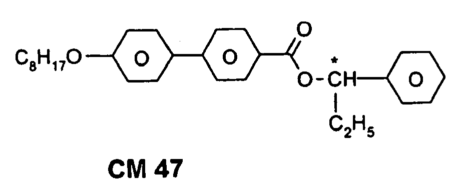 |
| 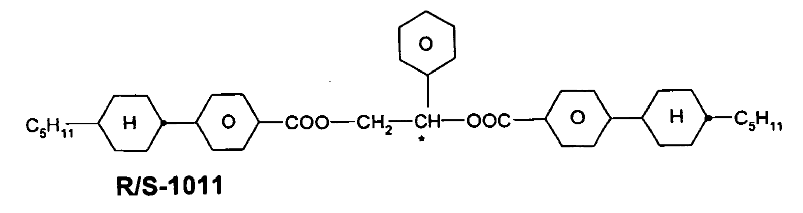 |
| 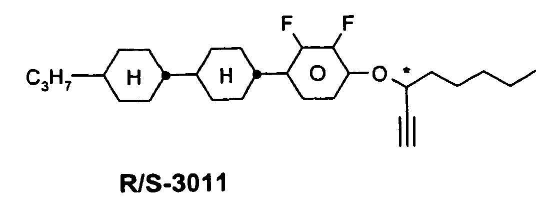 |
| 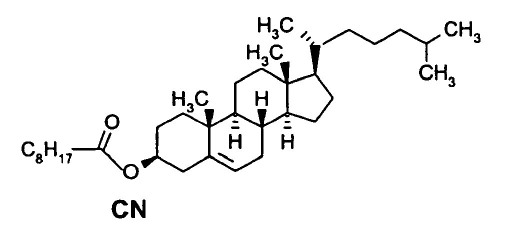 |
| 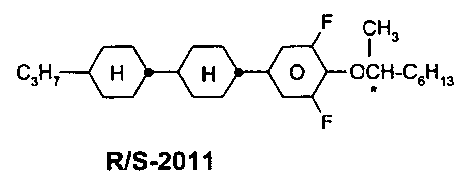 |
| 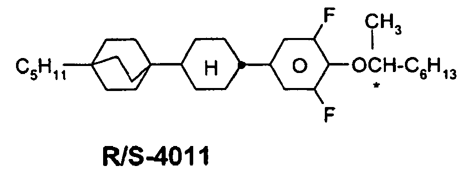 |
| 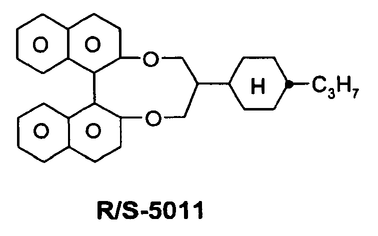 |

The liquid crystal media according to the instant invention do contain preferably
- four or more compounds selected from the group of compounds of tables A and B and/or
- five or more compounds selected from the group of compounds of table B and/or
- two or more compounds selected from the group of compounds of table A.

### Examples

The examples given in the following are illustrating the present invention, without limiting it in any way.

However, the physical data especially of the compounds illustrate to the expert, which properties can be achieved in which ranges. Especially the combination of the various properties, which can be preferably achieved, is thus well defined.

### Example 1: Preparation of 2-[Trans-4-Propylcyclohexyl]-propan-1,3-bis(oxyphenyl-4'trifluoromethane):

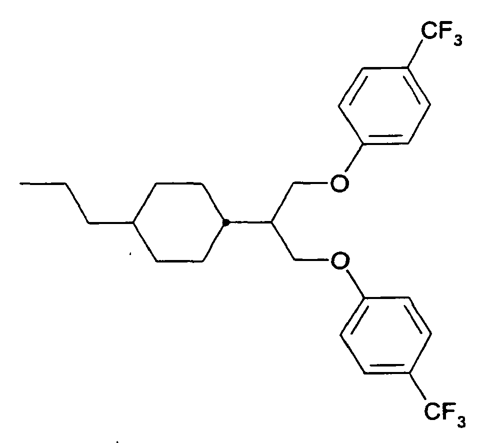

2-(4-*Trans*-cyclohexyl) propane-1,3-diol (4.63 g, 23.1 mmol), triphenylphosphine (12.4 g, 47.3 mmol) and 4-trifluoromethylphenol (7.50 g, 46.3 mmol) are dissolved in tetrahydrofuran (30 ml) under an atmosphere of nitrogen and cooled to -5°C. Diisopropyl diazocarboxylate (9.1 ml, 46.2 mmol) is slowly added dropwise; the mixture is allowed to warm to room temperature over night. The solvent is removed under reduced pressure and residue purified by flash column chromatography in heptane to give (upon evaporation of the appropriate fractions) the product as a colourless oil. The structure is confirmed by ¹H NMR spectroscopy.

### Example 2: Preparation of 2-[Trans-4-Propylcyclohexyl]-propan-1,3-bis(oxycarbonylphenyl-4'trifluoromethane):

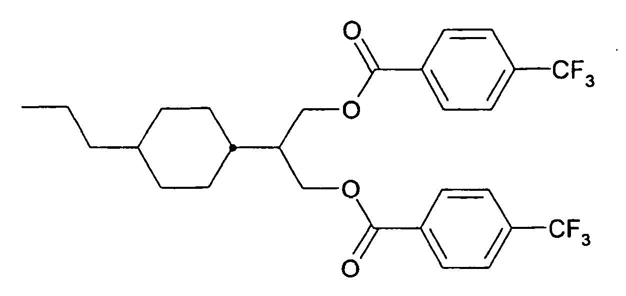

2-(4-*Trans*-cyclohexyl)propane-1,3-diol (3.15 g, 15.7 mmol), dicyclohexyl carbodiimide (6.5 g, 31.5 mmol), dimethylaminopyridine (0.2 g) and 4-trifluoromethyl benzoic acid (6.0 g, 31.5 mmol) are dissolved in dichloromethane (30 ml) under an atmosphere of nitrogen at 0°C. The mixture is allowed to warm to room temperature over night. The resulting precipitate is removed by filtration and the filtrate purified by flash column chromatography using petrol as eluant to give (upon evaporation of the appropriate fractions) the crude product as a white solid. Recrystallisation from IPA gives the product as a white crystalline solid. The structure is confirmed by ¹H NMR spectroscopy and the (M⁺ - F)-peak in GCMS is 525. The phase sequence is K 76.3 °C I.

### Example 3: Preparation of 2-[4'Pentylbiphenyl]propan-1,3-bis(oxycarbonylphenyl trifluoromethane):

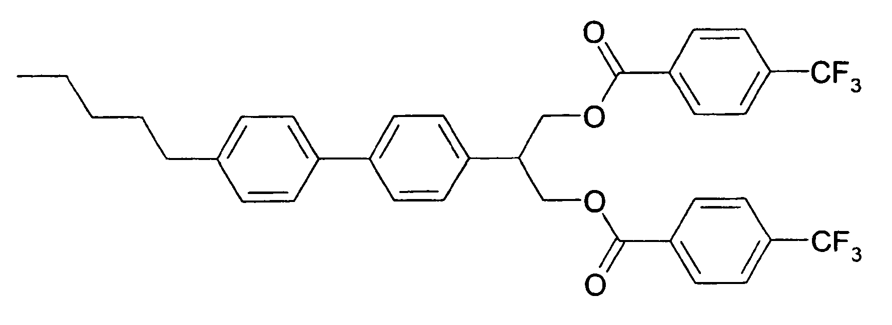

2-(4'-Pentylbiphenyl) propane-1,3-diol (3.0 g, 10.1 mmol), dicyclohexyl carbodiimide (4.2 g, 20.4 mmol), dimethylaminopyridine (0.2 g) and 4-trifluoromethyl benzoic acid (3.9 g, 20.5 mmol) are dissolved in dichloromethane (30 ml) under an atmosphere of nitrogen at 0°C. The mixture is allowed to warm to room temperature over night. The precipitate is removed by filtration and the filtrate purified by flash column chromatography using petrol with increasing concentration of dichloromethane as eluant to give (upon evaporation of the appropriate fractions) the crude product as a white solid. Recrystallisation from IPA ... gives the product as a white crystalline solid. The structure is confirmed by ¹H NMR spectroscopy and the mole- (M⁺) peak in GCMS is at 642. The phase sequence is K 100.9 °C I.

### Example 4: Preparation of Bis 1,3-(4-methoxybenzoic acid)-2-(4-propylcyclohexyl)-propyl ester

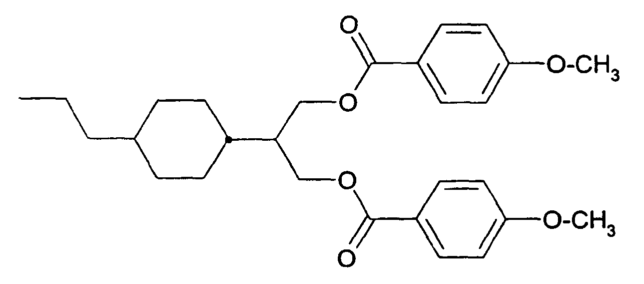

4-Propylcyclohexylpropane-1,3-diol (5.0 g, 25 mmol), 4-methoxybenzoic acid (7.6 g, 50 mmol), dicyclohexylcarbodiimide 1.0 M in dichloromethane (50 ml, 50 mmol) and dimethylaminopyridine (0.1 g) are dissolved in dichloromethane (50 ml) and stirred at room temperature for 16 hours. The mixture is filtered, the filtrate dried over sodium sulphate and evaporated to dryness. Purification by flash column chromatography afforded the product as a clear oil. The structure is confirmed by ¹H NMR spectroscopy and the mole- (M⁺) peak in GCMS is at 468.

### Examples 5 to 159

Analogously to example 1 the following compounds are prepared:

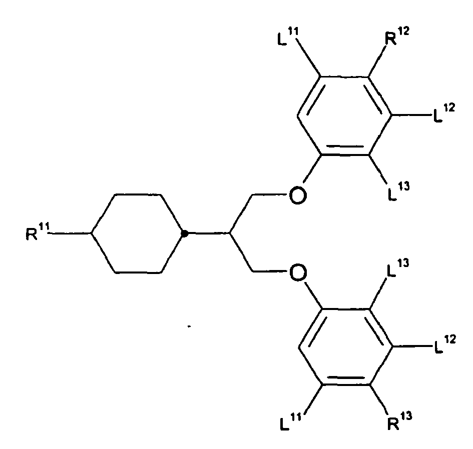

| No. | R¹¹ | R¹¹ | L¹¹ | L¹² | L¹³ | Phases (T/°C) |
|---|---|---|---|---|---|---|
| 5 | CH₃ | F | H | H | H | |
| 6 | C₂H₅ | F | H | H | H | |
| 7 | *n*-C₃H₇ | F | H | H | H | |
| 8 | *n*-C₄H₉ | F | H | H | H | |
| 9 | *n*-C₅H₁₁ | F | H | H | H | |
| 10 | *n*-C₆H₁₃ | F | H | H | H | |
| 11 | *n*-C₇H₁₅ | F | H | H | H | |
| 12 | *n*-C₈H₁₇ | F | H | H | H | |
| 13 | *n*-C₉H₁₉ | F | H | H | H | |
| 14 | *n*-C₁₀H₂₁ | F | H | H | H | |
| 15 | CH₂=CH | F | H | H | H | |
| 16 | CH₂=CH-CH₂ | F | H | H | H | |
| 17 | CH₃-CH=CH | F | H | H | H | |
| 18 | CH₃ | F | F | H | H | |
| 19 | C₂H₅ | F | F | H | H | |
| 20 | *n*-C₃H₇ | F | F | H | H | |
| 21 | *n*-C₄H₉ | F | F | H | H | |
| 22 | *n*-C₅H₁₁ | F | F | H | H | |
| 23 | *n*-C₆H₁₃ | F | F | H | H | |
| 24 | *n*-C₇H₁₅ | F | F | H | H | |
| 25 | *n*-C₈H₁₇ | F | F | H | H | |
| 26 | *n*-C₉H₁₉ | F | F | H | H | |
| 27 | *n*-C₁₀H₂₁ | F | F | H | H | |
| 28 | CH₂=CH | F | F | H | H | |
| 29 | CH₂=CH-CH₂ | F | F | H | H | |
| 30 | CH₃-CH=CH | F | F | H | H | |
| 31 | CH₃ | F | F | F | H | |
| 32 | C₂H₅ | F | F | F | H | |
| 33 | *n*-C₃H₇ | F | F | F | H | |
| 34 | *n*-C₄H₉ | F | F | F | H | |
| 35 | *n*-C₅H₁₁ | F | F | F | H | |
| 36 | *n*-C₆H₁₃ | F | F | F | H | |
| 37 | *n*-C₇H₁₅ | F | F | F | H | |
| 38 | *n*-C₈H₁₇ | F | F | F | H | |
| 39 | *n*-C₉H₁₉ | F | F | F | H | |
| 40 | *n*-C₁₀H₂₁ | F | F | F | H | |
| 41 | CH₂=CH | F | F | F | H | |
| 42 | CH₂=CH-CH₂ | F | F | F | H | |
| 43 | CH₃-CH=CH | F | F | F | H | |
| 44 | CH₃ | F | H | F | F | |
| 45 | C₂H₅ | F | H | F | F | |
| 46 | *n*-C₃H₇ | F | H | F | F | |
| 47 | *n*-C₄H₉ | F | H | F | F | |
| 48 | *n*-C₅H₁₁ | F | H | F | F | |
| 49 | *n*-C₆H₁₃ | F | H | F | F | |
| 50 | *n*-C₇H₁₅ | F | H | F | F | |
| 51 | *n*-C₈H₁₇ | F | H | F | F | |
| 52 | *n*-C₉H₁₉ | F | H | F | F | |
| 53 | *n*-C₁₀H₂₁ | F | H | F | F | |
| 54 | CH₂=CH | F | H | F | F | |
| 55 | CH₂=CH-CH₂ | F | H | F | F | |
| 56 | CH₃-CH=CH | F | H | F | F | |
| 57 | CH₃ | Cl | H | H | H | |
| 58 | C₂H₅ | Cl | H | H | H | |
| 59 | *n*-C₃H₇ | Cl | H | H | H | |
| 60 | *n*-C₄H₉ | Cl | H | H | H | |
| 61 | *n*-C₅H₁₁ | Cl | H | H | H | |
| 62 | *n*-C₆H₁₃ | Cl | H | H | H | |
| 63 | *n*-C₇H₁₅ | Cl | H | H | H | |
| 64 | *n*-C₈H₁₇ | Cl | H | H | H | |
| 65 | *n*-C₉H₁₉ | Cl | H | H | H | |
| 66 | *n*-C₁₀H₂₁ | Cl | H | H | H | |
| 67 | CH₂=CH | Cl | H | H | H | |
| 68 | CH₂=CH-CH₂ | Cl | H | H | H | |
| 69 | CH₃-CH=CH | Cl | H | H | H | |
| 70 | CH₃ | Cl | F | H | H | |
| 71 | C₂H₅ | Cl | F | H | H | |
| 72 | *n*-C₃H₇ | Cl | F | H | H | |
| 73 | *n*-C₄H₉ | Cl | F | H | H | |
| 74 | *n*-C₅H₁₁ | Cl | F | H | H | |
| 75 | *n*-C₆H₁₃ | Cl | F | H | H | |
| 76 | *n*-C₇H₁₅ | Cl | F | H | H | |
| 77 | *n*-C₈H₁₇ | Cl | F | H | H | |
| 78 | *n*-C₉H₁₉ | Cl | F | H | H | |
| 79 | *n*-C₁₀H₂₁ | Cl | F | H | H | |
| 80 | CH₂=CH | Cl | F | H | H | |
| 81 | CH₂=CH-CH₂ | Cl | F | H | H | |
| 82 | CH₃-CH=CH | Cl | F | H | H | |
| 83 | CH₃ | Cl | F | F | H | |
| 84 | C₂H₅ | Cl | F | F | H | |
| 85 | *n*-C₃H₇ | Cl | F | F | H | |
| 86 | *n*-C₄H₉ | Cl | F | F | H | |
| 87 | *n*-C₅H₁₁ | Cl | F | F | H | |
| 88 | *n*-C₆H₁₃ | Cl | F | F | H | |
| 89 | *n*-C₇H₁₅ | Cl | F | F | H | |
| 90 | *n*-C₈H₁₇ | Cl | F | F | H | |
| 91 | *n*-C₉H₁₉ | Cl | F | F | H | |
| 92 | *n*-C₁₀H₂₁ | Cl | F | F | H | |
| 93 | CH₂=CH | Cl | F | F | H | |
| 94 | CH₂=CH-CH₂ | Cl | F | F | H | |
| 95 | CH₃-CH=CH | Cl | F | F | H | |
| 96 | CH₃ | Cl | H | F | F | |
| 97 | C₂H₅ | Cl | H | F | F | |
| 98 | *n*-C₃H₇ | Cl | H | F | F | |
| 99 | *n*-C₄H₉ | Cl | H | F | F | |
| 100 | *n*-C₅H₁₁ | Cl | H | F | F | |
| 101 | *n*-C₆H₁₃ | Cl | H | F | F | |
| 102 | *n*-C₇H₁₅ | Cl | H | F | F | |
| 103 | *n*-C₈H₁₇ | Cl | H | F | F | |
| 104 | *n*-C₉H₁₉ | Cl | H | F | F | |
| 105 | *n*-C₁₀H₂₁ | Cl | H | F | F | |
| 106 | CH₂=CH | Cl | H | F | F | |
| 107 | CH₂=CH-CH₂ | Cl | H | F | F | |
| 108 | CH₃-CH=CH | Cl | H | F | F | |
| 109 | CH₃ | CF₃ | H | H | H | |
| 110 | C₂H₅ | CF₃ | H | H | H | |
| 1 | *n*-C₃H₇ | CF₃ | H | H | H | |
| 111 | *n*-C₄H₉ | CF₃ | H | H | H | |
| 112 | *n*-C₅H₁₁ | CF₃ | H | H | H | |
| 113 | *n*-C₆H₁₃ | CF₃ | H | H | H | |
| 114 | *n*-C₇H₁₅ | CF₃ | H | H | H | |
| 115 | *n*-C₈H₁₇ | CF₃ | H | H | H | |
| 116 | *n*-C₉H₁₉ | CF₃ | H | H | H | |
| 117 | *n*-C₁₀H₂₁ | CF₃ | H | H | H | |
| 118 | CH₂=CH | CF₃ | H | H | H | |
| 119 | CH₂=CH-CH₂ | CF₃ | H | H | H | |
| 120 | CH₃-CH=CH | CF₃ | H | H | H | |
| 121 | CH₃ | CF₃ | F | H | H | |
| 122 | C₂H₅ | CF₃ | F | H | H | |
| 123 | *n*-C₃H₇ | CF₃ | F | H | H | |
| 124 | *n*-C₄H₉ | CF₃ | F | H | H | |
| 125 | *n*-C₅H₁₁ | CF₃ | F | H | H | |
| 126 | *n*-C₆H₁₃ | CF₃ | F | H | H | |
| 127 | *n*-C₇H₁₅ | CF₃ | F | H | H | |
| 128 | *n*-C₈H₁₇ | CF₃ | F | H | H | |
| 129 | *n*-C₉H₁₉ | CF₃ | F | H | H | |
| 130 | *n*-C₁₀H₂₁ | CF₃ | F | H | H | |
| 131 | CH₂=CH | CF₃ | F | H | H | |
| 132 | CH₂=CH-CH₂ | CF₃ | F | H | H | |
| 133 | CH₃-CH=CH | CF₃ | F | H | H | |
| 134 | CH₃ | CF₃ | F | F | H | |
| 135 | C₂H₅ | CF₃ | F | F | H | |
| 136 | *n*-C₃H₇ | CF₃ | F | F | H | |
| 137 | *n*-C₄H₉ | CF₃ | F | F | H | |
| 138 | *n*-C₅H₁₁ | CF₃ | F | F | H | |
| 139 | *n*-C₆H₁₃ | CF₃ | F | F | H | |
| 140 | *n*-C₇H₁₅ | CF₃ | F | F | H | |
| 141 | *n*-C₈H₁₇ | CF₃ | F | F | H | |
| 142 | *n*-C₉H₁₉ | CF₃ | F | F | H | |
| 143 | *n*-C₁₀H₂₁ | CF₃ | F | F | H | |
| 144 | CH₂=CH | CF₃ | F | F | H | |
| 145 | CH₂=CH-CH₂ | CF₃ | F | F | H | |
| 146 | CH₃-CH=CH | CF₃ | F | F | H | |
| 147 | CH₃ | CF₃ | H | F | F | |
| 148 | C₂H₅ | CF₃ | H | F | F | |
| 149 | *n*-C₃H₇ | CF₃ | H | F | F | |
| 150 | *n*-C₄H₉ | CF₃ | H | F | F | |
| 151 | *n*-C₅H₁₁ | CF₃ | H | F | F | |
| 152 | *n*-C₆H₁₃ | CF₃ | H | F | F | |
| 153 | *n*-C₇H₁₅ | CF₃ | H | F | F | |
| 154 | *n*-C₈H₁₇ | CF₃ | H | F | F | |
| 155 | *n*-C₉H₁₉ | CF₃ | H | F | F | |
| 156 | *n*-C₁₀H₂₁ | CF₃ | H | F | F | |
| 157 | CH₂=CH | CF₃ | H | F | F | |
| 158 | CH₂=CH-CH₂ | CF₃ | H | F | F | |
| 159 | CH₃-CH=CH | CF₃ | H | F | F | |

### Examples 160 to 329

Analogously to example 2 the following compounds are prepared:

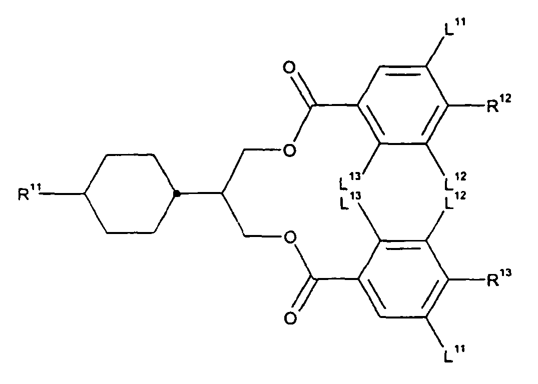

| No. | R¹¹ | R¹² | L¹¹ | L¹² | L¹³ | Phases (T/°C) |
|---|---|---|---|---|---|---|
| 160 | CH₃ | F | H | H | H | |
| 161 | C₂H₅ | F | H | H | H | |
| 162 | *n*-C₃H₇ | F | H | H | H | |
| 163 | *n*-C₄H₉ | F | H | H | H | |
| 164 | *n*-C₅H₁₁ | F | H | H | H | |
| 165 | *n*-C₆H₁₃ | F | H | H | H | |
| 166 | *n*-C₇H₁₅ | F | H | H | H | |
| 167 | *n*-C₈H₁₇ | F | H | H | H | |
| 168 | *n*-C₉H₁₉ | F | H | H | H | |
| 169 | *n*-C₁₀H₂₁ | F | H | H | H | |
| 170 | CH₂=CH | F | H | H | H | |
| 186 | CH₂=CH-CH₂ | F | H | H | H | |
| 187 | CH₃-CH=CH | F | H | H | H | |
| 188 | CH₃ | F | F | H | H | |
| 189 | C₂H₅ | F | F | H | H | |
| 190 | *n*-C₃H₇ | F | F | H | H | |
| 191 | *n*-C₄H₉ | F | F | H | H | |
| 192 | *n*-C₅H₁₁ | F | F | H | H | |
| 193 | *n*-C₆H₁₃ | F | F | H | H | |
| 194 | *n*-C₇H₁₅ | F | F | H | H | |
| 195 | *n*-C₈H₁₇ | F | F | H | H | |
| 196 | *n*-C₉H₁₉ | F | F | H | H | |
| 197 | *n*-C₁₀H₂₁ | F | F | H | H | |
| 198 | CH₂=CH | F | F | H | H | |
| 199 | CH₂=CH-CH₂ | F | F | H | H | |
| 200 | CH₃-CH=CH | F | F | H | H | |
| 201 | CH₃ | F | F | F | H | |
| 202 | C₂H₅ | F | F | F | H | |
| 203 | *n*-C₃H₇ | F | F | F | H | |
| 204 | *n*-C₄H₉ | F | F | F | H | |
| 205 | *n*-C₅H₁₁ | F | F | F | H | |
| 206 | *n*-C₆H₁₃ | F | F | F | H | |
| 207 | *n*-C₇H₁₅ | F | F | F | H | |
| 208 | *n*-C₈H₁₇ | F | F | F | H | |
| 209 | *n*-C₉H₁₉ | F | F | F | H | |
| 210 | *n*-C₁₀H₂₁ | F | F | F | H | |
| 211 | CH₂=CH | F | F | F | H | |
| 212 | CH₂=CH-CH₂ | F | F | F | H | |
| 213 | CH₃-CH=CH | F | F | F | H | |
| 214 | CH₃ | F | H | F | F | |
| 215 | C₂H₅ | F | H | F | F | |
| 216 | *n*-C₃H₇ | F | H | F | F | |
| 217 | *n*-C₄H₉ | F | H | F | F | |
| 218 | *n*-C₅H₁₁ | F | H | F | F | |
| 219 | *n*-C₆H₁₃ | F | H | F | F | |
| 220 | *n*-C₇H₁₅ | F | H | F | F | |
| 221 | *n*-C₈H₁₇ | F | H | F | F | |
| 222 | *n*-C₉H₁₉ | F | H | F | F | |
| 223 | *n*-C₁₀H₂₁ | F | H | F | F | |
| 224 | CH₂=CH | F | H | F | F | |
| 225 | CH₂=CH-CH₂ | F | H | F | F | |
| 226 | CH₃-CH=CH | F | H | F | F | |
| 227 | CH₃ | Cl | H | H | H | |
| 228 | C₂H₅ | Cl | H | H | H | |
| 229 | *n*-C₃H₇ | Cl | H | H | H | |
| 230 | *n*-C₄H₉ | Cl | H | H | H | |
| 231 | *n*-C₅H₁₁ | Cl | H | H | H | |
| 232 | *n*-C₆H₁₃ | Cl | H | H | H | |
| 233 | *n*-C₇H₁₅ | Cl | H | H | H | |
| 234 | *n*-C₈H₁₇ | Cl | H | H | H | |
| 235 | *n*-C₉H₁₉ | Cl | H | H | H | |
| 236 | *n*-C₁₀H₂₁ | Cl | H | H | H | |
| 237 | CH₂=CH | Cl | H | H | H | |
| 238 | CH₂=CH-CH₂ | Cl | H | H | H | |
| 239 | CH₃-CH=CH | Cl | H | H | H | |
| 240 | CH₃ | Cl | F | H | H | |
| 241 | C₂H₅ | Cl | F | H | H | |
| 242 | *n*-C₃H₇ | Cl | F | H | H | |
| 243 | *n*-C₄H₉ | Cl | F | H | H | |
| 244 | *n*-C₅H₁₁ | Cl | F | H | H | |
| 245 | *n*-C₆H₁₃ | Cl | F | H | H | |
| 246 | *n*-C₇H₁₅ | Cl | F | H | H | |
| 247 | *n*-C₈H₁₇ | Cl | F | H | H | |
| 248 | *n*-C₉H₁₉ | Cl | F | H | H | |
| 249 | *n*-C₁₀H₂₁ | Cl | F | H | H | |
| 250 | CH₂=CH | Cl | F | H | H | |
| 251 | CH₂=CH-CH₂ | Cl | F | H | H | |
| 252 | CH₃-CH=CH | Cl | F | H | H | |
| 253 | CH₃ | Cl | F | F | H | |
| 254 | C₂H₅ | Cl | F | F | H | |
| 255 | *n*-C₃H₇ | Cl | F | F | H | |
| 256 | *n*-C₄H₉ | Cl | F | F | H | |
| 257 | *n*-C₅H₁₁ | Cl | F | F | H | |
| 258 | *n*-C₆H₁₃ | Cl | F | F | H | |
| 259 | *n*-C₇H₁₅ | Cl | F | F | H | |
| 260 | *n*-C₈H₁₇ | Cl | F | F | H | |
| 261 | *n*-C₉H₁₉ | Cl | F | F | H | |
| 262 | *n*-C₁₀H₂₁ | Cl | F | F | H | |
| 263 | CH₂=CH | Cl | F | F | H | |
| 264 | CH₂=CH-CH₂ | Cl | F | F | H | |
| 265 | CH₃-CH=CH | Cl | F | F | H | |
| 266 | CH₃ | Cl | H | F | F | |
| 267 | C₂H₅ | Cl | H | F | F | |
| 268 | *n*-C₃H₇ | Cl | H | F | F | |
| 269 | *n*-C₄H₉ | Cl | H | F | F | |
| 270 | *n*-C₅H₁₁ | Cl | H | F | F | |
| 271 | *n*-C₆H₁₃ | Cl | H | F | F | |
| 272 | *n*-C₇H₁₅ | Cl | H | F | F | |
| 273 | *n*-C₈H₁₇ | Cl | H | F | F | |
| 274 | *n*-C₉H₁₉ | Cl | H | F | F | |
| 275 | *n*-C₁₀H₂₁ | Cl | H | F | F | |
| 276 | CH₂=CH | Cl | H | F | F | |
| 277 | CH₂=CH-CH₂ | Cl | H | F | F | |
| 278 | CH₃-CH=CH | Cl | H | F | F | |
| 279 | CH₃ | CF₃ | H | H | H | |
| 280 | C₂H₅ | CF₃ | H | H | H | |
| 2 | *n*-C₃H₇ | CF₃ | H | H | H | |
| 281 | *n*-C₄H₉ | CF₃ | H | H | H | |
| 282 | *n*-C₅H₁₁ | CF₃ | H | H | H | |
| 283 | *n*-C₆H₁₃ | CF₃ | H | H | H | |
| 284 | *n*-C₇H₁₅ | CF₃ | H | H | H | |
| 285 | *n*-C₈H₁₇ | CF₃ | H | H | H | |
| 286 | *n*-C₉H₁₉ | CF₃ | H | H | H | |
| 287 | *n*-C₁₀H₂₁ | CF₃ | H | H | H | |
| 288 | CH₂=CH | CF₃ | H | H | H | |
| 289 | CH₂=CH-CH₂ | CF₃ | H | H | H | |
| 290 | CH₃-CH=CH | CF₃ | H | H | H | |
| 291 | CH₃ | CF₃ | F | H | H | |
| 292 | C₂H₅ | CF₃ | F | H | H | |
| 293 | *n*-C₃H₇ | CF₃ | F | H | H | |
| 294 | *n*-C₄H₉ | CF₃ | F | H | H | |
| 295 | *n*-C₅H₁₁ | CF₃ | F | H | H | |
| 296 | *n*-C₆H₁₃ | CF₃ | F | H | H | |
| 297 | *n*-C₇H₁₅ | CF₃ | F | H | H | |
| 298 | *n*-C₈H₁₇ | CF₃ | F | H | H | |
| 299 | *n*-C₉H₁₉ | CF₃ | F | H | H | |
| 300 | *n*-C₁₀H₂₁ | CF₃ | F | H | H | |
| 301 | CH₂=CH | CF₃ | F | H | H | |
| 302 | CH₂=CH-CH₂ | CF₃ | F | H | H | |
| 303 | CH₃-CH=CH | CF₃ | F | H | H | |
| 304 | CH₃ | CF₃ | F | F | H | |
| 305 | C₂H₅ | CF₃ | F | F | H | |
| 306 | *n*-C₃H₇ | CF₃ | F | F | H | |
| 307 | *n*-C₄H₉ | CF₃ | F | F | H | |
| 308 | *n*-C₅H₁₁ | CF₃ | F | F | H | |
| 309 | *n*-C₆H₁₃ | CF₃ | F | F | H | |
| 310 | *n*-C₇H₁₅ | CF₃ | F | F | H | |
| 311 | *n*-C₈H₁₇ | CF₃ | F | F | H | |
| 312 | *n*-C₉H₁₉ | CF₃ | F | F | H | |
| 313 | *n*-C₁₀H₂₁ | CF₃ | F | F | H | |
| 314 | CH₂=CH | CF₃ | F | F | H | |
| 315 | CH₂=CH-CH₂ | CF₃ | F | F | H | |
| 316 | CH₃-CH=CH | CF₃ | F | F | H | |
| 317 | CH₃ | CF₃ | H | F | F | |
| 318 | C₂H₅ | CF₃ | H | F | F | |
| 319 | *n*-C₃H₇ | CF₃ | H | F | F | |
| 320 | *n*-C₄H₉ | CF₃ | H | F | F | |
| 321 | *n*-C₅H₁₁ | CF₃ | H | F | F | |
| 322 | *n*-C₆H₁₃ | CF₃ | H | F | F | |
| 323 | *n*-C₇H₁₅ | CF₃ | H | F | F | |
| 324 | *n*-C₈H₁₇ | CF₃ | H | F | F | |
| 325 | *n*-C₉H₁₉ | CF₃ | H | F | F | |
| 326 | *n*-C₁₀H₂₁ | CF₃ | H | F | F | |
| 327 | CH₂=CH | CF₃ | H | F | F | |
| 328 | CH₂=CH-CH₂ | CF₃ | H | F | F | |
| 329 | CH₃-CH=CH | CF₃ | H | F | F | |

### Examples 330 to 485

Analogously to example 3 the following compounds are prepared:

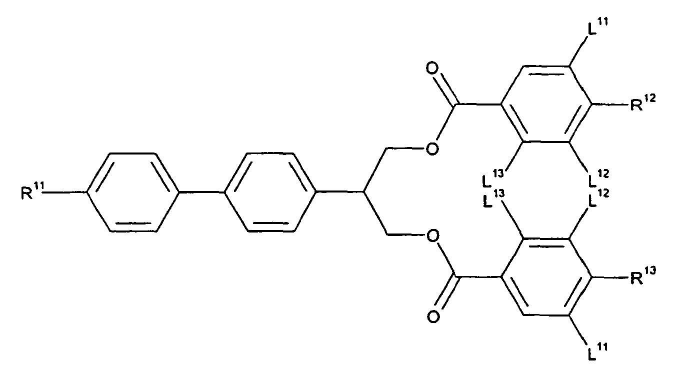

| No. | R¹¹ | R¹² | L¹¹ | L¹² | L¹³ | Phases (T/°C) |
|---|---|---|---|---|---|---|
| 330 | CH₃ | F | H | H | H | |
| 331 | C₂H₅ | F | H | H | H | |
| 332 | *n*-C₃H₇ | F | H | H | H | |
| 333 | *n*-C₄H₉ | F | H | H | H | |
| 334 | *n*-C₅H₁₁ | F | H | H | H | |
| 335 | *n*-C₆H₁₃ | F | H | H | H | |
| 336 | *n*-C₇H₁₅ | F | H | H | H | |
| 337 | *n*-C₈H₁₇ | F | H | H | H | |
| 338 | *n*-C₉H₁₉ | F | H | H | H | |
| 339 | *n*-C₁₀H₂₁ | F | H | H | H | |
| 340 | CH₂=CH | F | H | H | H | |
| 341 | CH₂=CH-CH₂ | F | H | H | H | |
| 342 | CH₃-CH=CH | F | H | H | H | |
| 343 | CH₃ | F | F | H | H | |
| 344 | C₂H₅ | F | F | H | H | |
| 345 | *n*-C₃H₇ | F | F | H | H | |
| 346 | *n*-C₄H₉ | F | F | H | H | |
| 347 | *n*-C₅H₁₁ | F | F | H | H | |
| 348 | *n*-C₆H₁₃ | F | F | H | H | |
| 349 | *n*-C₇H₁₅ | F | F | H | H | |
| 350 | *n*-C₈H₁₇ | F | F | H | H | |
| 351 | *n*-C₉H₁₉ | F | F | H | H | |
| 352 | *n*-C₁₀H₂₁ | F | F | H | H | |
| 353 | CH₂=CH | F | F | H | H | |
| 354 | CH₂=CH-CH₂ | F | F | H | H | |
| 355 | CH₃-CH=CH | F | F | H | H | |
| 356 | CH₃ | F | F | F | H | |
| 357 | C₂H₅ | F | F | F | H | |
| 358 | *n*-C₃H₇ | F | F | F | H | |
| 359 | *n*-C₄H₉ | F | F | F | H | |
| 360 | *n*-C₅H₁₁ | F | F | F | H | |
| 361 | *n*-C₆H₁₃ | F | F | F | H | |
| 362 | *n*-C₇H₁₅ | F | F | F | H | |
| 363 | *n*-C₈H₁₇ | F | F | F | H | |
| 364 | *n*-C₉H₁₉ | F | F | F | H | |
| 365 | *n*-C₁₀H₂₁ | F | F | F | H | |
| 366 | CH₂=CH | F | F | F | H | |
| 367 | CH₂=CH-CH₂ | F | F | F | H | |
| 368 | CH₃-CH=CH | F | F | F | H | |
| 369 | CH₃ | F | H | F | F | |
| 370 | C₂H₅ | F | H | F | F | |
| 371 | *n*-C₃H₇ | F | H | F | F | |
| 372 | *n*-C₄H₉ | F | H | F | F | |
| 373 | *n*-C₅H₁₁ | F | H | F | F | |
| 374 | *n*-C₆H₁₃ | F | H | F | F | |
| 375 | *n*-C₇H₁₅ | F | H | F | F | |
| 376 | *n*-C₈H₁₇ | F | H | F | F | |
| 377 | *n*-C₉H₁₉ | F | H | F | F | |
| 378 | *n*-C₁₀H₂₁ | F | H | F | F | |
| 379 | CH₂=CH | F | H | F | F | |
| 380 | CH₂=CH-CH₂ | F | H | F | F | |
| 381 | CH₃-CH=CH | F | H | F | F | |
| 382 | CH₃ | Cl | H | H | H | |
| 383 | C₂H₅ | Cl | H | H | H | |
| 384 | *n*-C₃H₇ | Cl | H | H | H | |
| 385 | *n*-C₄H₉ | Cl | H | H | H | |
| 386 | *n*-C₅H₁₁ | Cl | H | H | H | |
| 387 | *n*-C₆H₁₃ | Cl | H | H | H | |
| 388 | *n*-C₇H₁₅ | Cl | H | H | H | |
| 389 | *n*-C₈H₁₇ | Cl | H | H | H | |
| 390 | *n*-C₉H₁₉ | Cl | H | H | H | |
| 391 | *n*-C₁₀H₂₁ | Cl | H | H | H | |
| 392 | CH₂=CH | Cl | H | H | H | |
| 393 | CH₂=CH-CH₂ | Cl | H | H | H | |
| 394 | CH₃-CH=CH | Cl | H | H | H | |
| 395 | CH₃ | Cl | F | H | H | |
| 396 | C₂H₅ | Cl | F | H | H | |
| 397 | *n*-C₃H₇ | Cl | F | H | H | |
| 398 | *n*-C₄H₉ | Cl | F | H | H | |
| 399 | *n*-C₅H₁₁ | Cl | F | H | H | |
| 400 | *n*-C₆H₁₃ | Cl | F | H | H | |
| 401 | *n*-C₇H₁₅ | Cl | F | H | H | |
| 402 | *n*-C₈H₁₇ | Cl | F | H | H | |
| 403 | *n*-C₉H₁₉ | Cl | F | H | H | |
| 404 | *n*-C₁₀H₂₁ | Cl | F | H | H | |
| 405 | CH₂=CH | Cl | F | H | H | |
| 406 | CH₂=CH-CH₂ | Cl | F | H | H | |
| 407 | CH₃-CH=CH | Cl | F | H | H | |
| 408 | CH₃ | Cl | F | F | H | |
| 409 | C₂H₅ | Cl | F | F | H | |
| 410 | *n*-C₃H₇ | Cl | F | F | H | |
| 411 | *n*-C₄H₉ | Cl | F | F | H | |
| 412 | *n*-C₅H₁₁ | Cl | F | F | H | |
| 413 | *n*-C₆H₁₃ | Cl | F | F | H | |
| 414 | *n*-C₇H₁₅ | Cl | F | F | H | |
| 415 | *n*-C₈H₁₇ | Cl | F | F | H | |
| 416 | *n*-C₉H₁₉ | Cl | F | F | H | |
| 417 | *n*-C₁₀H₂₁ | Cl | F | F | H | |
| 418 | CH₂=CH | Cl | F | F | H | |
| 419 | CH₂=CH-CH₂ | Cl | F | F | H | |
| 420 | CH₃-CH=CH | Cl | F | F | H | |
| 421 | CH₃ | Cl | H | F | F | |
| 422 | C₂H₅ | Cl | H | F | F | |
| 423 | *n*-C₃H₇ | Cl | H | F | F | |
| 424 | *n*-C₄H₉ | Cl | H | F | F | |
| 425 | *n*-C₅H₁₁ | Cl | H | F | F | |
| 426 | *n*-C₆H₁₃ | Cl | H | F | F | |
| 427 | *n*-C₇H₁₅ | Cl | H | F | F | |
| 428 | *n*-C₈H₁₇ | Cl | H | F | F | |
| 429 | *n*-C₉H₁₉ | Cl | H | F | F | |
| 430 | *n*-C₁₀H₂₁ | Cl | H | F | F | |
| 431 | CH₂=CH | Cl | H | F | F | |
| 432 | CH₂=CH-CH₂ | Cl | H | F | F | |
| 433 | CH₃-CH=CH | Cl | H | F | F | |
| 434 | CH₃ | CF₃ | H | H | H | |
| 435 | C₂H₅ | CF₃ | H | H | H | |
| 436 | *n*-C₃H₇ | CF₃ | H | H | H | |
| 437 | *n*-C₄H₉ | CF₃ | H | H | H | |
| 438 | *n*-C₅H₁₁ | CF₃ | H | H | H | |
| 3 | *n*-C₆H₁₃ | CF₃ | H | H | H | |
| 439 | *n*-C₇H₁₅ | CF₃ | H | H | H | |
| 440 | *n*-C₈H₁₇ | CF₃ | H | H | H | |
| 441 | *n*-C₉H₁₉ | CF₃ | H | H | H | |
| 442 | *n*-C₁₀H₂₁ | CF₃ | H | H | H | |
| 443 | CH₂=CH | CF₃ | H | H | H | |
| 444 | CH₂=CH-CH₂ | CF₃ | H | H | H | |
| 445 | CH₃-CH=CH | CF₃ | H | H | H | |
| 446 | CH₃ | CF₃ | F | H | H | |
| 447 | C₂H₅ | CF₃ | F | H | H | |
| 448 | *n*-C₃H₇ | CF₃ | F | H | H | |
| 449 | *n*-C₄H₉ | CF₃ | F | H | H | |
| 450 | *n*-C₅H₁₁ | CF₃ | F | H | H | |
| 451 | *n*-C₆H₁₃ | CF₃ | F | H | H | |
| 452 | *n*-C₇H₁₅ | CF₃ | F | H | H | |
| 453 | *n*-C₈H₁₇ | CF₃ | F | H | H | |
| 454 | *n*-C₉H₁₉ | CF₃ | F | H | H | |
| 455 | *n*-C₁₀H₂₁ | CF₃ | F | H | H | |
| 456 | CH₂=CH | CF₃ | F | H | H | |
| 457 | CH₂=CH-CH₂ | CF₃ | F | H | H | |
| 458 | CH₃-CH=CH | CF₃ | F | H | H | |
| 459 | CH₃ | CF₃ | F | F | H | |
| 460 | C₂H₅ | CF₃ | F | F | H | |
| 461 | *n*-C₃H₇ | CF₃ | F | F | H | |
| 462 | *n*-C₄H₉ | CF₃ | F | F | H | |
| 463 | *n*-C₅H₁₁ | CF₃ | F | F | H | |
| 464 | *n*-C₆H₁₃ | CF₃ | F | F | H | |
| 465 | *n*-C₇H₁₅ | CF₃ | F | F | H | |
| 466 | *n*-C₈H₁₇ | CF₃ | F | F | H | |
| 467 | *n*-C₉H₁₉ | CF₃ | F | F | H | |
| 468 | *n*-C₁₀H₂₁ | CF₃ | F | F | H | |
| 469 | CH₂=CH | CF₃ | F | F | H | |
| 470 | CH₂=CH-CH₂ | CF₃ | F | F | H | |
| 471 | CH₃-CH=CH | CF₃ | F | F | H | |
| 472 | CH₃ | CF₃ | H | F | F | |
| 473 | C₂H₅ | CF₃ | H | F | F | |
| 474 | *n*-C₃H₇ | CF₃ | H | F | F | |
| 475 | *n*-C₄H₉ | CF₃ | H | F | F | |
| 476 | *n*-C₅H₁₁ | CF₃ | H | F | F | |
| 477 | *n*-C₆H₁₃ | CF₃ | H | F | F | |
| 478 | *n*-C₇H₁₅ | CF₃ | H | F | F | |
| 479 | *n*-C₈H₁₇ | CF₃ | H | F | F | |
| 480 | *n*-C₉H₁₉ | CF₃ | H | F | F | |
| 481 | *n*-C₁₀H₂₁ | CF₃ | H | F | F | |
| 482 | CH₂=CH | CF₃ | H | F | F | |
| 483 | CH₂=CH-CH₂ | CF₃ | H | F | F | |
| 484 | CH₃-CH=CH | CF₃ | H | F | F | |

### Comparative Use-example

5% of the chiral agent R-5011 are solved in the achiral liquid crystal mixture H-0 with the composition and properties given in table 1 below.

**Table 1: Composition and Properties of Host Mixture H-0**

| Compound Abbreviation | Concentration / mass-% | Physical Properties |
|---|---|---|
| GZU-3A-N | 15.0 | T(N, I) = 56.5 °C |
| GZU-4A-N | 15.0 | |
| GZU-4O-N | 15.0 | Δn (20°C, 589 nm) = 0.164 |
| UZU-3A-N | 8.0 | |
| CUZU-2-N | 9.0 | |
| CUZU-3-N | 9.0 | |
| CUZU-4-N | 9.0 | |
| HP-3N.F | 6.0 | |
| HP-4N.F | 6.0 | |
| HP-5N.F | 8.0 | |
| ∑ | 100.0 | |

The resulting mixture CM-0 is filled into an electro optical test cell with interdigital electrodes on one substrate side. The electrode width is 10 µm, the distance between adjacent electrodes is 10 µm and the cell gap is also 10 µm. This test cell is evaluated electro-optically between crossed polarisers.

At low temperatures, the filled cell showed the typical texture of a chiral nematic mixture, with an optical transmission between crossed polarisers without applied voltage. On heating, at a temperature of 36°C the mixture was optically isotropic, being dark between the crossed polarisers.

This indicated the transition from the chiral nematic phase to the blue phase at 36°C. This temperature is called T₁ or Tₜᵣₐₙₛ.

Up to a temperature of 43°C the cell shows a clear electro optical effect under applied voltage, for example at 38°C, applying a voltage of 46 V leads to a maximum of the optical transition. This temperature is called T₂ and threspective voltage is called Vₘₐₓ or V₁₀₀. At a temperature of 43°C the voltage needed for a visible electro-optical effect starts to increase strongly, indicating the transition from the blue phase to the isotropic phase at this temperature.

The temperature range (ΔT(BP)), where the mixture can be used electro-optically in the blue phase is identified as ranging from about 36°C to about 43°C, i.e. as being 7° wide (= T₂ - T₁ = 43°C - 36°C). The results are listed in table 2 below. Further the response times for switching on (τₒₙ) and for switching off (τ_{off}) are been determined. The response times decrease with increasing temperature above T₁ and the temperature at which both response times have fallen below 5 ms each is called T₃. This is the case in this comparative use example at a temperature of about 39.3°C or slightly above. Thus, the range of usable flat behaviour i.e. the usable flat range (ΔT(FR)), which is defined as ΔT(FR) = T₂ - T₃, in case T₂ ≥ T₃ and ΔT(FR) = 0, in case T₂ < T₃, is (43.0°C-39.3°C) = 3,7° in this comparative use example.

### Use-example 1

In this use-example alternatively 10 % of the compounds of examples 1 to 4, respectively, are solved each together with 5% of the chiral agent R-5011 in the achiral liquid crystal mixture H-0 used in the comparative use-example 1 described above. The resultant mixtures H-1a and H-2 to H-4 have the compositions and properties shown in table 2. As the mixture H-1 a containing 10 % of the compound of example 1 shows a blue phase only over a narrow range of temperatures alternatively 5 % of the compound are solved together with 5 % of the chiral dopant R-5011 and the resultant mixture H-1b is investigated.

**Table 2: Results**

| | | | | | | |
|---|---|---|---|---|---|---|
| Use-Ex. # | C.E. | 1-1a | 1-1b | 1-2 | 1-3 | 1-4 |
| Mixture # | CM-0 | H-1a | H-1b | H-2 | H-3 | H-4 |
| Cpd. of Ex. # | None | 1 | | 2 | 3 | 4 |
| c(Cpd.)/% | 0 | 10 | 5 | 10 | | |
| c(R-5011)/% | 0 | 5 | | | | |

| Characteristic Temparatures | | | | | | |
|---|---|---|---|---|---|---|
| T₂/°C | 43.0 | 5.9 | 20.2 | 16.5 | 20.0 | 20.5 |
| T₃/°C | 39.3 | n.d. | 19.2 | 15.5 | 15.6 | 17.5 |
| T₁/°C | 36.0 | 4.9 | 9.7 | 6.2 | 11.1 | 13.1 |
| ΔT(BP) /° | 7.0 | 1.0 | 10.5 | 10.3 | 8.9 | 7.4 |
| ΔT(FR) /° | 3.7 | n.d. | 1.0 | 1.0 | 4.4 | 3.0 |

| Characteristic Voltages | | | | | | |
|---|---|---|---|---|---|---|
| T_{op.} /°C | 38.0 | 6.9 | 11.7 | 8.2 | 13.1 | 15.1 |
| Vₘₐₓ/V | 46.0 | n.d. | 41.1 | 44.6 | 44.9 | 45.1 |
| dVₘₐₓ/dT/V/° | n.d. | n.d. | 0.0 | 1.0 | 1.1 | 2.2 |
| dVₘₐₓ/dT/V₀/° | n.d. | n.d. | 0.00 | 0.022 | 0.024 | 0.049 |

| | | | | | | |
|---|---|---|---|---|---|---|
| Remarks: n.d.: not determined. | | | | | | |

The resulting mixtures H-1 a, H-1 b and H-2 to H-4 are filled into respective electro optical test cells like those used in the comparative use-example 1 and investigated as described there.

At low temperatures, the cell filled with the mixture H-1b shows the typical texture of a chiral nematic mixture, with an optical transmission between crossed polarisers without applied voltage. On heating, at a temperature of 9.7°C the mixture becomes optically isotropic, being dark between the crossed polarisers. This indicates the transition from the chiral nematic phase to the blue phase at 9.7°C. Up to a temperature of 20.2°C, the cell shows a clear electro optical effect under applied voltage. For example at 11.7°C, applying 41.1 volts lead to a maximum of the optical transition. At a temperature of 20.2°C the voltage needed for a visible electro- optical effect increases strongly, indicating the transition from the blue phase to the isotropic phase at 20.2 °C.

The range, where the mixture can be used electro-optically in the blue phase is thus identified as 20.2°C - 9.7°C = 10.5°.

This is significantly larger than the range of 7°, being found in the mixture CM-0 of the comparative use example 1, which is containing 5% R-5011, only.

The results are listed in table 2.

The mixtures H-2 to H-4 are investigated in the same way as the mixture H-1 b. The results are also listed in table 2. All use-examples investigated show a larger temperature range compared to the comparative use-example and at the same time characteristic voltage even is reduced.

### Use-example 2

In this use-example a nematic liquid crystal mixture (called A) has been prepared, which contains 10 % of the compound of example 1, and evaluated for its physical properties, as given in the following table.

**Table 3: Composition and Properties of Mixture A**

| Compound Abbreviation | Concentration / mass-% | Physical Properties |
|---|---|---|
| Cpd.(Ex. 1) | 10.0 | T(N, I) = 52.5 °C |
| PCH-5F | 9.0 | |
| PCH-6F | 7.2 | Δn (20°C, 589 nm) = 0.805 |
| PCH-7F | 5.4 | |
| CCP-2OCF3 | 7.2 | Δε(20°C) = 4.6 |
| CCP-3OCF3 | 10.8 | |
| CCP-4OCF3 | 6.3 | |
| CCP-5OCF3 | 9.9 | |
| ECCP-3OCF3 | 4.5 | |
| ECCP-5OCF3 | 4.5 | |
| BCH-3F.F | 10.8 | |
| BCH-5F.F | 9.0 | |
| CBC-33F | 1.8 | |
| CBC-53F | 1.8 | |
| CBC-55F | 1.8 | |
| Σ | 100.0 | |

### Use-example 3

In this use-example a nematic liquid crystal mixture (called B) has been prepared, which contains 10 % of the compound of example 2, and evaluated for its physical properties, as given in the following table.

**Table 4: Composition and Properties of Mixture B**

| Compound Abbreviation | Concentration / mass-% | Physical Properties |
|---|---|---|
| Cpd.(Ex. 2) | 10.0 | T(N, I) = 67.5 °C |
| PCH-5F | 9.0 | |
| PCH-6F | 7.2 | Δn (20°C, 589 nm) = 0.884 |
| PCH-7F | 5.4 | |
| CCP-2OCF3 | 7.2 | Δε(20°C) = 4.1 |
| CCP-3OCF3 | 10.8 | |
| CCP-4OCF3 | 6.3 | |
| CCP-5OCF3 | 9.9 | |
| ECCP-3OCF3 | 4.5 | |
| ECCP-5OCF3 | 4.5 | |
| BCH-3F.F | 10.8 | |
| BCH-5F.F | 9.0 | |
| CBC-33F | 1.8 | |
| CBC-53F | 1.8 | |
| CBC-55F | 1.8 | |
| Σ | 100.0 | |

### Use-example 4

In this use-example a nematic liquid crystal mixture (called C) has been prepared, which contains 10 % of the compound of example 3, and evaluated for its physical properties, as given in the following table.

**Table 5: Composition and Properties of Mixture C**

| Compound Abbreviation | Concentration / mass-% | Physical Properties |
|---|---|---|
| Cpd.(Ex. 3) | 10.0 | T(N, I) = n.d. °C |
| PCH-5F | 9.0 | |
| PCH-6F | 7.2 | Δn (20°C, 589 nm) = n.d. |
| PCH-7F | 5.4 | |
| CCP-2OCF3 | 7.2 | Δε(20°C) = n.d. |
| CCP-3OCF3 | 10.8 | |
| CCP-4OCF3 | 6.3 | |
| CCP-5OCF3 | 9.9 | |
| ECCP-3OCF3 | 4.5 | |
| ECCP-5OCF3 | 4.5 | |
| BCH-3F.F | 10.8 | |
| BCH-5F.F | 9.0 | |
| CBC-33F | 1.8 | |
| CBC-53F | 1.8 | |
| CBC-55F | 1.8 | |
| Σ | 100.0 | |

| | | |
|---|---|---|
| Remarks: n.d.: not determined. | | |

### Use-example 5

In this use-example a nematic liquid crystal mixture (called D) has been prepared, which contains 10 % of the compound of example 4, and evaluated for its physical properties, as given in the following table.

**Table 6: Composition and Properties of Mixture D**

| Compound Abbreviation | Concentration / mass-% | Physical Properties |
|---|---|---|
| Cpd.(Ex. 4) | 10.0 | T(N, I) = 73.0 °C |
| PCH-5F | 9.0 | |
| PCH-6F | 7.2 | Δn (20°C, 589 nm) = 0.937 |
| PCH-7F | 5.4 | |
| CCP-2OCF3 | 7.2 | Δε(20°C) = 5.2 |
| CCP-3OCF3 | 10.8 | |
| CCP-4OCF3 | 6.3 | |
| CCP-5OCF3 | 9.9 | |
| ECCP-3OCF3 | 4.5 | |
| ECCP-5OCF3 | 4.5 | |
| BCH-3F.F | 10.8 | |
| BCH-5F.F | 9.0 | |
| CBC-33F | 1.8 | |
| CBC-53F | 1.8 | |
| CBC-55F | 1.8 | |
| Σ | 100.0 | |

## Claims

1. Compound of formula I 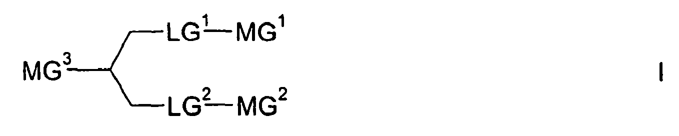 wherein
LG¹ and LG² are, independently of one another, LG, and
LG is -O-, -O-(C=O)- or -OCF₂-,
MG¹, MG² and MG ³ are, independently of each other, MG, and
MG in each occurrence, independently of one another, is 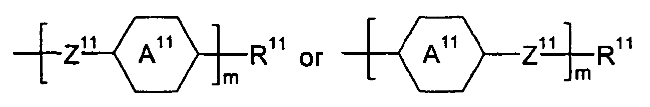
wherin
R¹¹ is H, F, Cl, CN, NCS, SF₅ , SO₂CF₃ or alkyl, which is straight chain or branched, is unsubstituted, mono- or poly-substituted by F, Cl, or CN, and in which one or more non-adjacent CH₂ groups are optionally replaced, in each case independently from one another, by -O-, -S-,-NH-, -NR⁰¹-, -SiR⁰¹R⁰²-, -CO-, -COO-, -OCO-, -OCO-O-, -S-CO-, -CO-S-, -CY⁰¹=CY⁰¹- or -C≡C- in such a manner that O and/or S atoms are not linked directly to one another, 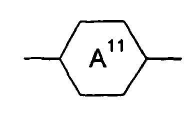 is and, in case it is occurring more than once, also these are in each occurrence, independently of each other, an aromatic and/or alicyclic ring, or a group comprising two or more fused aromatic or alicyclic rings, wherein these rings optionally contain one or more hetero atoms selected from N, O and/or S, and are optionally monosubstituted or polysubstituted by R,
R has the meaning given for R¹¹ and preferably is alkyl with 1 to 12 C-atoms,
Z¹¹ is, and in case it is occurring more than once, these are in each occurrence, independently of each other, -CO-O-, -O-CO-, -S-CO-, -CO-S-, -CO-NR⁰¹-, -NR⁰¹-CO-, -OCH₂-, -CH₂O-, -SCH₂-, -CH₂S-, -CF₂O-, -OCF₂-, -CF₂S-, -SCF₂-. -CH₂CH₂-, -CF₂CH₂-, -CH₂CF₂-, -CF₂CF₂-, -CH=N-, -N=CH-, -N=N-, -CH=CR⁰¹-, -CR⁰¹=CH-, -CY⁰¹=CY⁰²-, -C≡C-, -(CH₂)₄-, -CH=CH-CO-O-, -O-CO-CH=CH- or a single bond,
Y⁰¹ and Y⁰² are, independently of each other, F, Cl or CN, and alternatively one of them may be H,
R⁰¹ and R⁰² are, independently of each other, H or alkyl with 1 to 12 C-atoms,
m is , in each occurrence independently, 1, 2 or 3.

2. Compound according to claim 1, **characterized in that** it is of formula I' or of Formula I" 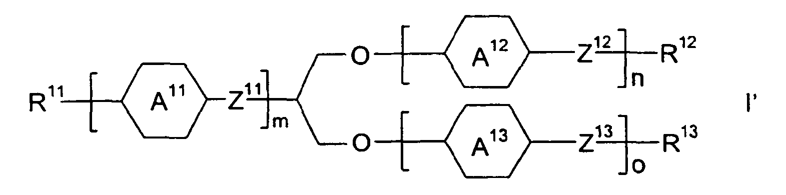 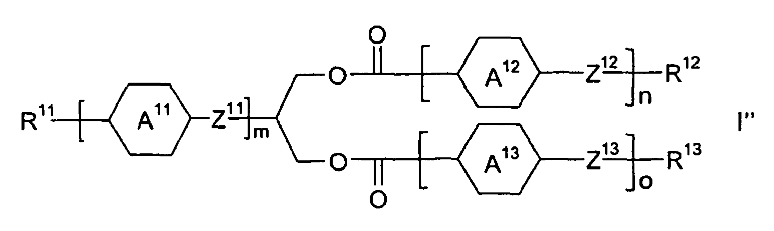 wherein the parameters have the respective meanings given in claim 1 and
R¹² and R¹³, independently from one another, have the meaning given for R¹¹ in claim 1, 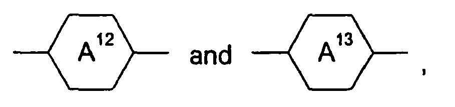 independently of each other in each occurrence, have the meaning given for 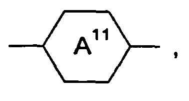 in claim 1
Z¹² and Z¹³, independently from one another, have the meaning given for Z¹¹ in claim 1,
n and o, independently from one another, have the meaning given for m in claim 1.

3. Compound according to atleast one of claims 1 and 2, **characterized in that** at least one of the groups 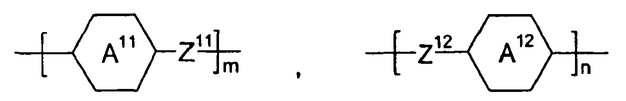 and, 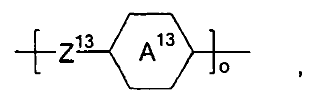 most preferably all of them, are selected from the following formulae la to Ir and their respective mirror images 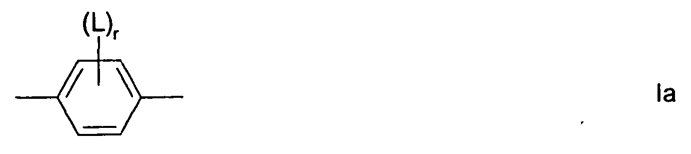 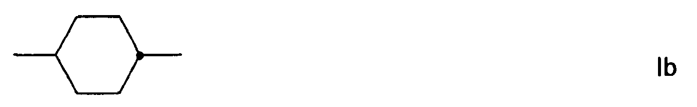 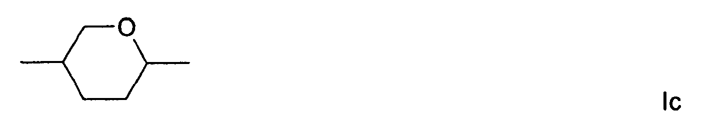 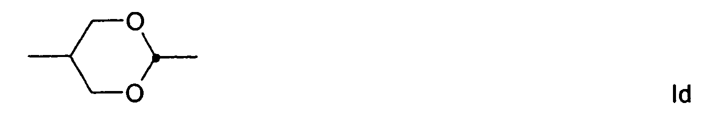 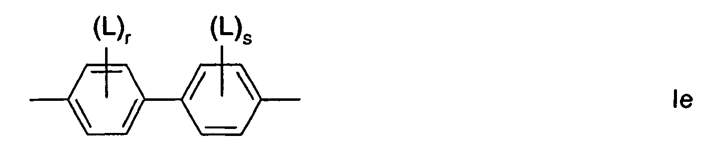 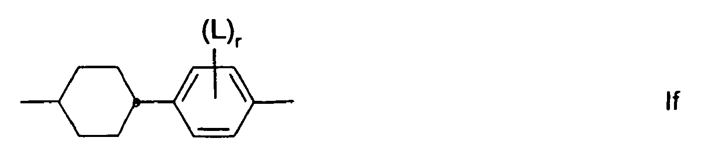 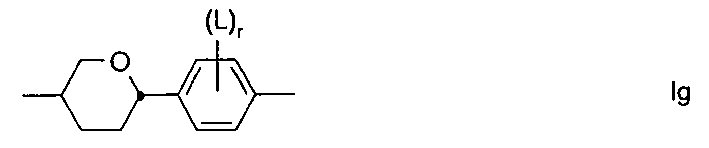 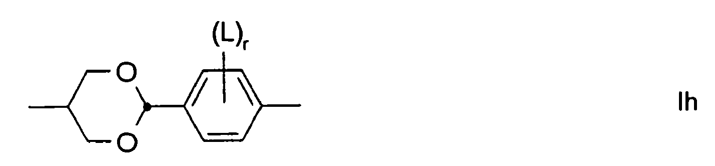 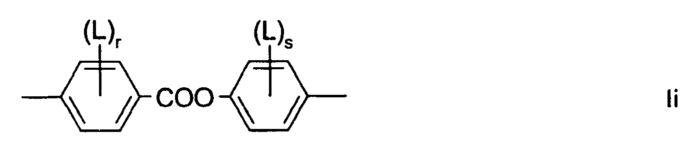 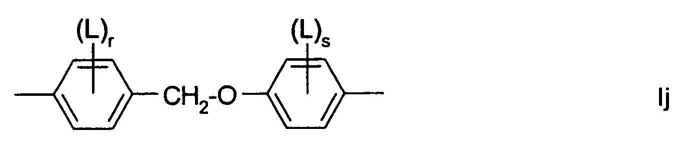 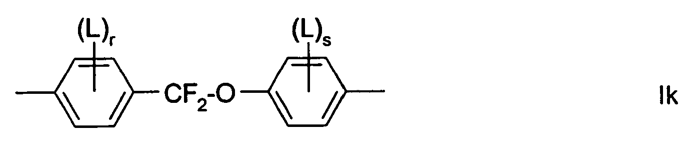 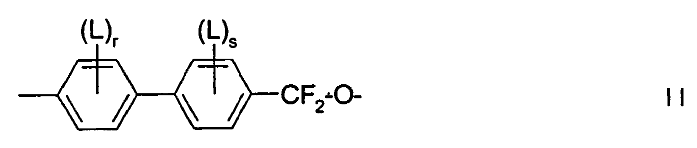 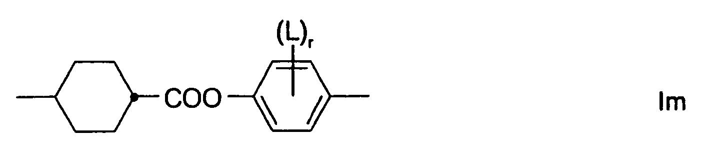 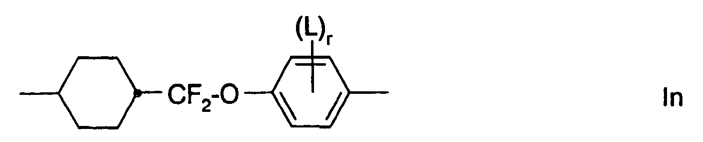 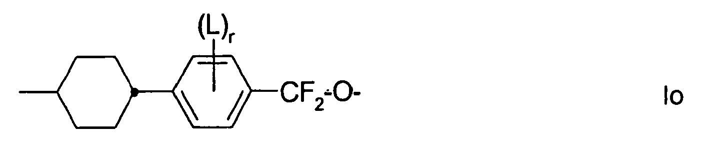 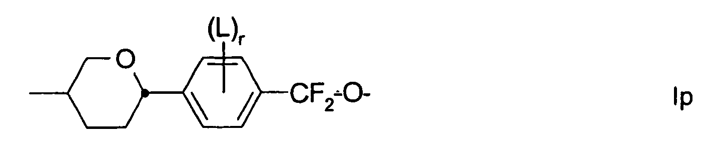 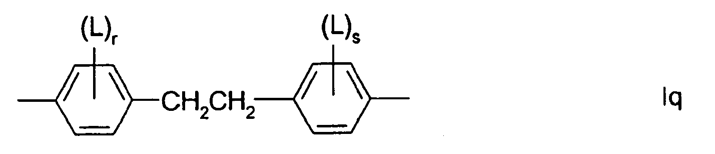 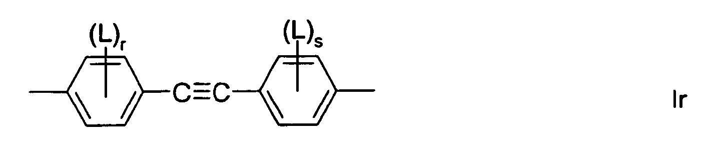 wherein L has the meaning given above and r and s are independently of each other, 0, 1, 2, 3 or 4.

4. Compound according to at least one of claims 1 to 3, **characterized in that** it comprises two mesogenic groups MG¹ and MG³, which are identical to each other.

5. Medium, **characterized in that** it comprises a compound according to at least one of claims 1 and 4.

6. Medium according to claims 5, **characterized in that** it is a light modulation medium and that it has a blue phase.

7. Light modulation element, **characterized in that** it comprises a medium according to at least one of claims 5 and 6.

8. Use of a compound according to at least one of claims 1 to 4 in a mesogenic medium.

9. Use of a medium according to at least one of claims 5 and 6 in a light modulation element.

10. Electro-optical display, **characterized in that** it comprises a medium according to at least one of claims 5 and 6.

## Patentansprüche

1. Verbindung der Formel I 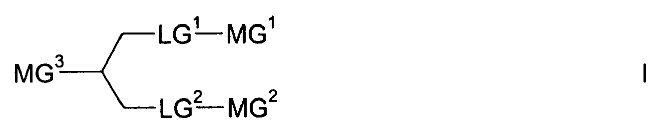 worin
LG¹ und LG² unabhängig voneinander LG bedeuten und
LG -O-, -O-(C=O)- oder -OCF₂- bedeutet,
MG¹, MG² und MG ³ unabhängig voneinander MG bedeuten und
MG bei jedem Auftreten unabhängig voneinander 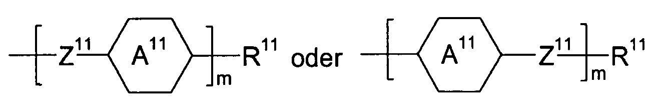 bedeutet,
worin
R¹¹ H, F, Cl, CN, NCS, SF₅ , SO₂CF₃ oder Alkyl bedeutet, das geradkettig oder verzweigt ist und unsubstituiert oder ein- oder mehrfach durch F, Cl oder CN substituiert ist und in dem gegebenenfalls eine oder mehrere nicht benachbarte CH₂-Gruppen jeweils unabhängig voneinander so durch -O-, -S-, -NH-, -NR⁰¹-, -SiR⁰¹R⁰²-, -CO-, -COO-, -OCO-, -OCO-O-, -S-CO-, -CO-S-, -CY⁰¹=CY⁰¹- oder -C≡C- ersetzt sind, dass O- und/oder S-Atome nicht direkt miteinander verknüpft sind, 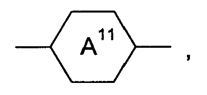 und für den Fall, dass er mehr als einmal auftritt, auch diese bei jedem Auftreten unabhängig voneinander einen aromatischen und/oder alicyclischen Ring oder eine Gruppe mit zwei oder mehr anellierten aromatischen oder alicyclischen Ringen bedeuten, worin diese Ringe gegebenenfalls ein oder mehrere Heteroatome ausgewählt aus N, O und/oder S enthalten und gegebenenfalls einfach oder mehrfach durch R substituiert sind,
R die für R¹¹ angegebene Bedeutung besitzt und vorzugsweise Alkyl mit 1 bis 12 C-Atomen bedeutet,
Z¹¹, und für den Fall, dass es mehr als einmal auftritt, auch diese bei jedem Auftreten unabhängig voneinander -CO-O-, -O-CO-, -S-CO-, -CO-S-, -CO-NR⁰¹-, -NR⁰¹-CO-, -OCH₂-, -CH₂O-, -SCH₂-, -CH₂S-, -CF₂O-, -OCF₂-, -CF₂S-, -SCF₂-, -CH₂CH₂-, -CF₂CH₂-, -CH₂CF₂-, -CF₂CF₂-, -CH=N-, -N=CH-, -N=N-, -CH=CR⁰¹-, -CR⁰¹=CH-, -CY⁰¹=CY⁰²-, -C≡C-, -(CH₂)₄-, -CH=CH-CO-O-, -O-CO-CH=CH- oder eine Einfachbindung bedeuten,
Y⁰¹ und Y⁰² unabhängig voneinander F, Cl oder CN bedeuten und alternativ eines von ihnen H bedeuten kann,
R⁰¹ und R⁰² unabhängig voneinander H oder Alkyl mit 1 bis 12 C-Atomen bedeuten,
m bei jedem Auftreten unabhängig 1, 2 oder 3 bedeutet.

2. Verbindung nach Anspruch 1, **dadurch gekennzeichnet, dass** sie die Formel I' oder die Formel I" 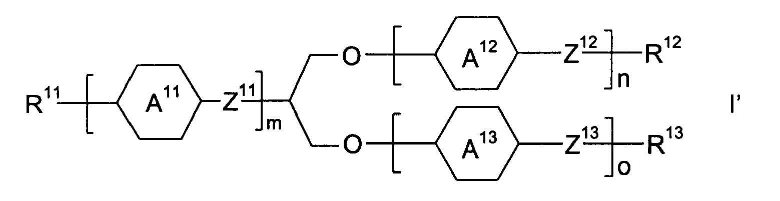 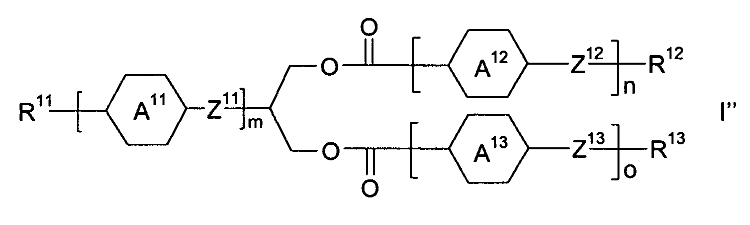 besitzt, worin die Parameter die jeweiligen in Anspruch 1 angegebenen Bedeutungen besitzen und
R¹² und R¹³ unabhängig voneinander die für R¹¹ in Anspruch 1 angegebene Bedeutung besitzen, 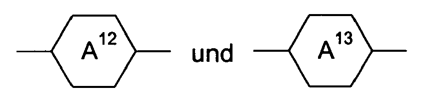 bei jedem Auftreten unabhängig voneinander die für 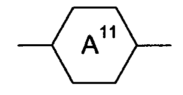 in Anspruch 1 angegebene Bedeutung besitzen,
Z¹² und Z¹³ unabhängig voneinander die für Z¹¹ in Anspruch 1 angegebene Bedeutung besitzen,
n und o unabhängig voneinander die für m in Anspruch 1 angegebene Bedeutung besitzen.

3. Verbindung nach mindestens einem der Ansprüche 1 und 2, **dadurch gekennzeichnet, dass** mindestens eine der Gruppen 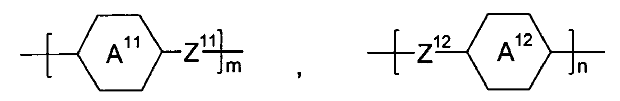 und 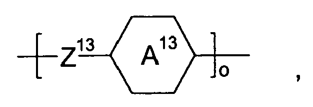 insbesondere bevorzugt alle von ihnen, ausgewählt sind aus den folgenden Formeln la bis Ir und deren jeweiligen Spiegelbildern 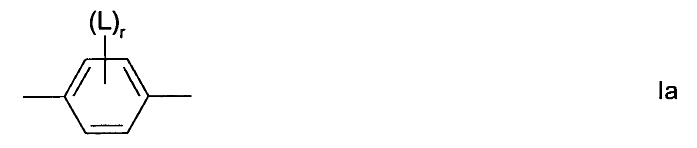 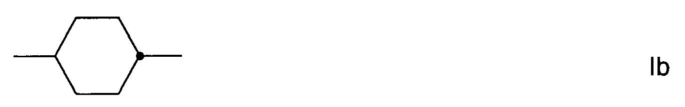 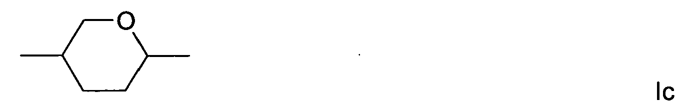 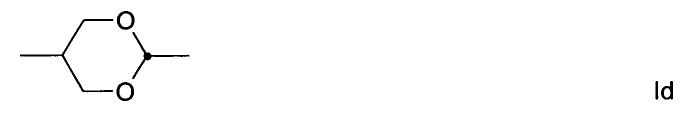 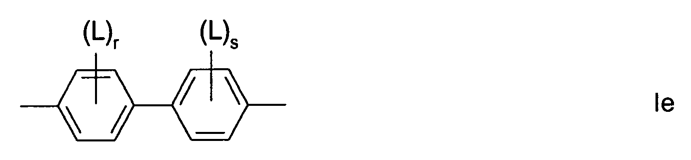 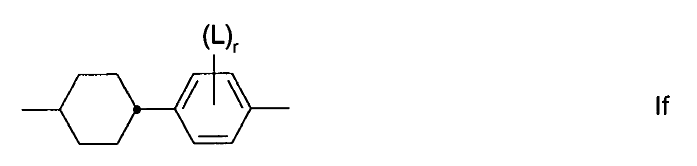 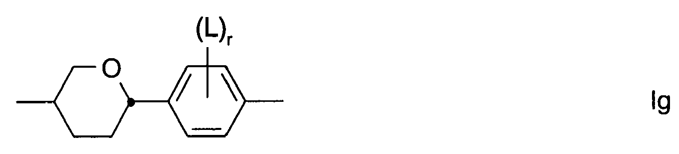 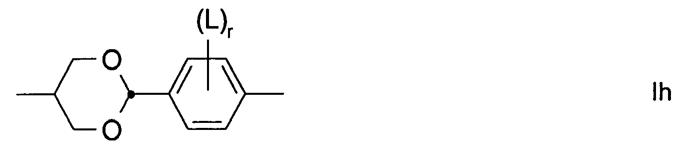 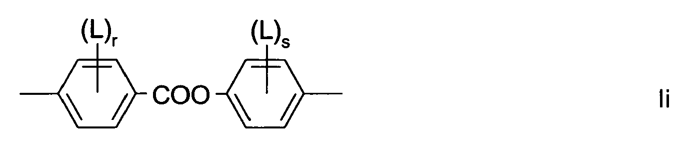 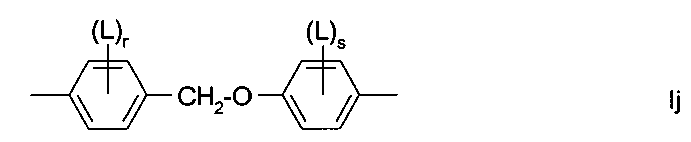 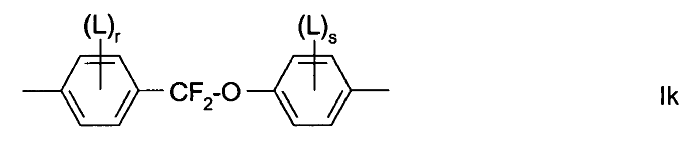 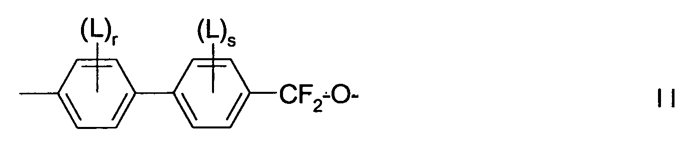 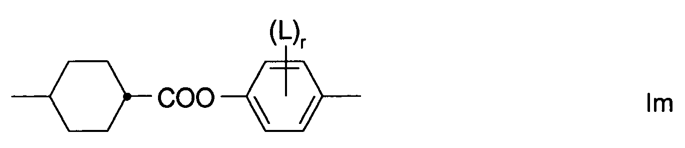 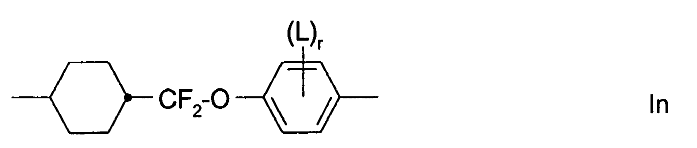 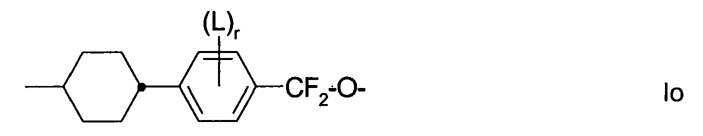 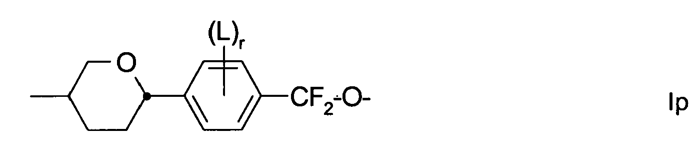 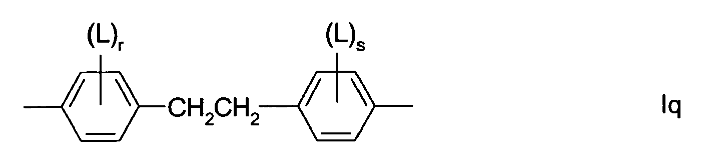 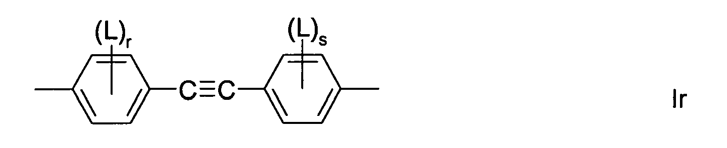 worin L die oben angegebene Bedeutung besitzt und r und s unabhängig voneinander 0, 1, 2, 3 oder 4 bedeuten.

4. Verbindung nach mindestens einem der Ansprüche 1 bis 3, **dadurch gekennzeichnet, dass** sie zwei mesogene Gruppen MG¹ und MG³ enthält, die einander gleich sind.

5. Medium, **dadurch gekennzeichnet, dass** es eine Verbindung nach mindestens einem der Ansprüche 1 und 4 enthält.

6. Medium nach Anspruch 5, **dadurch gekennzeichnet, dass** es sich um ein Lichtmodulationsmedium handelt und dass es eine blaue Phase aufweist.

7. Lichtmodulationselement, **dadurch gekennzeichnet, dass** es ein Medium nach mindestens einem der Ansprüche 5 und 6 enthält.

8. Verwendung einer Verbindung nach mindestens einem der Ansprüche 1 bis 4 in einem mesogenen Medium.

9. Verwendung eines Mediums nach mindestens einem der Ansprüche 5 und 6 in einem Lichtmodulationselement.

10. Elektrooptische Anzeige, **dadurch gekennzeichnet, dass** sie ein Medium nach mindestens einem der Ansprüche 5 und 6 enthält.

## Revendications

1. Composé de la formule I 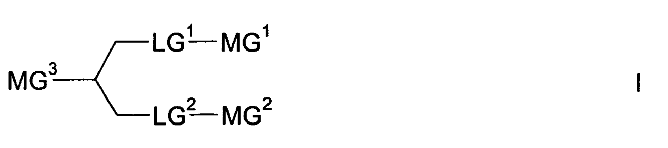 dans laquelle
LG¹ et LG² sont, indépendamment l'un de l'autre, LG, et
LG est -O-, -O-(C=O)- ou -OCF₂-,
MG¹, MG² et MG³ sont, indépendamment les uns des autres, MG, et
MG pour chaque occurrence, indépendamment les unes des autres, est 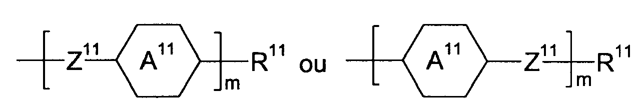
dans laquelle
R¹¹ est H, F, Cl, CN, NCS, SF₅, SO₂CF₃ ou alkyle, qui est en chaîne droite ou ramifié, non substitué, monosubstitué ou polysubstitué par F, Cl ou CN et où un ou plusieurs groupes CH₂ non adjacents sont en option remplacés, dans chaque cas indépendamment les uns des autres, par -O-, -S-, -NH-, -NR⁰¹-, -SiR⁰¹R⁰²-, -CO-, -COO-, -OCO-, -OCO-O-, -S-CO-, -CO-S-, -CY⁰¹=CY⁰¹- ou -C≡C- de telle sorte que des atomes de O et/ou de S ne soient pas liés directement les uns aux autres, 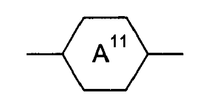 est, et dans le cas où son occurrence est plus d'une fois, également ces occurrences, sont, pour chaque occurrence, indépendamment les uns des autres, un cycle aromatique et/ou alicyclique ou un groupe comprenant deux cycles aromatiques ou alicycliques fusionnés ou plus, dans lequel ces cycles contiennent en option un ou plusieurs hétéro-atomes choisis parmi N, O et/ou S et sont en option monosubstitués ou polysubstitués par R,
R présente la signification donnée pour R¹¹et de préférence, est alkyle avec de 1 à 12 atomes de C,
Z¹¹ est, et dans le cas où son occurrence est plus d'une fois, ces occurrences, sont, pour chaque occurrence, indépendamment les uns des autres, -CO-O-, -O-CO-, -S-CO-, -CO-S-, -CO-NR⁰¹-, -NR⁰¹-CO-, -OCH₂-, -CH₂O-, -SCH₂-, -CH₂S-, -CF₂O-, -OCF₂-, -CF₂S-, -SCF₂-, -CH₂CH₂-, -CF₂CH₂-, -CH₂CF₂-, -CF₂CF₂-, -CH=N-, -N=CH-, -N=N-, -CH=CR⁰¹-, -CR⁰¹=CH-, -CY⁰¹=CY⁰²-, -C≡C-, -(CH₂)₄-, -CH=CH-CO-O-, -O-CO-CH=CH- ou une liaison simple,
Y⁰¹ et Y⁰² sont, indépendamment l'un de l'autre, F, Cl ou CN et à titre d'alternative, l'un d'entre eux peut être H,
R⁰¹ et R⁰² sont, indépendamment l'un de l'autre, H ou alkyle avec de 1 à 12 atomes de C,
m est, pour chaque occurrence de manière indépendante, 1, 2 ou 3.

2. Composé selon la revendication 1, **caractérisé en ce qu'**il est de la formule I' ou de la formule I" 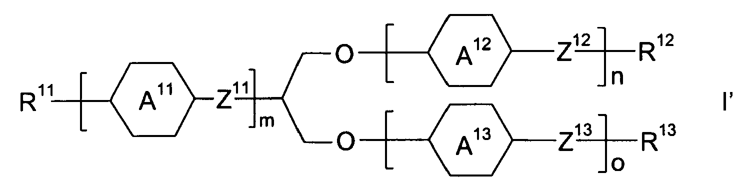 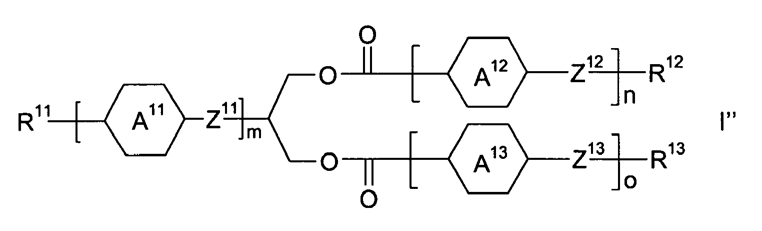 dans lesquelles les paramètres présentent les significations respectives données dans la revendication 1 et
R¹² et R¹³ indépendamment l'un de l'autre, présentent la signification donnée pour R¹¹ dans la revendication 1, 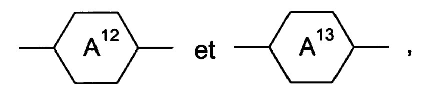 indépendamment l'un de l'autre pour chaque occurrence, présentent la signification donnée pour 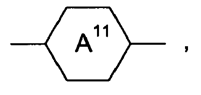 dans la revendication 1
Z¹² et Z¹³ indépendamment l'un de l'autre, présentent la signification donnée pour Z¹¹ dans la revendication 1,
n et o indépendamment l'un de l'autre, présentent la signification donnée pour m dans la revendication 1.

3. Composé selon au moins l'une des revendications 1 et 2, **caractérisé en ce qu'**au moins l'un des groupes 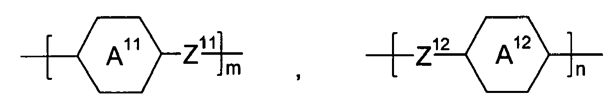 et, 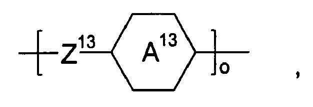 de la façon la plus préférable tous, est/sont choisi(s) parmi les formules qui suivent la à Ir et leurs images miroir respectives 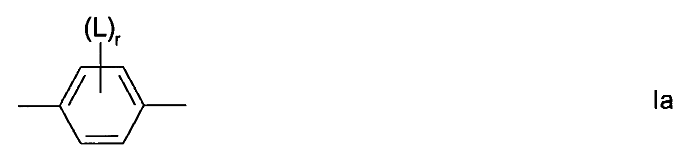 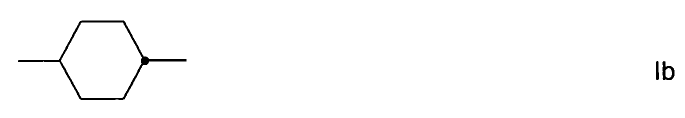 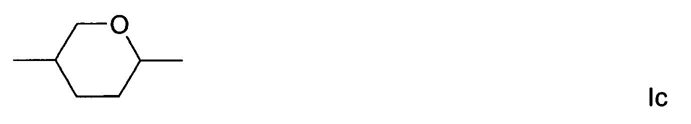 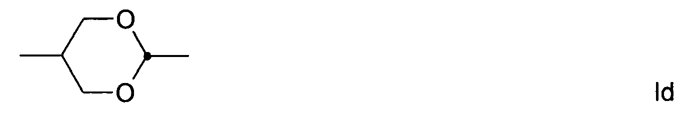 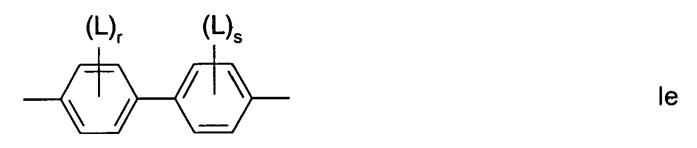 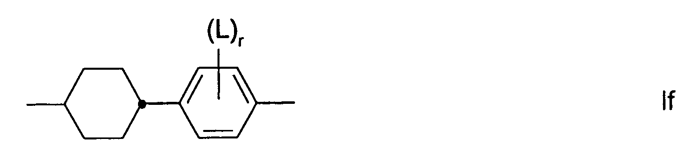 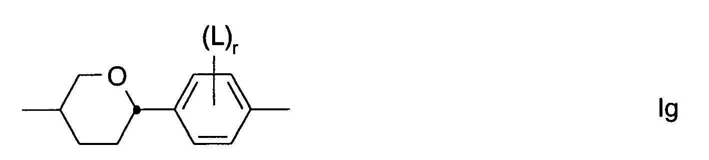 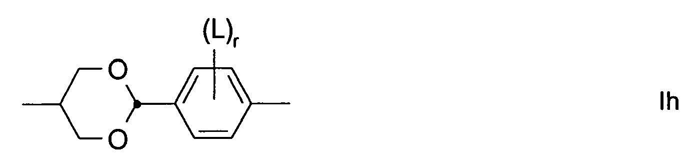 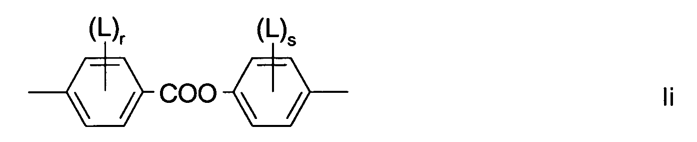 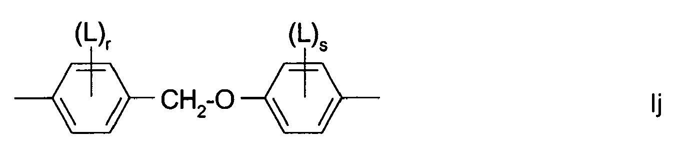 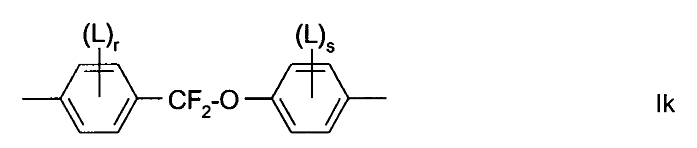 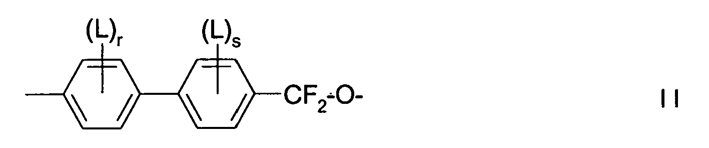 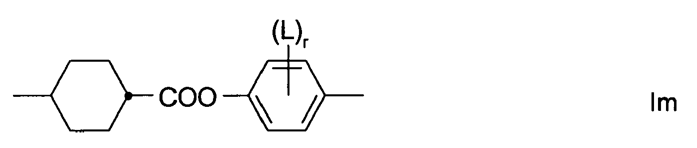 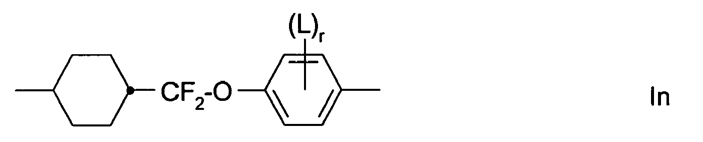 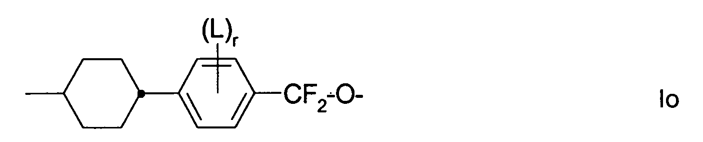 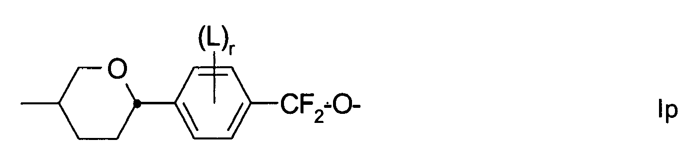 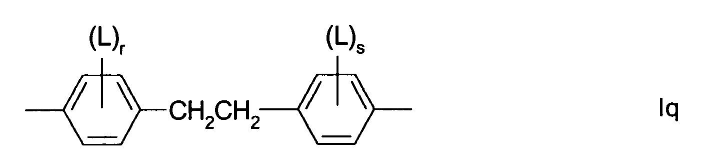 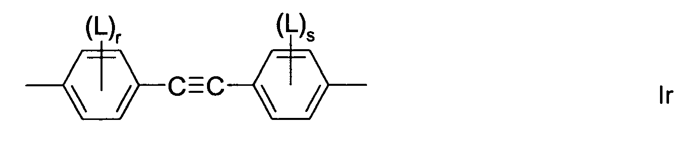 dans lesquelles L présente la signification donnée ci avant et r et s sont, indépendamment l'un de l'autre 0, 1, 2, 3 ou 4.

4. Composé selon au moins l'une des revendications 1 à 3, **caractérisé en ce qu'**il comprend deux groupes mésogènes MG¹ et MG³, qui sont identiques l'un à l'autre.

5. Milieu, **caractérisé en ce qu'**il comprend un composé selon au moins l'une des revendications 1 et 4.

6. Milieu selon la revendication 5, **caractérisé en ce qu'**il s'agit d'un milieu de modulation de lumière et **en ce qu'**il présente une phase bleue.

7. Elément de modulation de lumière, **caractérisé en ce qu'**il comprend un milieu selon au moins l'une des revendications 5 et 6.

8. Utilisation d'un composé selon au moins l'une des revendications 1 à 4 dans un milieu mésogène.

9. Utilisation d'un milieu selon au moins l'une des revendications 5 et 6 dans un élément de modulation de lumière.

10. Affichage électro-optique, **caractérisé en ce qu'**il comprend un milieu selon au moins l'une des revendications 5 et 6.
